# EUROPEAN PATENT SPECIFICATION

(11) **EP 2 198 012 B1**
(45) Date of publication and mention of the grant of the patent: **31.12.2014**
(21) Application number: 08802355.1
(22) Date of filing: 18.09.2008
(51) Int. Cl.: C12N 5/077

(54) **CARDIOMYOCYTE-LIKE CELL CLUSTERS DERIVED FROM HBS CELLS**
VON HBS-ZELLEN ABGELEITETE KARDIOMYOZYTENÄHNLICHE ZELLHAUFEN
AGRÉGAT DE CELLULES DE TYPE CARDIOMYOCYTE ISSUES DE CELLULES HBS

(30) Priority: 18.09.2007 DK 200701345; 18.09.2007 US 960160 P; 04.12.2007 US 996768 P; 09.06.2008 US 129177
(43) Date of publication of application: 23.06.2010
(73) Proprietor: Cellartis AB, 413 46 Göteborg (SE)
(72) Inventor: SARTIPY, Peter, S-434 96 Kungsbacka (SE); ÅKESSON, Karolina, S-412 82 Gothenburg (SE); AMÉEN, Caroline, S-414 64 Gothenburg (SE); SYNNERGREN, Jane, S-531 98 Lidköping (SE); DAHLENBORG, Kerstin, S-436 38 Askim (SE); STEEL, Daniella, S-414 60 Gothenburg (SE)
(74) Representative: Zacco Denmark A/S
(86) International application number: PCT/EP2008/007835
(87) International publication number: WO 2009/036982

(56) References cited:
- WO-A-2007/070964
- WO-A-2008/006605
- WO-A-2008/114204
- US-A1- 2005 214 938
- BURRIDGE PAUL W ET AL: "Improved human embryonic stem cell embryoid body homogeneity and cardiomyocyte differentiation from a novel V-96 plate aggregation system highlights interline variability" STEM CELLS, ALPHAMED PRESS, DAYTON, OH, US, vol. 25, no. 4, 21 December 2006 (2006-12-21), pages 929-938, XP002501116 ISSN: 1066-5099 [retrieved on 2006-12-21] cited in the application
- XU CHUNHUI ET AL: "Cardiac bodies: a novel culture method for enrichment of cardiomyocytes derived from human embryonic stem cells" STEM CELLS AND DEVELOPMENT, ELSEVIER, AMSTERDAM, vol. 15, no. 5, 1 October 2006 (2006-10-01), pages 631-639, XP009110259 ISSN: 1547-3287
- BADER ALICE ET AL: "Leukemia inhibitory factor modulates cardiogenesis in embryoid bodies in opposite fashions" CIRCULATION RESEARCH, vol. 86, no. 7, 14 April 2000 (2000-04-14), pages 787-794, XP002512699 ISSN: 0009-7330
- CAPI ET AL: "Myocardial regeneration strategies using human embryonic stem cell-derived cardiomyocytes" JOURNAL OF CONTROLLED RELEASE, ELSEVIER, AMSTERDAM, NL, vol. 116, no. 2, 6 December 2006 (2006-12-06), pages 211-218, XP005794273 ISSN: 0168-3659
- SARTIANI LAURA ET AL: "Developmental changes in cardiomyocytes differentiated from human embryonic stem cells: A molecular and electrophysiological approach" STEM CELLS (MIAMISBURG), vol. 25, no. 5, 2007, pages 1136-1144, XP002512700 ISSN: 1066-5099
- SYNNERGREN JANE ET AL: "Differentiating human embryonic stem cells express a unique housekeeping gene signature" STEM CELLS (MIAMISBURG), vol. 25, no. 2, February 2007 (2007-02), pages 473-480, XP002512701 ISSN: 1066-5099
- NORSTROM ANDERS ET AL: "Molecular and pharmacological properties of human embryonic stem cell-derived cardiomyocytes" EXPERIMENTAL BIOLOGY AND MEDICINE, SOCIETY FOR EXPERIMENTAL BIOLOGY AND MEDICINE, vol. 231, no. 11, 1 December 2006 (2006-12-01), pages 1753-1762, XP002471552 ISSN: 1535-3702 cited in the application
- GRAICHEN RALPH ET AL: "Enhanced cardiomyogenesis of human embryonic stem cells by a small molecular inhibitor of p38 MAPK" DIFFERENTIATION, vol. 76, no. 4, April 2008 (2008-04), pages 357-370, XP002512702 ISSN: 0301-4681
- BEQQALI ABDELAZIZ ET AL: "Genome-wide transcriptional profiling of human embryonic stem cells differentiating to cardiomyocytes", STEM CELLS (MIAMISBURG), vol. 24, no. 8, August 2006 (2006-08), pages 1956-1967, ISSN: 1066-5099
- FILIPCZYK A A ET AL: "Cardiovascular development: towards biomedical applicability; Regulation of cardiomyocyte differentiation of embryonic stem cells by extracellular signalling", CMLS CELLULAR AND MOLECULAR LIFE SCIENCES, BIRKHÄUSER-VERLAG, BA LNKD- DOI:10.1007/S00018-007-6523-2, vol. 64, no. 6, 13 February 2007 (2007-02-13), pages 704-718, XP019488196, ISSN: 1420-9071
- CHUNG YOUNG ET AL: "Human embryonic stem cell lines generated without embryo destruction", CELL STEM CELL, CELL PRESS, US, vol. 2, no. 2, 7 February 2008 (2008-02-07), pages 113-117, XP002604696, ISSN: 1934-5909, DOI: 10.1016/J.STEM.2007.12.013

## Description

### Field of the invention

The present invention relates to novel cardiomyocyte-like cell clusters (CMLC) derived from hBS cells and to the potential use of such cardiomyocyte-like cell clusters in e.g., pharmaceutical drug discovery and development, toxicity testing, cell therapy and medical treatment. The clusters contain cells expressing endodermal, mesodermal as well as cardiac markers. The invention also relates to a method for preparing the cluster, to composition comprising one or more CMLC for use in therapy and toxicity testing. The compositions are stable after storage of the composition at a temperature of at least -80 °C for at least 2 years, i.e. the characteristics and viability of the clusters are not substantially changed during this storage period.

### Background of the invention

Mature cardiomyocytes are considered terminally differentiated cells and as such they have no, or very low, proliferative capacity. Human cardiomyocytes can be isolated from heart biopsies but the procedure is complicated and it is difficult to obtain viable cell preparations. In addition, the access to human heart tissue is very limited and it is thus not possible to isolate large number of cells. An easy access to cells with a phenotype of human cardiomyocytes is critical for the development of cell therapy interventions for cardiac disease. In addition, due to the lack of donor material as well as the somewhat problematic procedure of cell isolation, human primary cardiomyocytes are not currently available for *in vitro* testing during pre-clinical drug discovery.

Populations of pluripotent human stem cells can be isolated from the inner cell mass of blastocysts and these cells have the capacity for indefinite, undifferentiated proliferation in vitro (Thomson et al., 1998; Reubinoff et al., 2000; Heins et al., 2004). Differentiation of human blastocysts derived stem (hBS) cells may occur spontaneously in vitro, especially during, for undifferentiated cells, sub-optimal culture conditions (Thomson et al., 1998; Reubinoff et al., 2000). In addition, hBS cells can be coaxed to differentiate in a directed fashion along specific pathways forming a variety of specialized cell types including cardiomyocytes, endothelial cells, neuronal cells, insulin producing β-cells, and hematopoietic cells (reviewed in: Keller, Genes Dev 2005). However, relatively little is currently known about how to control and manipulate hBS cell differentiation to produce exclusive populations of specific cell types.

For the derivation of cardiomyocytes from hBS cells, in principle two different procedures have been reported. The first is through differentiation of hBS cells initiated when the cells are cultured in suspension and form embryoid bodies (EBs) (Itskovitz-Eldor et al 2000, Kehat et al 2001). Within these mixed population of cells contracting areas with functional properties of cardiomyocytes can be observed. The second procedure is based on co-culture of hBS cells with END-2 cells (a visceral endoderm-like cell line) which results in the formation of beating clusters of cells that also display characteristics of cardiomyocytes (Mummery et al 2003, Passier et al 2005). Other variants of the described protocols included forced aggregation (Burridge et al 2007) and the hanging drop procedure (Yoon et al 2006). Based on the variations of the protocols used and the efficiency at which hBS cells differentiate to cardiomyocytes, it appears that hBS cell lines behave quite differently, implicating that the actual hBS cell line of use might affect the final result. Thus, each cell line may require a specific differentiation protocol for induction of cardiogenesis.

During recent years, a number of papers have described, in various ways, the basic characteristics of hBS cell-derived cardiomyocytes. In these reports, cell analysis has been based on the expression of molecular markers for cardiomyocytes, structural architecture, and functionality (reviewed in: Ameen et al 2007 Crit Rev Oncol Hematol). The morphology and ultrastructure of hBS cell-derived cardiomyocytes share similarities with adult cardiomyocytes although the myofibrillar and sarcomeric organization indicate an immature phenotype in the stem cell derived population (Kehat et al 2001, Snir et al 2003, Norström et al 2006, Yoon et al 2006). On a molecular level, several markers expressed by cardiomyocytes are also expressed by hBS cell-derived cardiomyocytes, including transcription factors, structural proteins, hormones, ion-channels, and tight junction proteins (Kehat et al 2001, Xu et al 2002, Mummery et al 2003, He et al 2003, Passier et al 2005, Norström et al 2006, Yoon et al 2006).

Taken together, the molecular and structural properties of the hBS cell derived cardiomyocytes suggest that these cells share similarities with their adult counterparts. More importantly, however, are the functional characteristics of the cells, and different pharmacological and electrophysiological approaches have been used to examine these properties. One major advantage of cardiomyocytes derived from hBS cells is that they can be maintained in culture for extended time periods without loosing their spontaneous contractile capacity. This allows for repeated non-invasive examination of the same cell preparations. Several studies have demonstrated that hBS cell-derived cardiomyocytes respond to α/β-adrenergic- and muscarinic stimuli suggesting that the cells express specific surface membrane receptors coupled to a signalling pathway that activate ion channels, membrane transporters and myofilament proteins (Kehat et al 2001, Xu et al 2002, Xue et al 2005, Norström et al 2006). In addition, action potentials indicative of nodal-, atrial- and ventricular-like origin have been identified in hBS cell-derived cardiomyocytes using intracellular electrophysiological measurements (Xu et al 2002, Mummery et al 2003, He et al 2003). Taken together, these results indicate that *in vitro* developed stem cell derived cardiomyocytes have a basal functionality which makes them attractive for further evaluation in terms of applicability in drug discovery.

Being a tissue like structure, cardiomyocyte like cell clusters from hBS cells specifically, has a great potential for several of these *in vitro* applications were they make up a platform for measurements of functional attributes such as action potentials and conduction in cardiomyocytes. In addition, the developing clusters may serve as in vitro models to study early events during human cardiogenesis. Furthermore, based on the tissue similarities the clusters may be optimal for use *in vivo* for restoration of cardiac function after injury or disease. The present invention presents unique cardiomyocyte-like cell clusters derived from hBS cells for use according to the above.

### Brief description of the invention

The present invention relates to novel cardiomyocyte-like cell clusters (CMLC) and the methods for their preparation from hBS cells. The CMLC of the present invention are especially well suited for use in drug discovery and toxicity testing.

A cluster according to the invention comprises cardiomyocyte-like cells, wherein the cluster has
i) contracting cells,
ii) cells that are electrically connected,
   and expresses
iii) cardiac markers including Nkx.2.5, troponin and myosin,
iv) markers for functional adrenergic receptors,
v) markers for functional muscarinic receptors,
vi) markers for functional ion-channels including hERG, Na+, Ca2+ and K+ channels,
vii) one or more endodermal markers selected from the group consisting of AFP, TF, APOA2, AHSG, SERPINA1, APOA1, APOC3, TTR, APOB, and RBP4,
viii) absence of expression of one or more of the following markers for undifferentiated cells: OCT-3/4, SSEA-4, TRA-1-60, and
   the cluster comprising genes that are up-regulated and have,
   i) expression values of 500 or more,
   ii) a fold change in gene expression between cardiomyocyte-like cell clusters and undifferentiated hBS cells (FC_{CMLC}) of 10 or more,
   iii) a ratio between FC_{CMLC} and FC_{MC} (i.e. the fold change between mixed differentiated hBS cells and undifferentiated hBS cells) of 10 or more, and
   the cluster comprises cells expressing all of the following genes:

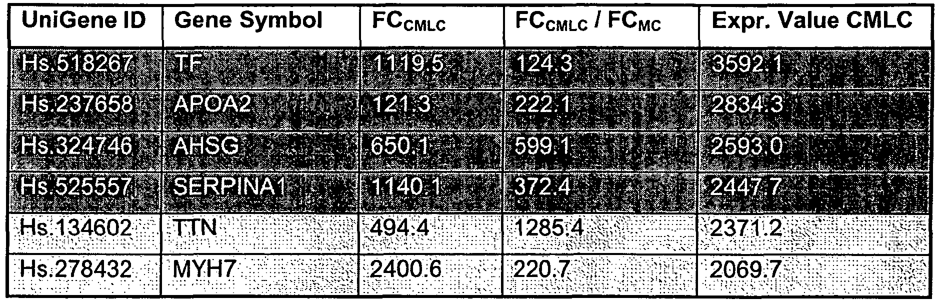

The electrical connection between cells mentioned above may be by gap junctions.

It is important that the cluster contains cells that respond to pharmacological stimuli such as beta-adrenergic stimulation (isoproterenol, adrenalin), alpha-adrenergic stimulation (phenylephrine), muscarinic stimulation (carbachol, acetylcholine, atropine), blockage of calcium channels (verapamil), blockage of hERG channels (E4031) and inhibition of funny channels.

A cluster according to the present invention comprises cardiomyocyte-like cells and the cluster comprising genes that are up-regulated and have,
i) expression values of 500 or more,
ii) a fold change in gene expression between cardiomyocyte-like cell clusters and undifferentiated hBS cells (FC_{CMLC}) of 10 or more.

In the examples herein are demonstrated suitable methods for the determination of the above-mentioned values.

The present inventors have found that cardiomyocyte-like cells suitable for use in e.g. medical applications, drug screenings and cardiotoxicity studies can be isolated in the form of clusters that are relatively easy to prepare in a reproducible manner and without any need for further differentiation into cardiomyocyte cells. Besides cardiac markers, the clusters also contains markers e.g. for endodermal lineage, express one or more ion channels commonly used for cardiotoxicity testing, and express transcription factors.

Accordingly, the present invention provides a relative simple means of obtaining a suitable cardiomyocyte-like cell product for medical use as well as for drug screening and testing without the need of purification and complete differentiation to mature cardiomyocytes.

Moreover, the present invention provides a composition that presents the CMLC in a form that is easy to use for the end-user. The composition is prepared by suspending the CMLC in an aqueous medium notably containing agents that ensure the stability of the CMLC during freezing/vitrification, storage, thawing and use of the cluster. Accordingly, the properties of such an aqueous medium must be chosen in consideration of all these four different processes in order to obtain a sole medium that is suitable for the handling of the clusters from the clusters have been prepared and until use of the end-user. The product is presented in fresh or frozen form.

### Detailed description of the invention

As used herein, the term "blastocyst-derived stem cell" is denoted BS cell, and the human form is termed "hBS cells". In literature the cells are often referred to as embryonic stem cells, and more specifically human embryonic stem cells. The pluripotent stem cells used in the present invention can be commercially available hBS cells or cell lines. However, it is further envisaged that any human pluripotent stem cell can be used in the present invention, including differentiated adult cells which are reprogrammed to pluripotent cells by e.g. the treating adult cells with certain transcription factors, such as OCT4, SOX2, NANOG, and LIN28 as disclosed in Junying Yu, et al., 2007.

As used herein the term "DMSO" is intended to mean dimethylsulfoxide.

As used herein the term "cardiomyocyte-like cells" is intended to mean cells sharing features with mature cardiomyocytes. Cardiomyocyte-like cells are further defined by morphological characteristics as well as by specific marker characteristics.

As used herein the terms "cardiomyocyte-like cells clusters" (CMLC) is intended to mean a group comprising at least two cardiomyocyte-like cells attached to each other, and which also comprise other cell types. A cluster contains beating cells.

As used herein the term "nodal-like cells" is intended to mean cells with the following action potential characteristics:
Membrane resting potential (MRP): -40 to -60 mV,
Duration: 80-130 ms, and
A pronounced phase 4 depolarization.

As used herein the term "atrial-like cells" is intended to mean cells with the following action potential characteristics:
Membrane resting potential (MRP): -50 to -80 mV, and
Duration: < 150 ms.

As used herein the term "ventricle-like cells" is intended to mean cells with the following action potential characteristics:
Membrane resting potential (MRP): -50 to -80 mV,
Duration: > 150 ms, and

### A pronounced plateau phase.

In the present context, the term "expression" is used for gene and/or protein expression, whatever must be relevant in the context.

In the present application, the term "cryopreservation" denotes the preservation of biological material at an extremely low temperature.

In the present context, the term "mixed differentiated hBS cells" is used for a population of spontaneously differentiated hBS cells were cells from all three germ layers mesoderm, endoderm and ectoderm are present. This sample is used as control material to distinguish genes up regulated in many types of differentiated cells from genes specifically up regulated in cardiomyocyte-like cell clusters. In the examples herein is given instructions how to obtain mixed differentiated hBS cells.

In the present context, the term "forced aggregation" is used for 3D aggregation of cells by an external force, such as centrifugation with a centrifugal force or sedimentation by gravity.

### Feeder cells

As used herein feeder cells are intended to mean supporting cell types used alone or in combination. The cell type may further be of human or other species origin. The tissue from which the feeder cells may be derived include embryonic, fetal, neonatal, juvenile or adult tissue, and it further includes tissue derived from skin, including foreskin, umbilical chord, muscle, lung, epithelium, placenta, fallopian tube, glandula, stroma or breast. The feeder cells may be derived from cell types pertaining to the group consisting of human fibroblasts, fibrocytes, myocytes, keratinocytes, endothelial cells and epithelial cells. Examples of specific cell types that may be used for deriving feeder cells include embryonic fibroblasts, extraembryonic endoderm cells, extraembryonic mesoderm cells, fetal fibroblasts and/or fibrocytes, fetal muscle cells, fetal skin cells, fetal lung cells, fetal endothelial cells, fetal epithelial cells, umbilical chord mesenchymal cells, placental fibroblasts and/or fibrocytes, placental endothelial cells,

As used herein, the term "MEF cells" is intended to mean mouse embryonic fibroblasts.

As used herein, the term "TGF-β" means transforming growth factor beta, preferably of human and/or recombinant origin. TGF-β is a protein that comes in three isoforms called TGF-β1, TGF-β2 and TGF-β3. The TGF-β family is part of a superfamily of proteins known as the transforming growth factor beta superfamily, which includes inhibins, activin, anti-müllerian hormone, bone morphogenetic protein, decapentaplegic and Vg-1.

As used herein, the term "FGF" means fibroblast growth factor, preferably of human and/or recombinant origin, and subtypes belonging thereto are e.g. bFGF (sometimes also referred to as FGF2) and FGF4.

The term "BMP2" is intended to mean bone-morphogenic protein-2 which is one member of the BMP family of growth factors.

As used herein the term "xeno-free" is intended to mean complete circumvention of direct or in-direct exposure to non-human animal components.

Cardiac stem cell marker:
c-kit and cardiac progenitor markers: Islet-1, Nkx2.5, GATA-4. Here regarded as early cardiac markers.
Markers for cardiomyocytes: cTnI, cTnT (cardiac troponin I and T), α-MHC (alpha-myosin-heavy-chain), connexin-43.

Other markers appear from the examples herein.

### CMLC

A cluster according to the invention comprises cardiomyocyte-like cells, wherein the cluster has
i) contracting cells,
ii) cells that are electrically connected,
   and expresses
iii) cardiac markers including Nkx.2.5, troponin and myosin,
iv) markers for functional adrenergic receptors,
v) markers for functional muscarinic receptors,
vi) markers for functional ion-channels including hERG, Na+, Ca2+ and K+ channels,
vii) one or more endodermal markers selected from the group consisting of AFP, TF, APOA2, AHSG, SERPINA1, APOA1, APOC3, TTR, APOB, and RBP4,
viii) absence of expression of one or more of the following markers for undifferentiated cells: OCT-3/4, SSEA-4, TRA-1-60, and
   the cluster comprising genes that are up-regulated and have,
   i) expression values of 500 or more,
   ii) a fold change in gene expression between cardiomyocyte-like cell clusters and undifferentiated hBS cells (FC_{CMLC}) of 10 or more,
   iii) a ratio between FC_{CMLC} and FC_{MC} (i.e. the fold change between mixed differentiated hBS cells and undifferentiated hBS cells) of 10 or more, and
      the cluster comprises cells expressing all of the following genes:

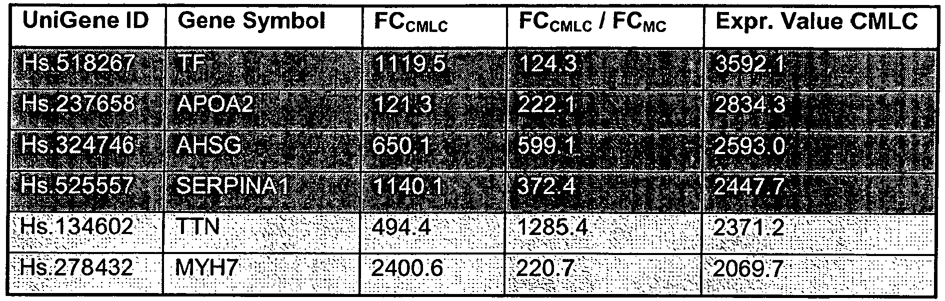

The electrical connection between cells mentioned above may be by gap junctions.

In a specific embodiment the cluster does not express any markers for undifferentiated cells.

The cluster comprising genes that are up-regulated and have,
i) expression values of 500 or more,
ii) a fold change in gene expression between cardiomyocyte-like cell clusters and undifferentiated hBS cells (FC_{CMLC}) of 10 or more.

The above mentioned values can be measured as described in Example 2 herein. The present inventors have applied two specific ways of obtaining the fold change values:
i) by measuring the fold change in gene expression between CMLC and undifferentiated cells,
ii) as above but supplemented with measuring the fold change in gene expression between CMLC and mixed differentiated hBS cells (see below).

The results from the two methods differ slightly and it is contemplated that the use of method ii), i.e. where a further selection is performed, may lead to a cluster having properties more suitable for use in specific applications.

Accordingly, in a specific embodiment, the present invention provides a cluster comprising cardiomyocyte-like cells, the cluster comprising genes that are up-regulated and have,
i) expression values of 500 or more,
ii) a fold change in gene expression between cardiomyocyte-like cell clusters and undifferentiated hBS cells (FC_{CMLC}) of 10 or more, and
iii) a ratio between FC_{CMLC} and FC_{MC} of 100 or more.

F_{MC} denoted the fold change for a mixed differentiated hBS cell population. In the present context, the term "mixed differentiated hBS cells" is used for a population of spontaneously differentiated hBS cells where cells from all three germ layers mesoderm, endoderm and ectoderm are present. This sample is used as control material to distinguish genes up-regulated in all types of differentiated cells from genes specifically up-regulated in cardiomyocyte-like cell clusters.

Furthermore, the expression values are obtained by measurement with a specific chip (GeneChip® Human Genome U133 Plus 2.0 (Affymetrix Inc., Santa Clara, CA, USA) as detailed described in Example 2 herein. Use of other chips may give different values and therefore any comparison with the values herein should be made using the same kind of chip or a chip giving similar results.

In a more specific embodiment of the invention, a cluster is thus provided wherein the cluster comprising genes which are up-regulated and have,
i) expression values of 2000 or more,
ii) a FC_{CMLC} value of 100 or more, and
iii) a ratio between FC_{CMLC} and FC_{MC} of 100 or more.

In the examples herein details are given with respect to how a person skilled in the art can carry out experiments in order to determine the above-mentioned values.

The clusters are derived from hBS cells. Accordingly, the starting material may be commercially available hBS cells or cell lines. Such material can be obtained from Cellartis AB (www.cellartis.com) and is also available through the NIH stem cell registry (http://stemcells.nih.gov/research/registry) as well as from the UK Stem Cell Bank (http://www.ukstemcellbank.org.uk).

As mentioned before, not every individual cell contained in the cluster may fulfill the above-mentioned requirements. The requirements are fulfilled for the cluster and not necessarily for any individual cell. Furthermore, the cells contained in the cluster may be of different degree of differentiation; some may be entirely differentiated into cardiomyocyte-like cells, whereas others may possess characteristics like endodermal cells. However, in any event, the cluster must contain contracting cells. As mentioned in the examples herein, the clusters are typically prepared by subjecting cells to spontaneous differentiation via forced aggregation to obtain 3D-structures containing undifferentiated cells. The cardiomyocyte like cell clusters appear after further cultivation of the 3D-structures for up to 22 days.

Furthermore, the different cells and differentiation degree of the cells contained in the cluster is envisaged to be advantageous in the applications of the CMLC. For example, the various cell types may be of importance for increasing adhesion and binding of the CMLC to different surfaces in vitro (e.g. culture dishes or glass surfaces, coated or non-coated). They could also contribute to enhanced cell survival during cryopreservation and improved engraftment of the cells when CMLC are used for in vivo applications (e.g. cell transplantation). Accordingly, an objective of the present invention is to provide a cluster containing different types of cells in their development from hBS cells to cardiomyocytes, i.e. from the very beginning of differentiation via intermediate differentiation to fully differentiated cells into cardiomyocytes. As demonstrated in the examples herein none of the cells in the clusters has been found to be undifferentiated cells. Accordingly, and as demonstrated in the examples and figures, a cluster of the invention has a variety of markers.

Normally, the clusters contain a plurality of cells such as from about 10 to about 5000 cells.

In a specific embodiment, a cluster according to the invention contains cells with up-regulated genes (compared to undifferentiated cells), wherein the expression value is about 750 or more such as, e.g., an expression value of about 1000 or more, about 1500 or more, about 2000 or more, about 2500 or more or about 3000 or more.
Alternatively, or moreover, a cluster according to the invention contains cells with up-regulated genes having a FC(_{CMLC}) value of about 20 or more such as, e.g., about 50 or more, about 100 or more, about 500 or more, about 750 or more, about 1000 or more. Alternatively or additionally (when the method involving mixed differentiated hBS cells) is employed), the ratio of FC_{CMLC}/FC_{MC} is about 15 or more such as, e.g., about 20 or more, about 50 or more, about 100 or more, about 500 or more, about 750 or more, about 1000 or more. A set of criteria can be set in order to select clusters with different content of up-regulated genes.

Some of the genes identified in the clusters are genes that are not normally associated with cardiomyocyte-like cells (see the genes marked as "white" in Table II). These findings seem to be of interest and they may play an important role for the functionality of the CMLC with respect to medical use and testing. Specifically cells and tissue of endodermal origin seem to play an essential role in early cardiac development. The CMLC make up a tissue like structure simulating early developing cardiac tissue.

As shown in the Examples herein, a cluster according to the invention has the following characteristics (Table II and III):

**TABLE II**. Summary of genes that are specifically up-regulated in the CMLC. All FC values in the table are, as indicated above, calculated in relation to undifferentiated hBS cells. The genes are sorted in descending order based on the Expression value of CMLC.

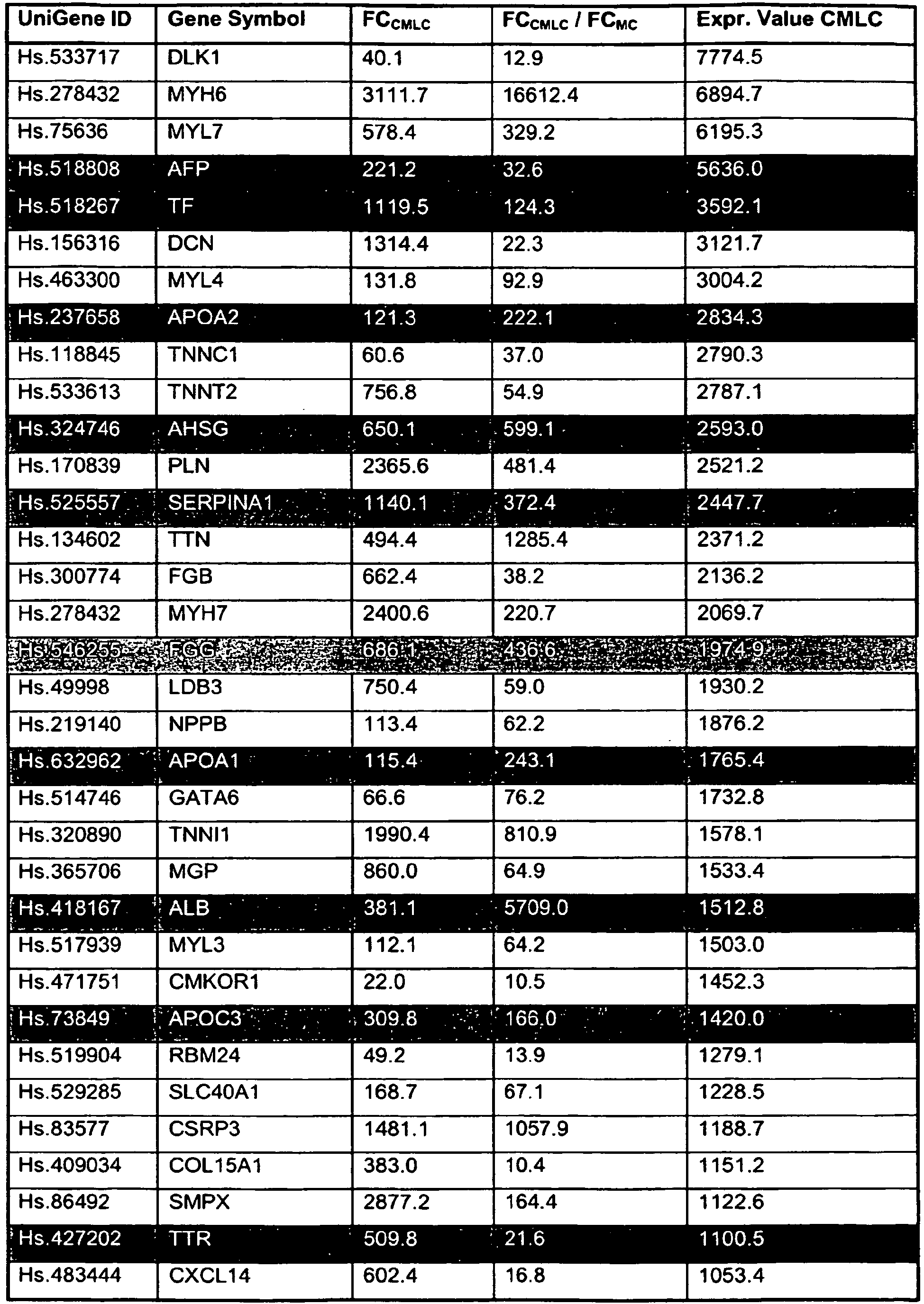

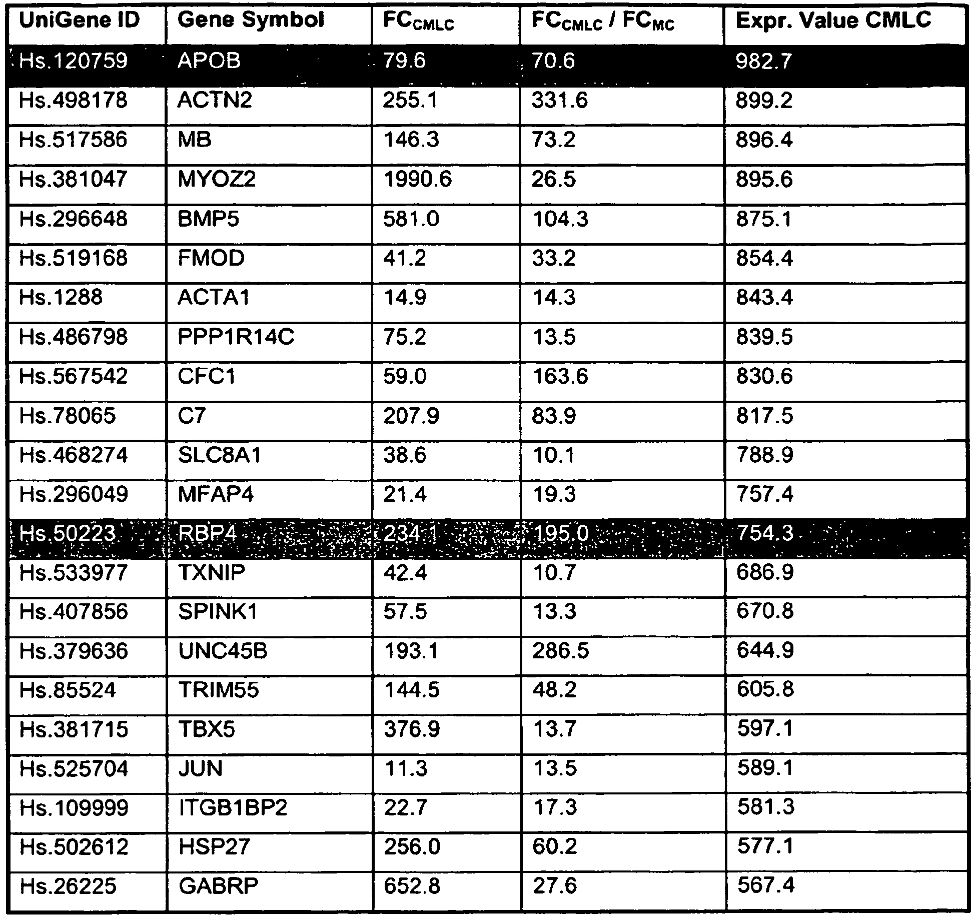

Of particular note is that the majority of the genes in Table II can be classified into two different groups. The first group consists of genes previously associated with cardiac cells and the second of genes previously associated with endodermal cells, such as hepatocytes. Examples of genes belonging to the first group are (marked in light grey in table II): MYH6, MYL7, MYL4, TNNC1, TNNT2, PLN, TTN, MYH7, LDB3, NPPB, GATA6, MYL3, CSRP3, ACTN2, MB, MYOZ2, TBX5, and HSP27. Examples of genes belonging to the second group are (marked in dark grey in table II): AFP, TF, APOA2, AHSG, SERPINA1, APOA1, ALB, APOC3, TTR, APOB, and RBP4.

To get an even stricter selection of genes specifically up-regulated in CMLC the criteria were narrowed to:
i) Genes with Expression values below 2000 in the CMLC were removed
ii) Genes with FC_{CMLC} below 100 were removed
iii) Genes with a FC_{CMLC} / FC_{MC} ratio below 100 were removed.

The remaining 9 genes are listed in Table III and these represent suitable marker genes for the CMLC.

**TABLE III.** Summary of genes that are specifically up-regulated in the CMLC after selection with stricter criteria. All FC values in the table are, as indicated above, calculated in relation to undifferentiated hBS cells. The genes are sorted in descending order based on the Expression value of CMLC.

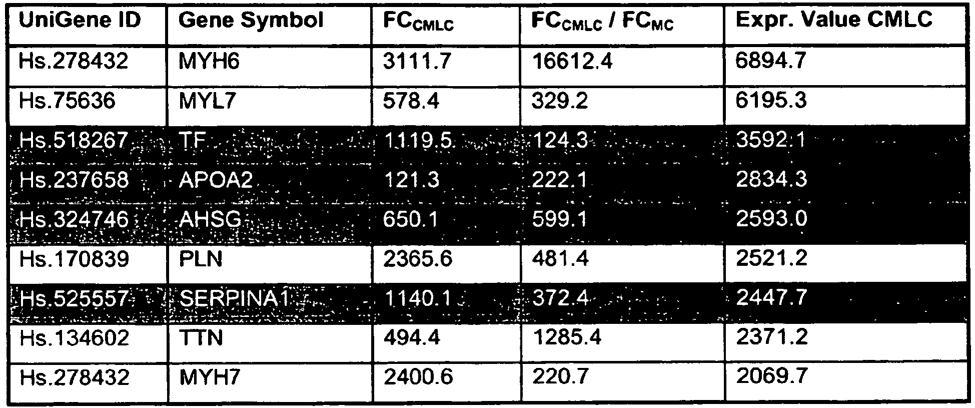

The selection shows an even distribution between endodermal and mesodermal genes expressed in the clusters. The mesodermal genes and the endodermal are marked in, light grey and dark grey respectively in Table III.

In other specific embodiment, a cluster according to the invention has 2 or more such as, e.g., 4 or more, 6 or more, 8 or more, 10 or more, 12 or more or 16 or more of the up-regulated genes are genes associated with cardiac cells, and/or
2 or more such as, e.g., 4 or more, 6 or more, 8 or more of the up-regulated genes are genes associated with endodermal cells, and/or
2 or more such as, e.g., 4 or more, 6 or more, 8 or more of the up-regulated genes are genes associated with non cardiac or non endodermal cells, described in Table II herein, and/or
the up-regulated genes comprise 10 or more such as, e.g., 20 or more, 30 or more, 40 or more, 50 or more, 55 or more or all genes listed in Table II herein.

In a more specific embodiment of the invention a cluster is provided wherein the cluster comprising genes which are up-regulated and have,
i) expression values of 2000 or more,
ii) a FC_{CMLC} value of 100 or more, and
iii) a ratio between FC_{CMLC} and FC_{MC} of 100 or more.

In a further embodiment of the present invention a cluster is provided wherein the cluster comprising genes which are up-regulated and have,
i) expression values of 2000 or more,
ii) a FC_{CMLC} value of 100 or more.

The cluster is normally derived from hBS cells and the number of cells in the cluster is normally 10 to about 5000 cells or from 10 to about 2000 cells.

Moreover, a cluster according to the invention expresses one or more ion channels. Notably, the ion channels identified are ion channels commonly used in cardiotoxicity testing. This is a further indication of the suitability of the CMLC for cardiotoxicity testing.

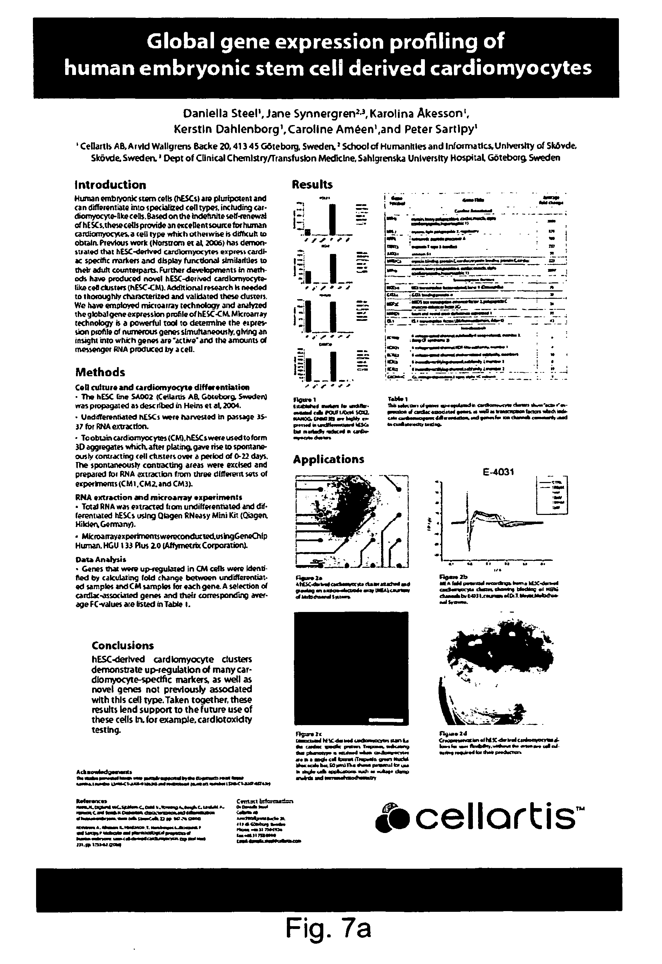

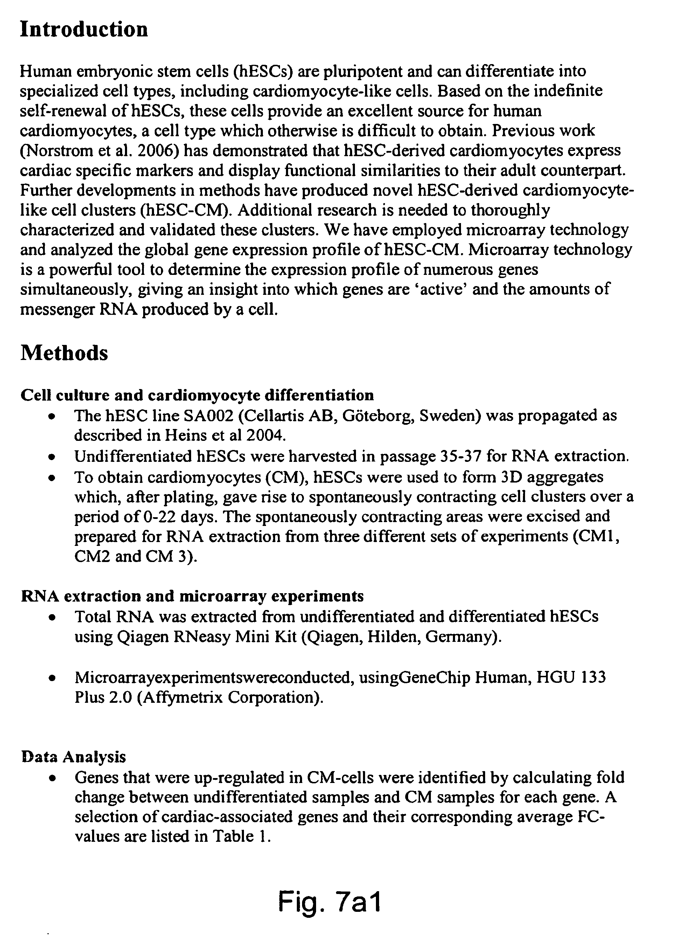

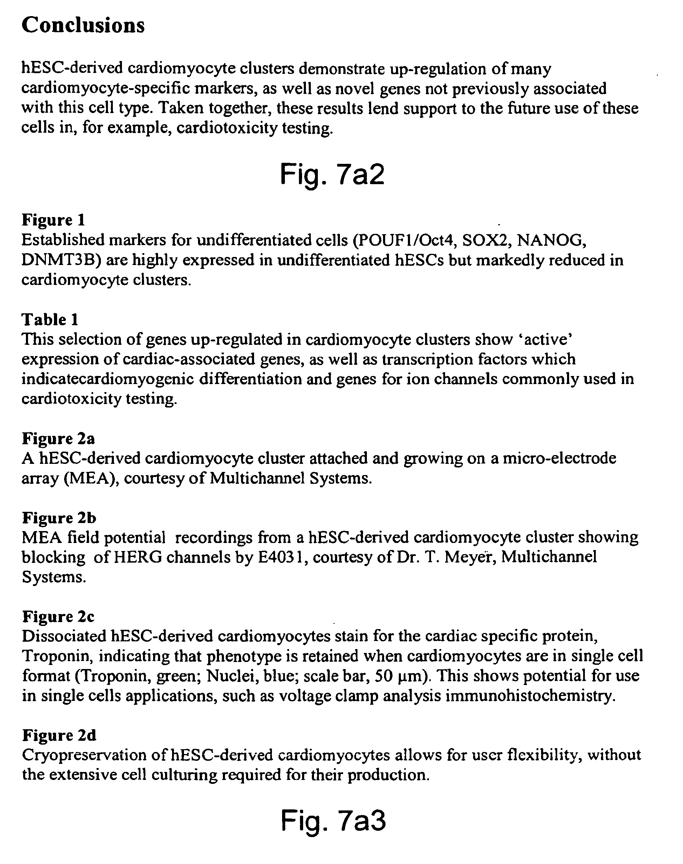

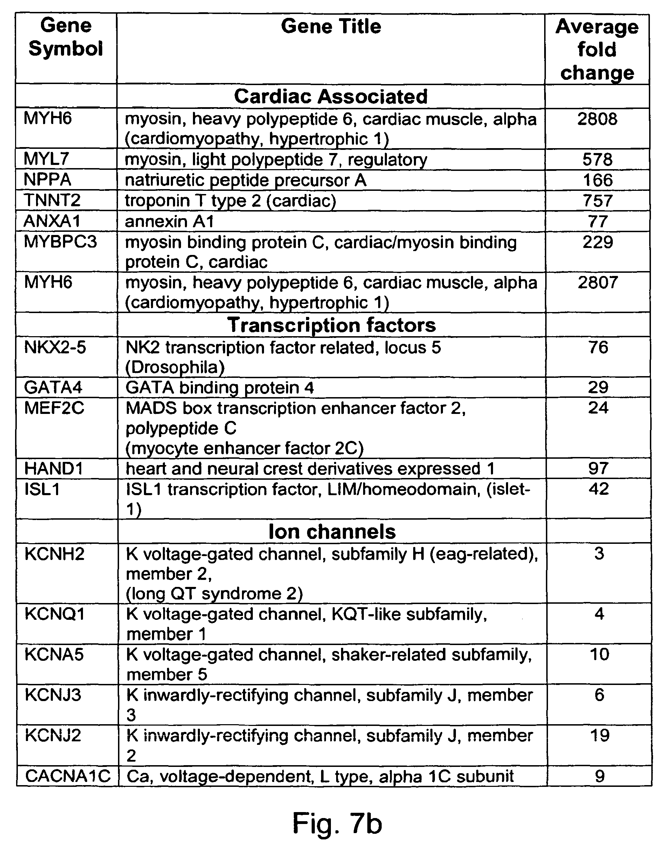

The ion-channel is typically a K-, Na, and/or Ca-ion channel such as a K-voltage-gated channel, a K-inwardly-rectifying channel, a Na-voltage-gated channel, a Na-ligand-gated channel, and/or a Ca-voltage-dependent channel. More specific ion channels are listed in Figure 7 a and b, poster Table 1. In a specific embodiment a cluster expresses at least 3 such as, e.g., at least 4, at least 5 or all of the ion channels listed in Figure 7 a and b, poster Table 1.

In a very specific embodiment, a cluster according to the invention has all genes listed in any of Table II, III and/or listed in Figure 7 a and b, poster Table 1.

More specific embodiments appear from the appended items and claims.

The clusters of cardiomyocyte-like cells described above may also be characterized by measuring their transmembrane action potential. By using a sharp microelectrode to enter the clusters, as further described in Example 11, the characteristic action potential were recorded and the AP duration at 50%, 70% and 90% of repolarization (dur50 (APD50), dur70 (APD70), dur90 (APD90)), AP amplitude (amp), membrane resting potential (MRP), and maximum rate of rise of the AP upstroke (Vmax) were determined.

The clusters were thus characterized as predominately nodal-like, atrial-like, or ventricle-like based on the results of transmembrane action potential (TAP) recordings. Cluster, or suspension of clusters, were thus found that comprised at least 10%, (such as e.g. at least 13%, at least 15%, or about 17%) of nodal-like cells and/or at least 30% (such as e.g. at least 35%, at least 40%, at least 45 % or about 50%) of atrial-like cells and/or at least 20% (such as e.g. at least 23%, at least 26%, at least 30%, or about 33%) ventricle-like cells.

As seen from the Tables above, the clusters contain cells with non-cardiac markers and express a gene number exceeding previously reported studies. Thus, a cluster according to the invention typically comprises cells expressing at least 10, at least 15, at least 20, at least 25 or all of the following genes:

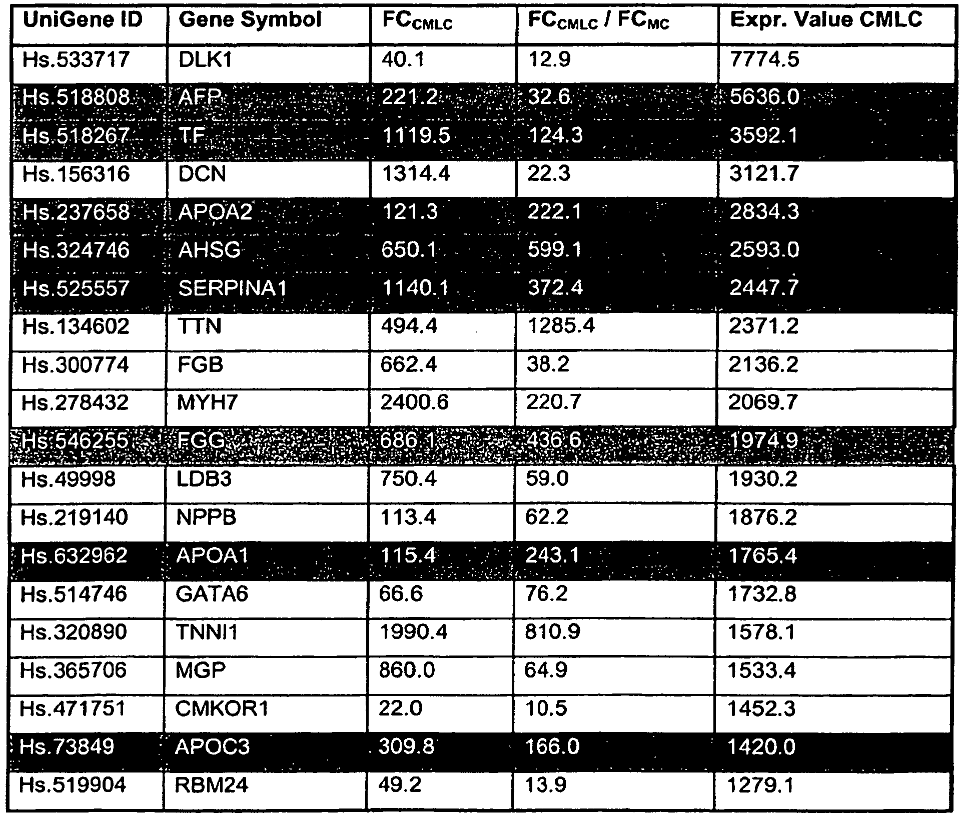

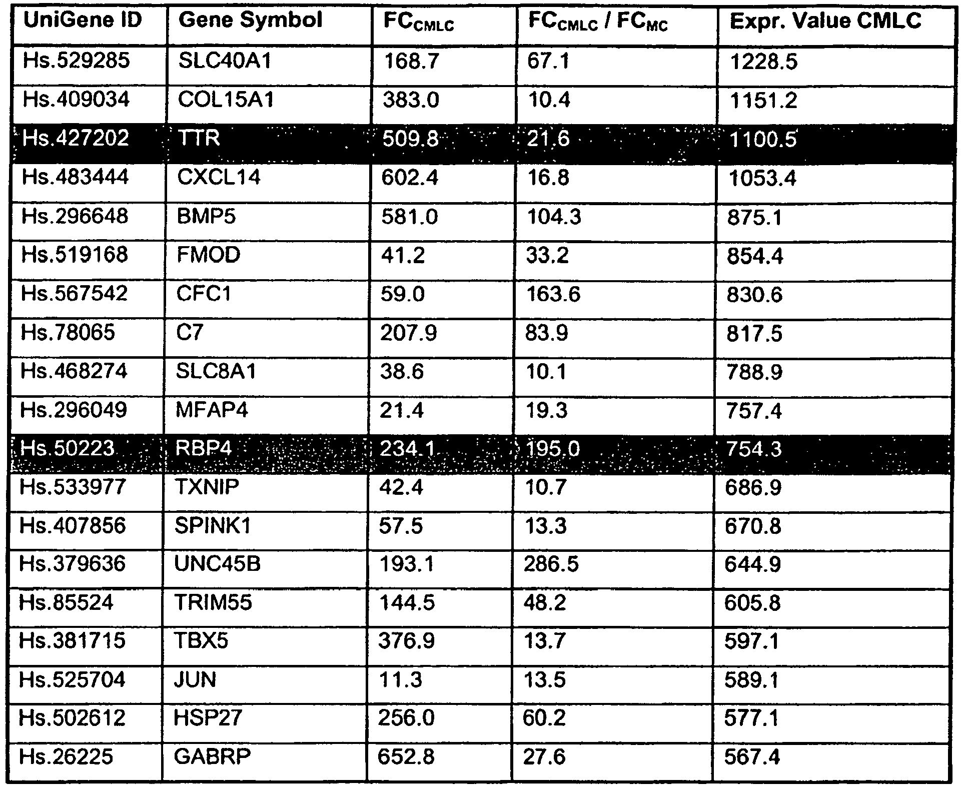

A cluster of the invention comprises cells expressing all of the following genes:

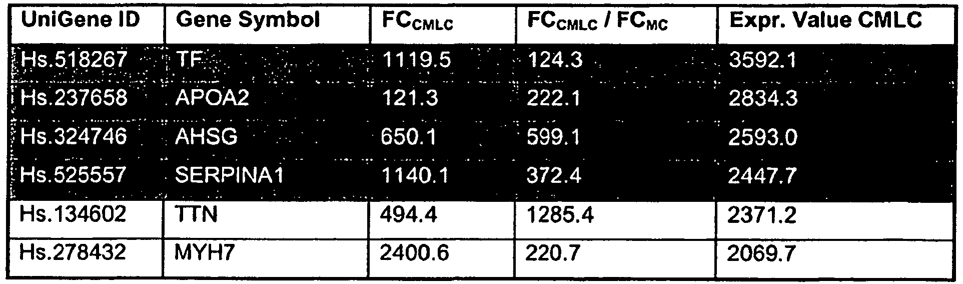

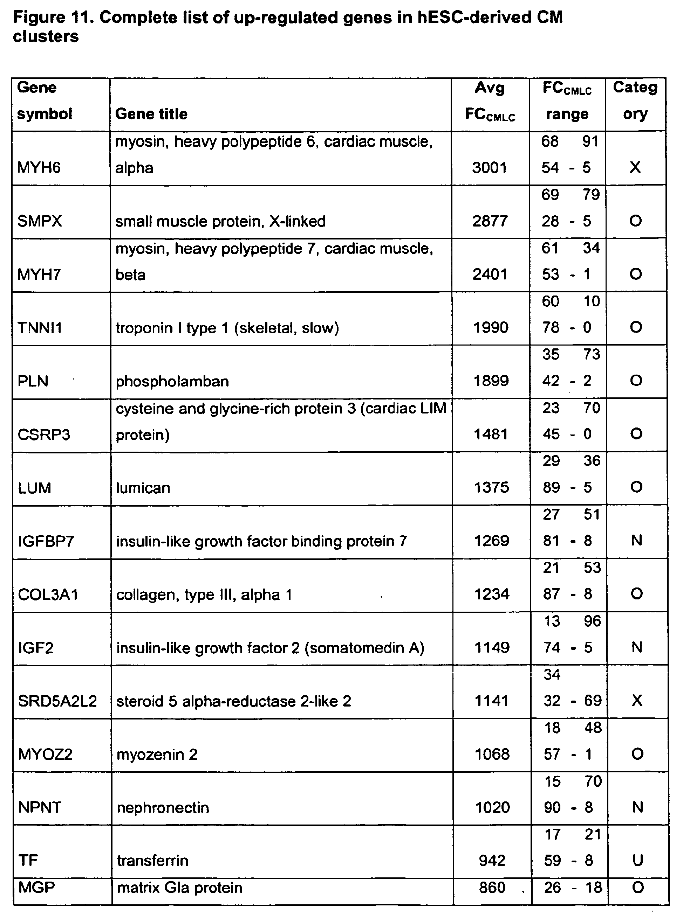

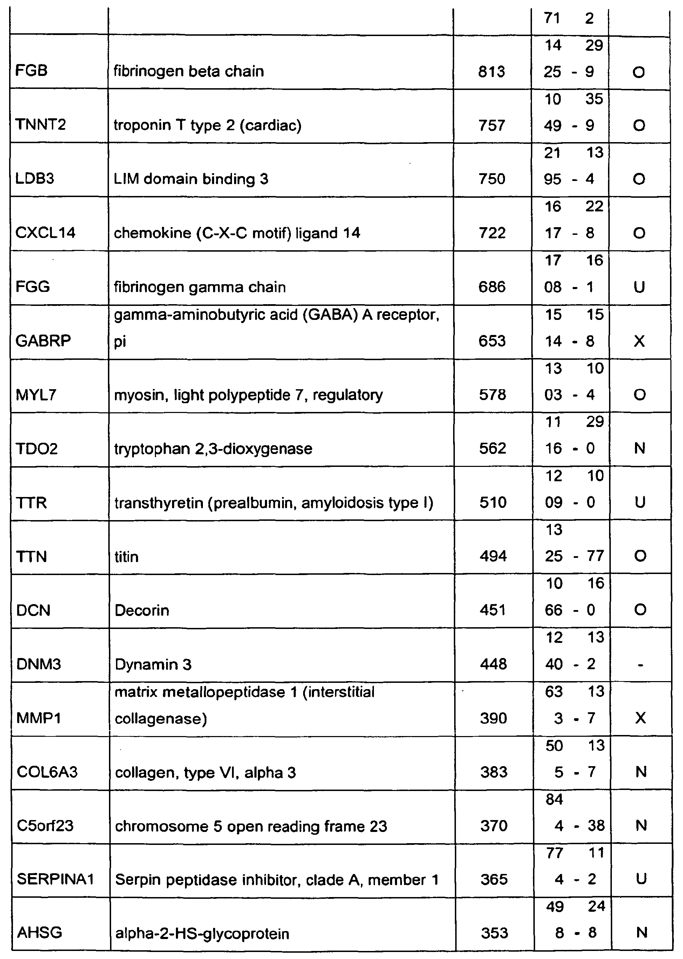

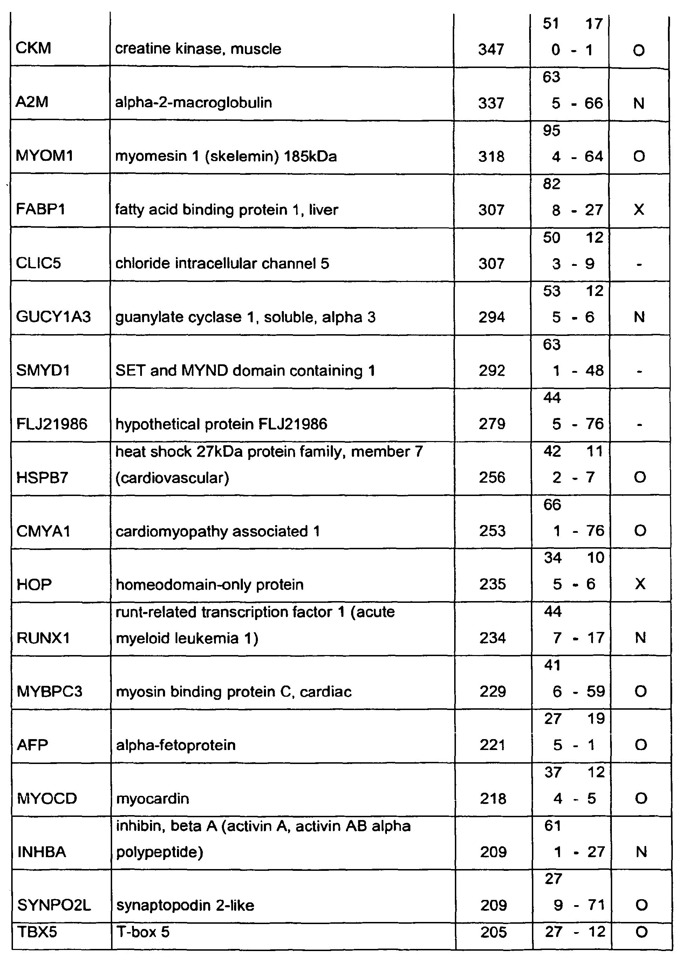

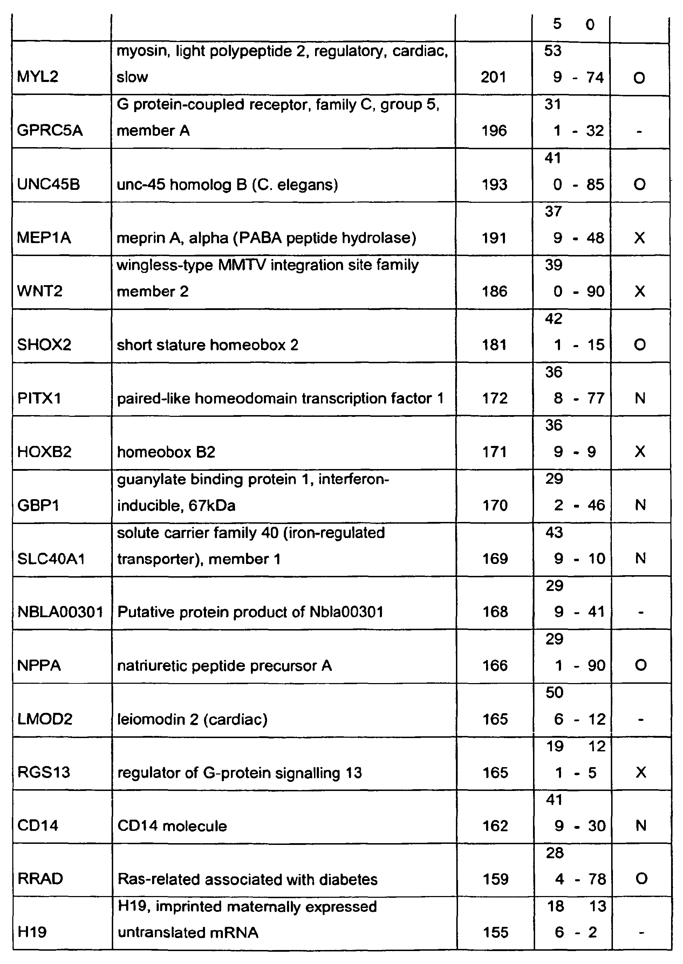

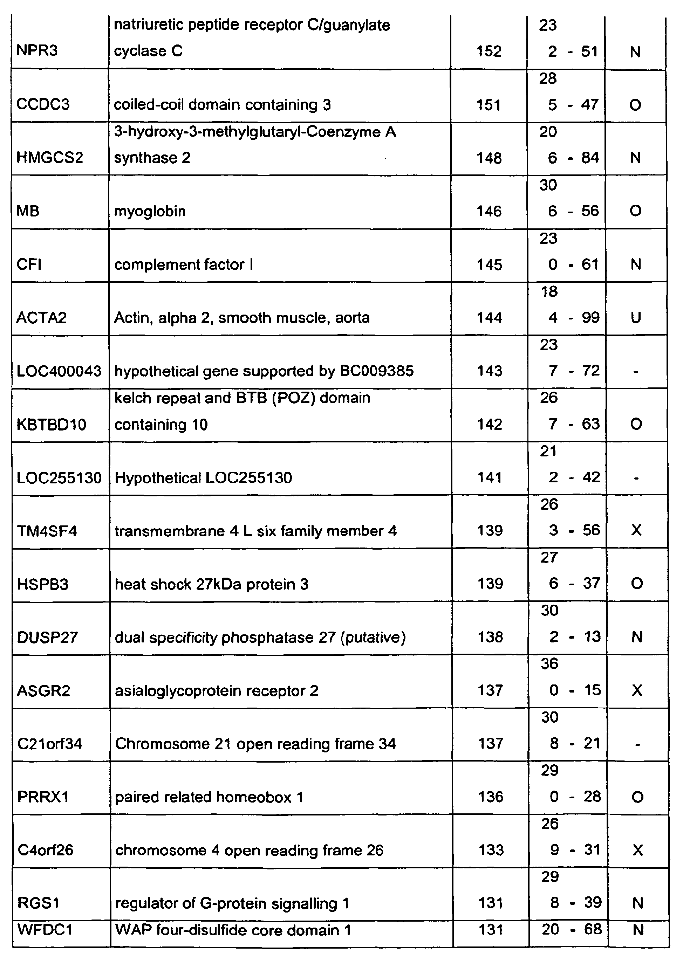

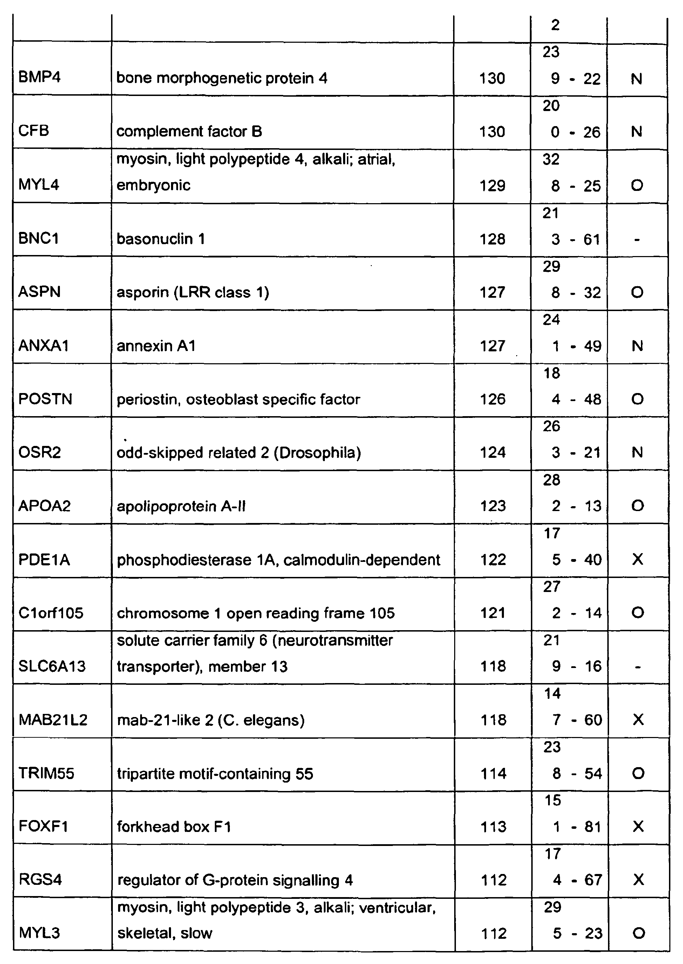

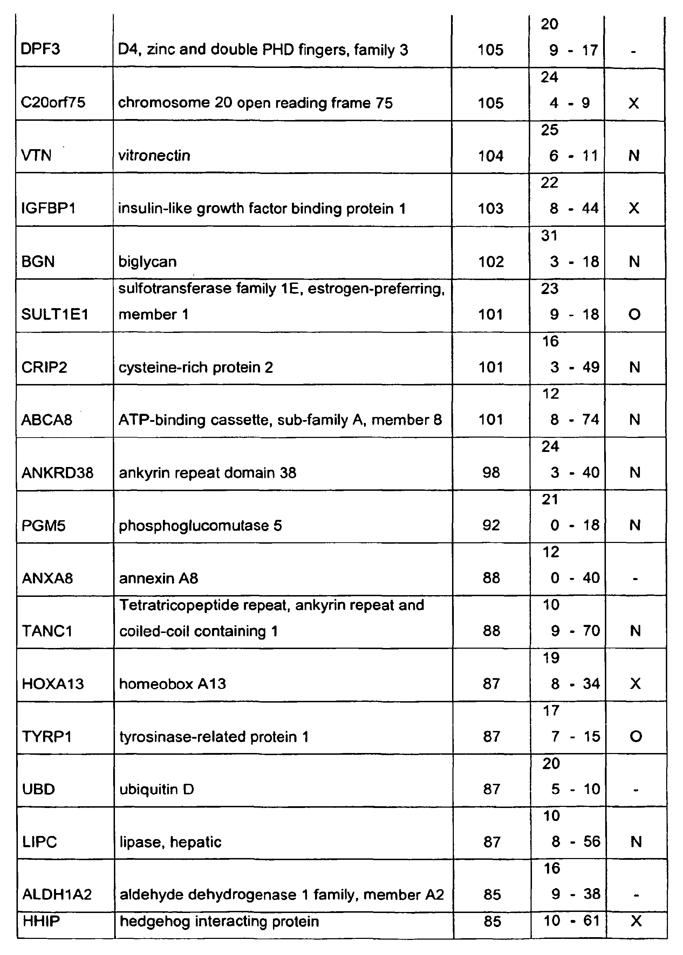

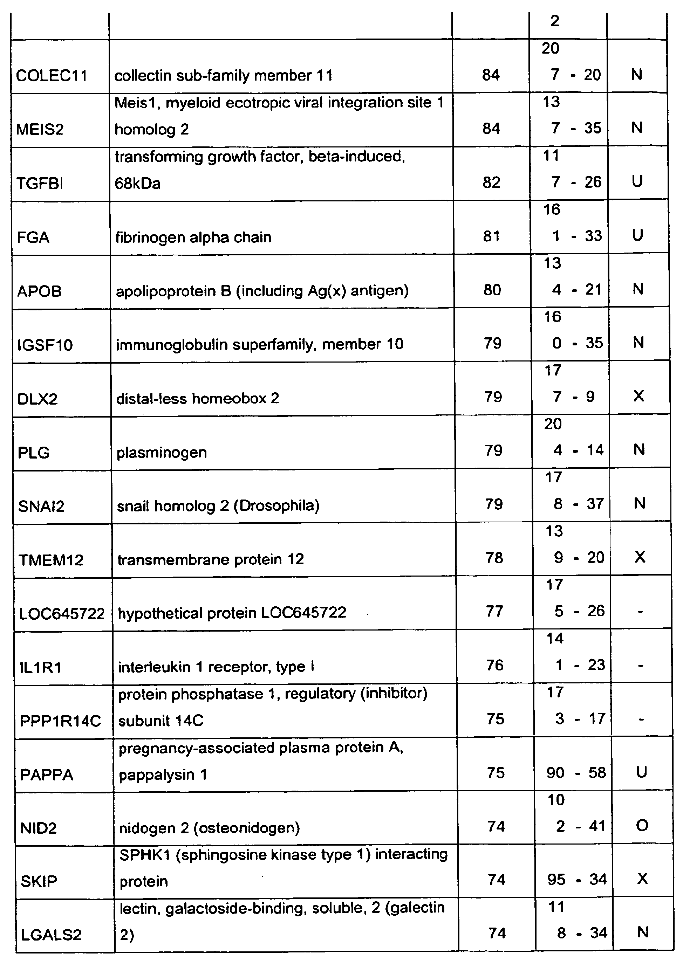

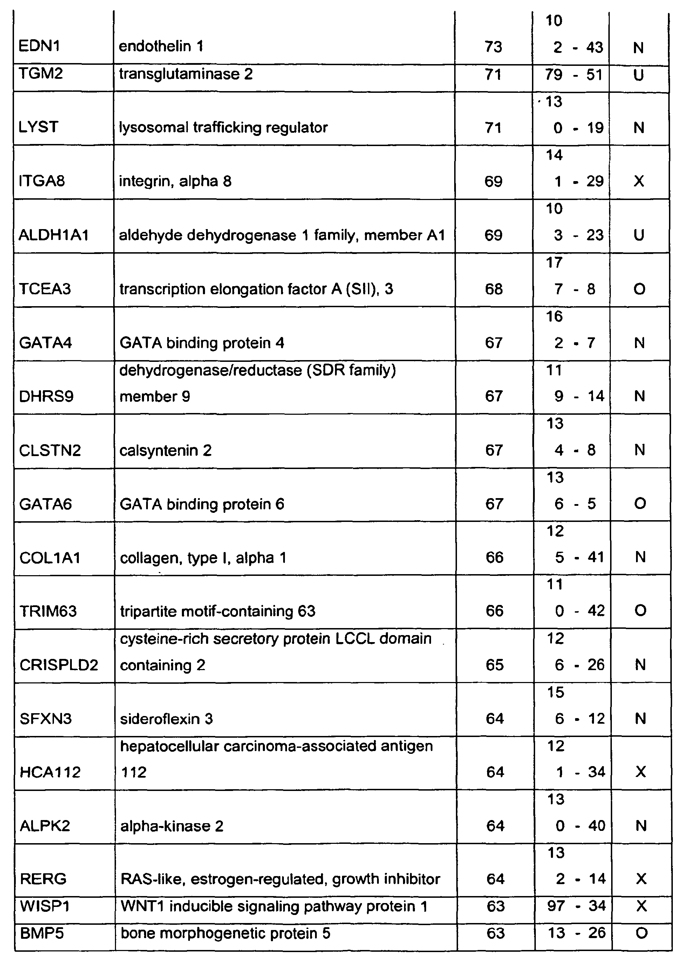

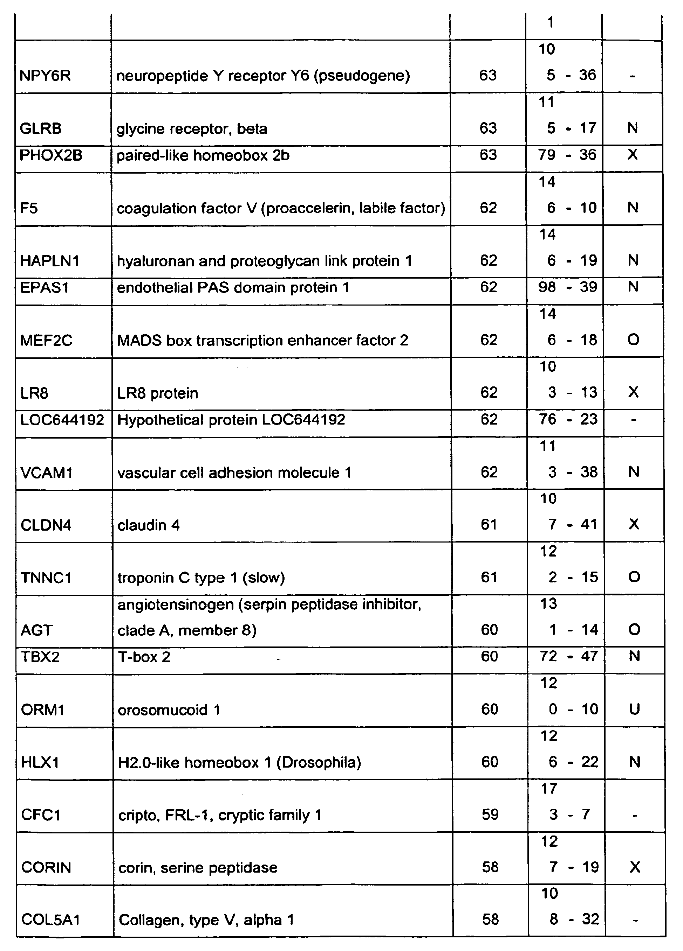

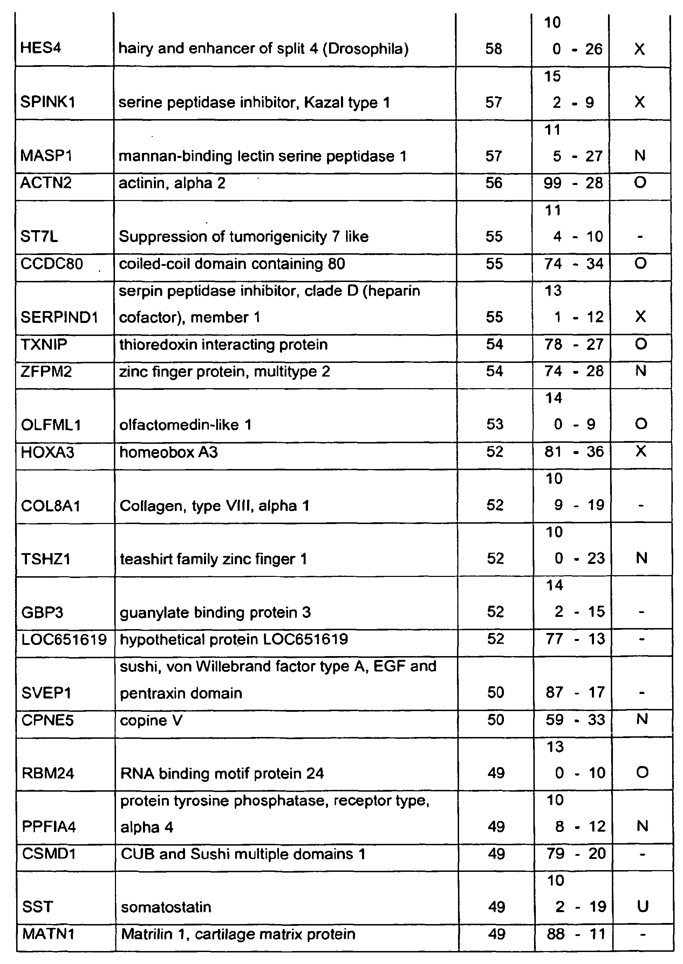

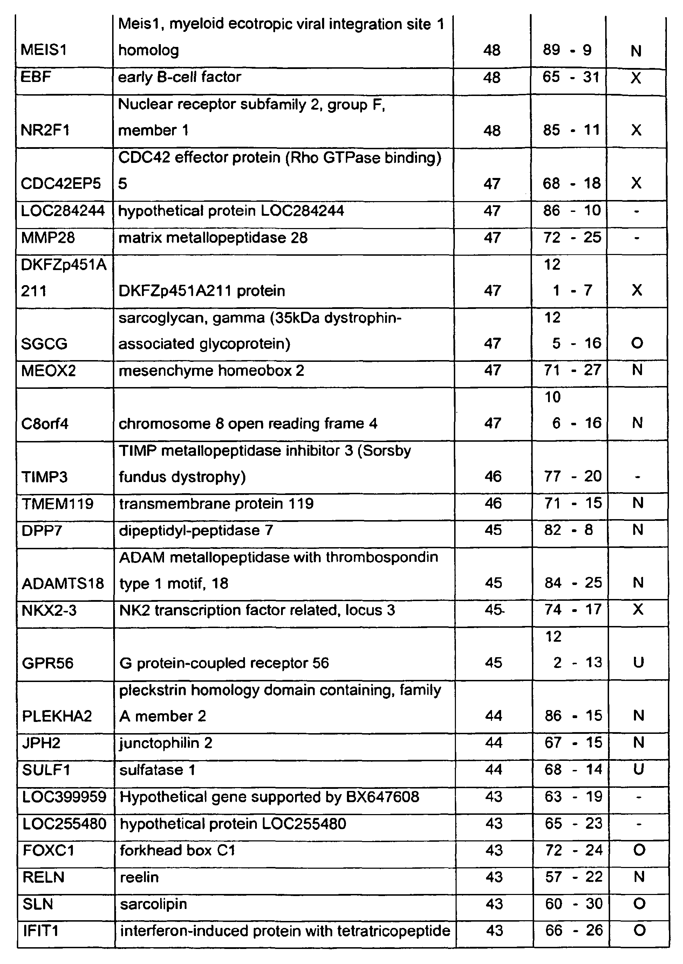

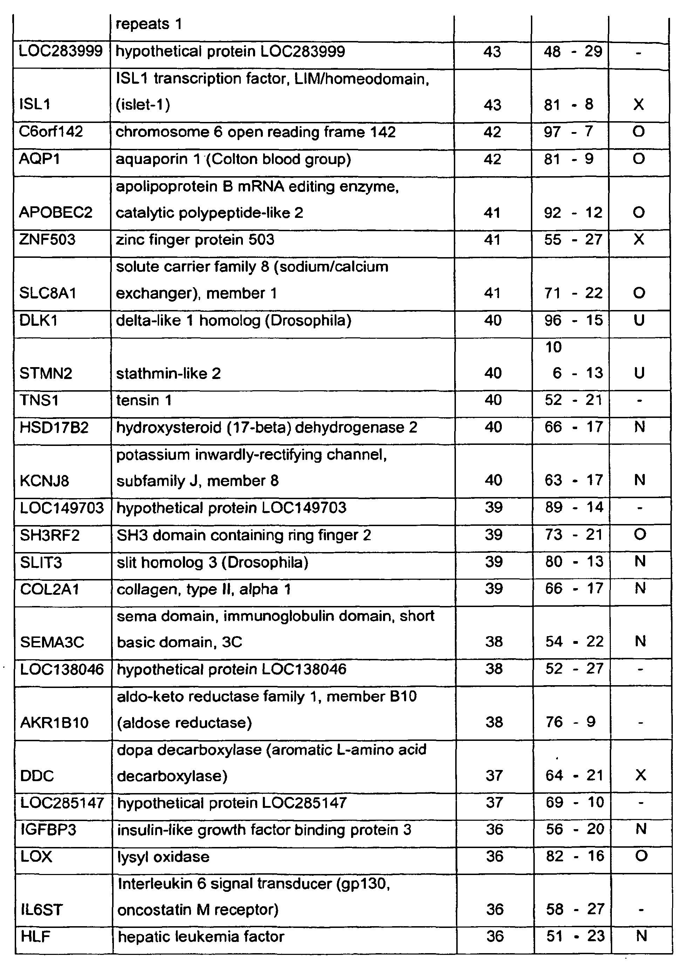

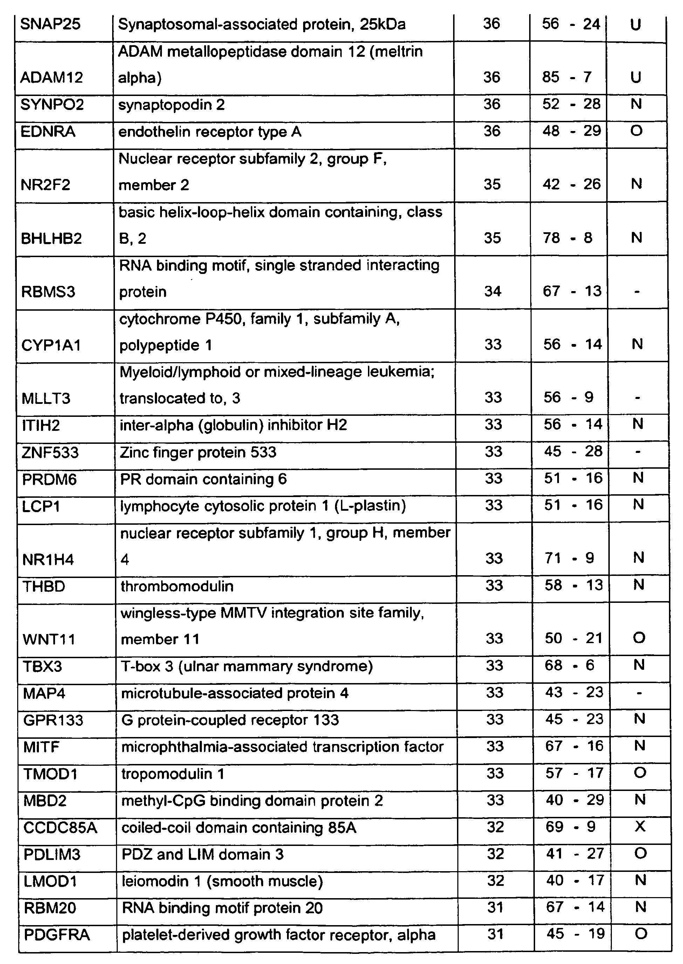

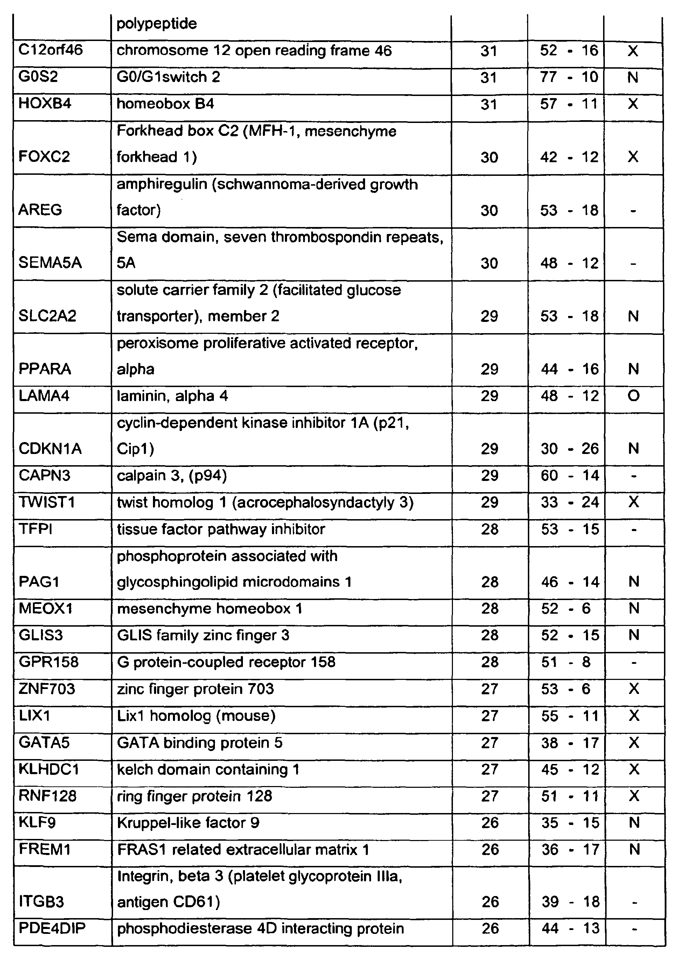

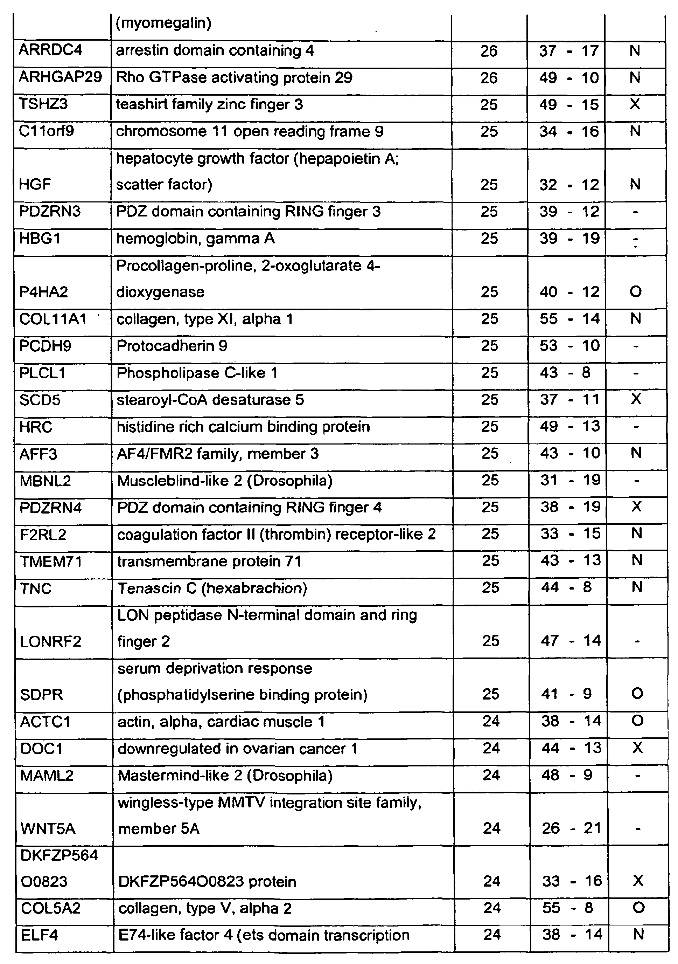

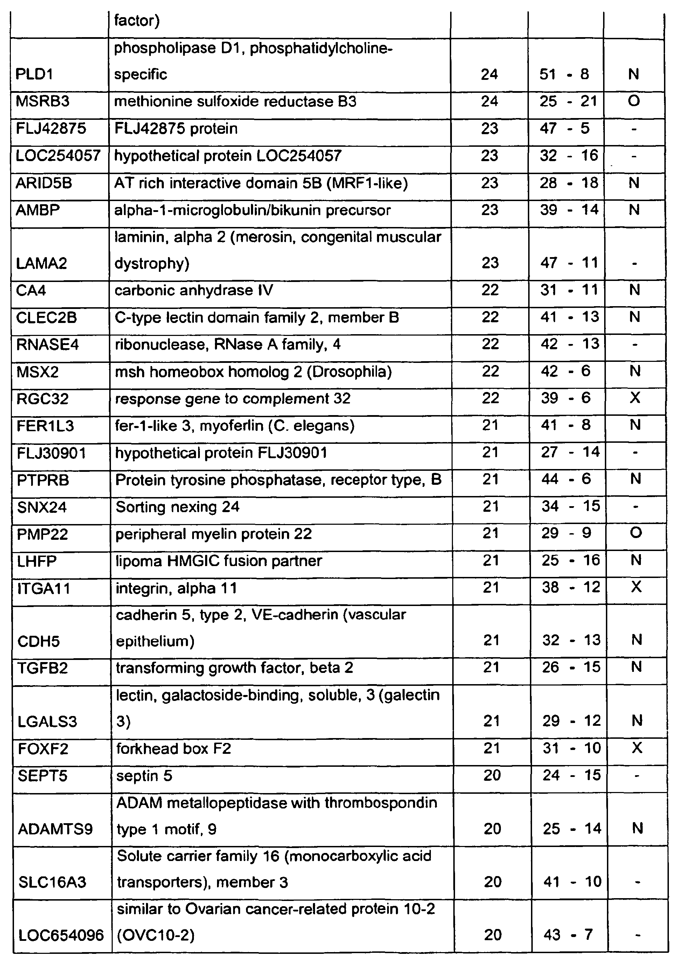

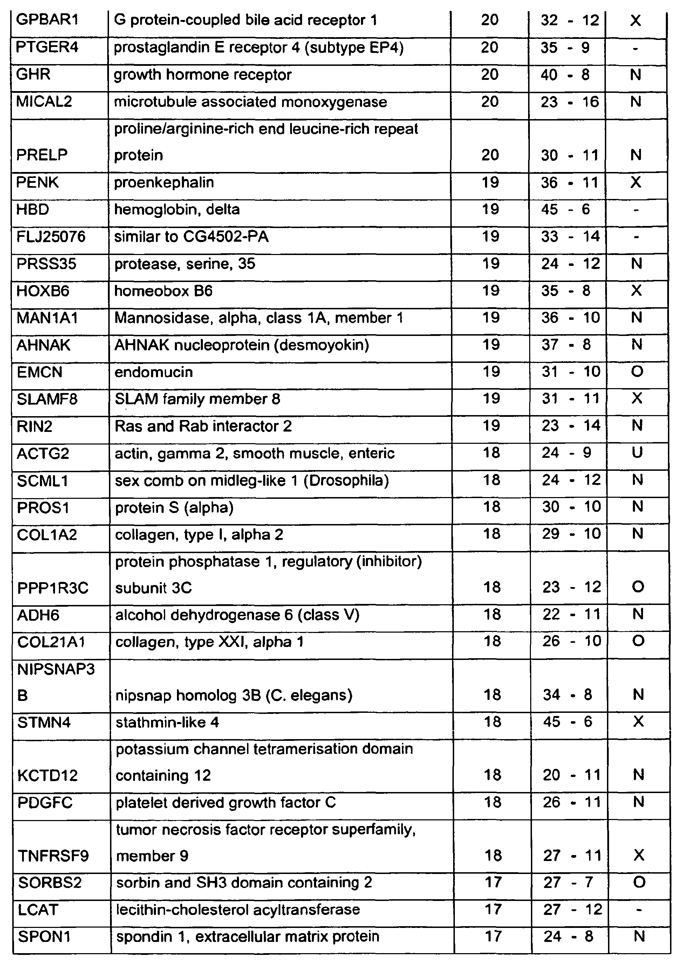

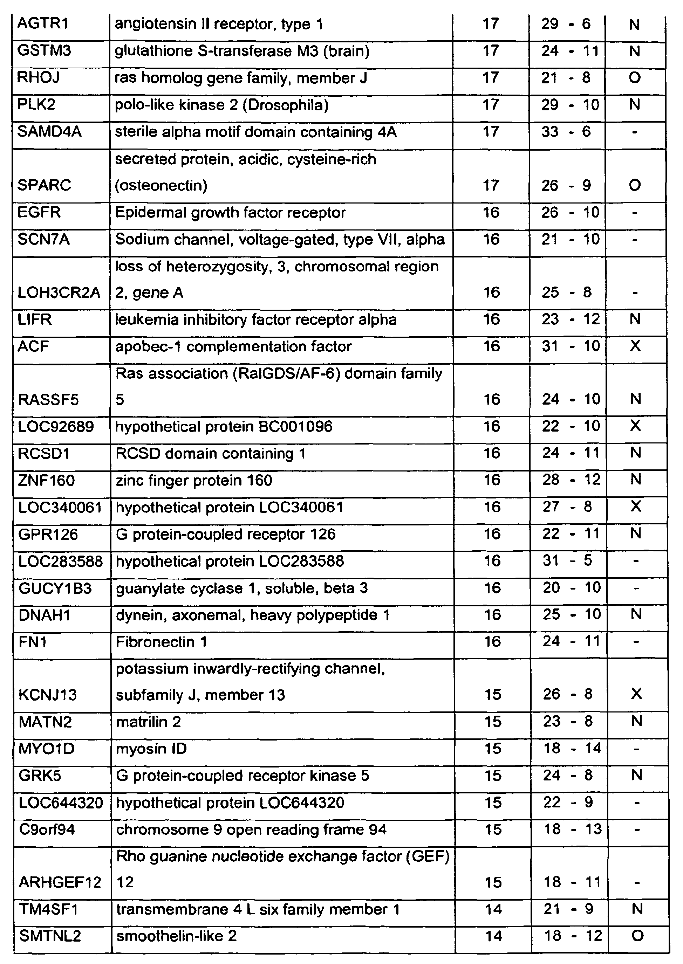

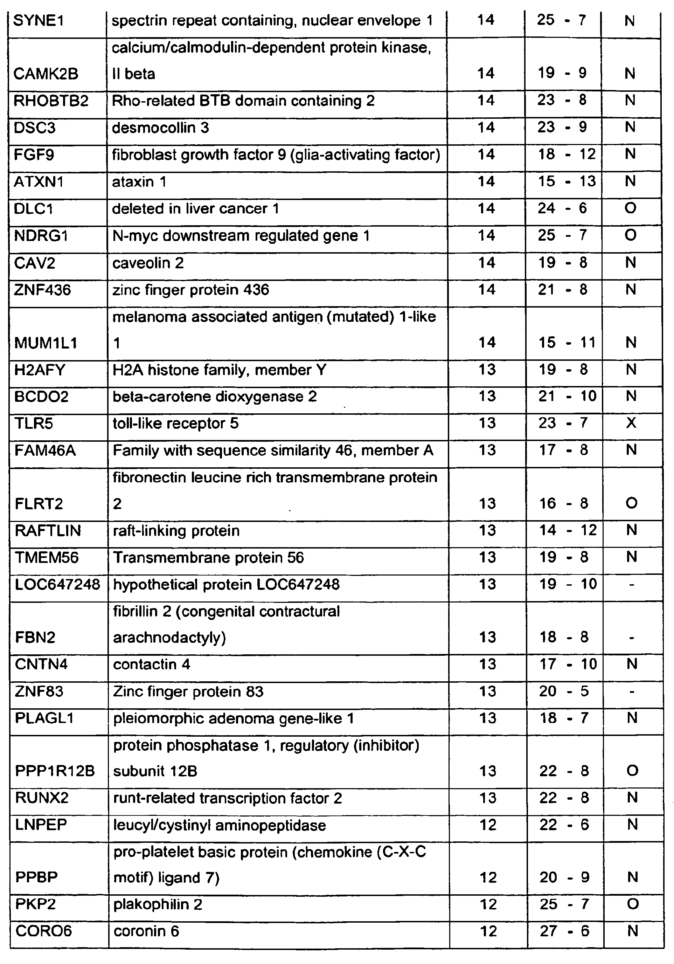

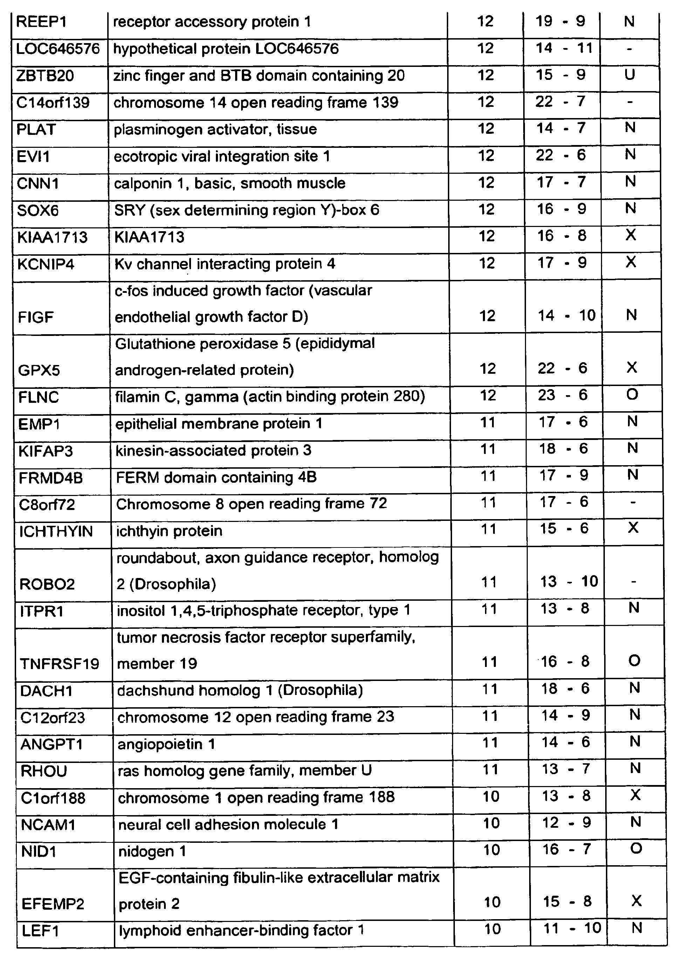

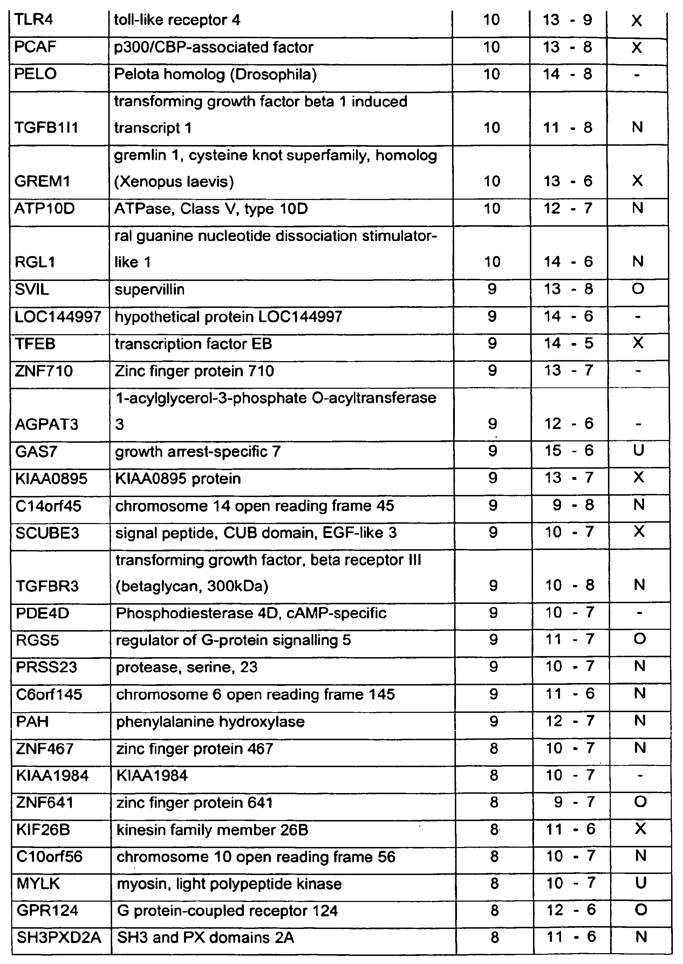

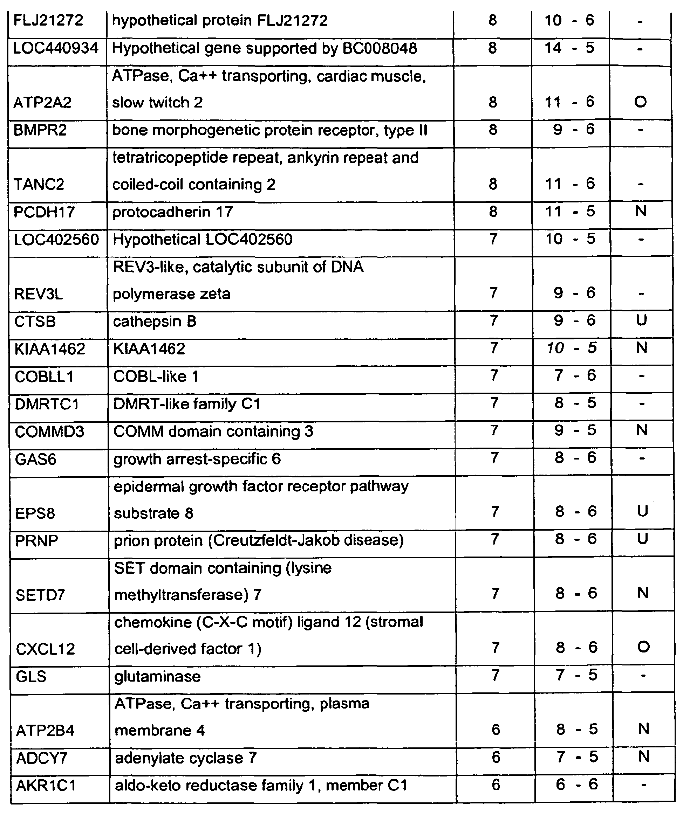

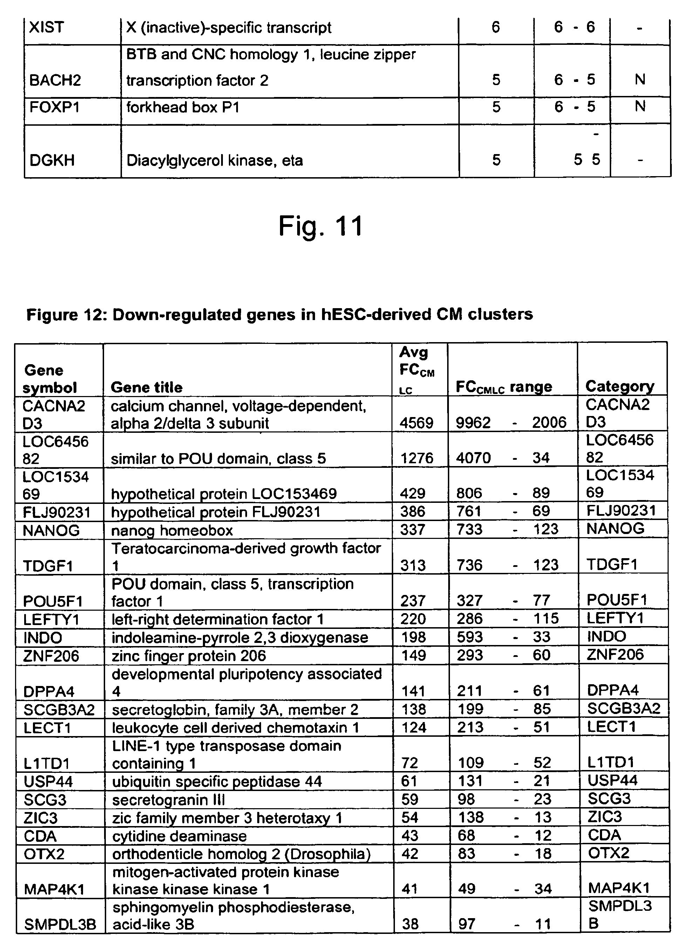

More details are given in Fig. 11 herein. To this end, in a specific embodiment a cluster of the invention comprises cells expressing at least 10 such as at least 50, at least 100, at least 200, at least 300 or at least 400 of the markers mentioned in Fig. 11. More specifically, a cluster of the invention comprises cells expression at least 10 such as at least 20, at least 40, at least 60 or all of the following markers:

| **Gene symbol** | **Gene title** | **Avg FC_{CMLC}** | **FC_{CMLC} range** | **Category** |
|---|---|---|---|---|
| TNNI1 | troponin I type 1 (skeletal, slow) | 1990 | 6078 - 100 | O |
| LUM | lumican | 1375 | 2989 - 365 | O |
| IGFBP7 | insulin-like growth factor binding protein 7 | 1269 | 2781 - 518 | N |
| COL3A1 | collagen, type III, alpha 1 | 1234 | 2187 - 538 | O |
| IGF2 | insulin-like growth factor 2 (somatomedin A) | 1149 | 1374 - 965 | N |
| NPNT | nephronectin | 1020 | 1590 - 708 | N |
| TF | transferrin | 942 | 1759 - 218 | U |
| MGP | matrix Gla protein | 860 | 2671 - 182 | O |
| FGB | fibrinogen beta chain | 813 | 1425 - 299 | O |
| LDB3 | LIM domain binding 3 | 750 | 2195 - 134 | O |
| CXCL14 | chemokine (C-X-C motif) ligand 14 | 722 | 1617 - 228 | O |
| FGG | fibrinogen gamma chain | 686 | 1708 - 161 | U |
| GABRP | gamma-aminobutyric acid (GABA) A receptor, pi | 653 | 1514 - 158 | X |
| TDO2 | tryptophan 2,3-dioxygenase | 562 | 1116 - 290 | N |
| TTR | transthyretin (prealbumin, amyloidosis type I) | 510 | 1209 - 100 | U |
| TTN | titin | 494 | 1325 - 77 | O |
| DCN | Decorin | 451 | 1066 - 160 | O |
| DNM3 | Dynamin 3 | 448 | 1240 - 132 | - |
| MMP1 | matrix metallopeptidase 1 (interstitial collagenase) | 390 | 633 - 137 | X |
| COL6A3 | collagen, type VI, alpha 3 | 383 | 505 - 137 | N |
| C5orf23 | chromosome 5 open reading frame 23 | 370 | 844 - 38 | N |
| SERPINA1 | Serpin peptidase inhibitor, clade A, member 1 | 365 | 774 - 112 | U |
| AHSG | alpha-2-HS-alycoprotein | 353 | 498 - 248 | N |
| A2M | alpha-2-macroglobulin | 337 | 635 - 66 | N |
| FABP1 | fatty acid binding protein 1, liver | 307 | 828 - 27 | X |
| CLIC5 | chloride intracellular channel 5 | 307 | 503 - 129 | - |
| GUCY1A3 | guanylate cyclase 1, soluble, alpha 3 | 294 | 535 - 126 | N |
| SMYD1 | SET and MYND domain containing 1 | 292 | 631 - 48 | - |
| FLJ21986 | hypothetical protein FLJ21986 | 279 | 445 - 76 | - |
| CMYA1 | cardiomyopathy associated 1 | 253 | 661 - 76 | O |
| HOP | homeodomain-only protein | 235 | 345 - 106 | X |
| RUNX1 | runt-related transcription factor 1 (acute myeloid leukemia 1) | 234 | 447 - 17 | N |
| AFP | alpha-fetoprotein | 221 | 275 - 191 | O |
| MYOCD | myocardin | 218 | 374 - 125 | O |
| TBX5 | T-box 5 | 205 | 275 - 120 | O |
| MYL2 | myosin, light polypeptide 2, regulatory, cardiac, slow | 201 | 539 - 74 | O |
| GPRC5A | G protein-coupled receptor, family C, group 5, member A | 196 | 311 - 32 | - |
| UNC45B | unc-45 homolog B (C. elegans) | 193 | 410 - 85 | O |
| MEP1A | meprin A, alpha (PABA peptide hydrolase) | 191 | 379 - 48 | X |
| WNT2 | wingless-type MMTV integration site family member 2 | 186 | 390 - 90 | X |
| SHOX2 | short stature homeobox 2 | 181 | 421 - 15 | O |
| PITX1 | paired-like homeodomain transcription factor 1 | 172 | 368 - 77 | N |
| HOXB2 | homeobox B2 | 171 | 369 - 9 | X |
| GBP1 | guanylate binding protein 1, interferon-inducible. 67kDa | 170 | 292 - 46 | N |
| SLC40A1 | solute carrier family 40 (iron-regulated transporter), member 1 | 169 | 439 - 10 | N |
| NBLA00301 | Putative protein product of Nbla00301 | 168 | 299 - 41 | - |
| NPPA | natriuretic peptide precursor A | 166 | 291 - 90 | O |
| LMOD2 | leiomodin 2 (cardiac) | 165 | 506 - 12 | - |
| RGS13 | regulator of G-protein signalling 13 | 165 | 191 - 125 | X |
| CD14 | CD14 molecule | 162 | 419 - 30 | N |
| H19 | H19, imprinted maternally expressed untranslated mRNA | 155 | 186 - 132 | - |
| NPR3 | natriuretic peptide receptor C/guanylate cyclase C | 152 | 232 - 51 | N |
| CCDC3 | coiled-coil domain containing 3 | 151 | 285 - 47 | O |
| HMGCS2 | 3-hydroxy-3-methylglutaryl-Coenzyme A synthase 2 | 148 | 206 - 84 | N |
| CFI | complement factor I | 145 | 230 - 61 | N |
| ACTA2 | Actin, alpha 2, smooth muscle, aorta | 144 | 184 - 99 | U |
| LOC400043 | hypothetical gene supported bv BC009385 | 143 | 237 - 72 | - |
| KBTBD10 | kelch repeat and BTB (POZ) domain containing 10 | 142 | 267 - 63 | O |
| LOC255130 | Hypothetical LOC255130 | 141 | 212 - 42 | - |
| TM4SF4 | transmembrane 4 L six family member 4 | 139 | 263 - 56 | X |
| DUSP27 | dual specificity phosphatase 27 (putative) | 138 | 302 - 13 | N |
| ASGR2 | asialoglycoprotein receptor 2 | 137 | 360 - 15 | X |
| C21orf34 | Chromosome 21 open reading frame 34 | 137 | 308 - 21 | - |
| PRRX1 | paired related homeobox 1 | 136 | 290 - 28 | O |
| C4orf26 | chromosome 4 open reading frame 26 | 133 | 269 - 31 | X |
| RGS1 | regulator of G-protein signalling 1 | 131 | 298 - 39 | N |
| WFDC1 | WAP four-disulfide core domain 1 | 131 | 202 - 68 | N |
| BMP4 | bone morphogenetic protein 4 | 130 | 239 - 22 | N |
| CFB | complement factor B | 130 | 200 - 26 | N |
| BNC1 | basonuclin 1 | 128 | 213 - 61 | - |
| ASPN | asporin (LRR class 1) | 127 | 298 - 32 | O |
| ANXA1 | annexin A1 | 127 | 241 - 49 | N |
| POSTN | periostin, osteoblast specific factor | 126 | 184 - 48 | O |
| OSR2 | odd-skipped related 2 (Drosophila) | 124 | 263 - 21 | N |
| APOA2 | apolipoprotein A-II | 123 | 282 - 13 | O |
| PDE1A | phosphodiesterase 1A, calmodulin-dependent | 122 | 175 - 40 | X |
| C1orf105 | chromosome 1 open reading frame 105 | 121 | 272 - 14 | O |
| SLC6A13 | solute carrier family 6 (neurotransmitter transporter), member 13 | 118 | 219 - 16 | - |
| MAB21L2 | mab-21-like 2 (C. elegans) | 118 | 147 - 60 | X |
| FOXF1 | forkhead box F1 | 113 | 151 - 81 | X |
| RGS4 | regulator of G-protein signalling 4 | 112 | 174 - 67 | X |
| C20orf75 | chromosome 20 open reading frame 75 | 105 | 244 - 9 | X |
| VTN | vitronectin | 104 | 256 - 11 | N |
| IGFBP1 | insulin-like growth factor binding protein 1 | 103 | 228 - 44 | X |
| BGN | biglycan | 102 | 313 - 18 | N |
| SULT1E1 | sulfotransferase family 1E, estrogen-preferring, member 1 | 101 | 239 - 18 | O |
| ABCA8 | ATP-binding cassette, sub-family A, member 8 | 101 | 128 - 74 | N |

A suspension or composition of cardiomyocyte-like clusters may contain a mixture of clusters containing nodal-like cells, clusters containing atrial-like cells and clusters containing ventricle-like cells.

One embodiment the invention thus relates to a suspension or composition of cardiomyocyte-like clusters, wherein the ratio between the number of clusters containing nodal-like cells and the number of clusters containing atrial-like cells is in a range of from about 1:100 to about 50:100 such as from about 10:100 to about 40:100, from about 20:100 to about 40:100, from about 30:100 to about 40:100 or about 33:100-34:100.

In another embodiment the invention relates to a suspension or composition of cardiomyocyte-like clusters, wherein the ratio between the number of clusters containing nodal-like cells and the number of clusters containing ventricle-like cells is in a range of from about 1:100 to about 80:100 such as from about 10:100 to about 70:100, from about 30:100 to about 70:100, from about 45:100 to about 55:100 or about 50:100.

In a further embodiment the invention relates to a suspension or composition of cardiomyocyte-like clusters, wherein the ratio between the number of clusters containing ventricle-like cells and the number of clusters containing atrial-like cells is in a range of from about 1:100 to about 90:100 such as from about 40:100 to about 80:100, from about 50:100 to about 75:100 or about 66:100.

In a still further embodiment the invention relates to a suspension or composition of cardiomyocyte-like clusters, wherein the ratio between the clusters containing nodal-like cells, the clusters containing atrial-like cells and the clusters containing ventricle-like cells is 17:50:33.

### Compositions comprising one or more cluster

In another aspect of the invention relates to a cluster-containing composition, i.e. one or more clusters of cardiomyocyte-like cells as described above may be in a composition comprising a carrier, such as e.g. an aqueous medium. The composition may further contain one or more additives including one or more cryoprotectants, one or more stabilizers and/or one or more viscosity-adjusting agents. The composition may be in liquid or frozen form.

The one or more cryoprotectants may be selected from the group consisting of ethylene glycol, propylene glycol, dimethylsulfoxide, glycerol, propanediol, and methyl pentanediol, and/or mixtures thereof. The additive may also be a sugar or sugar alcohol including sucrose, trehalose, maltose or lactose.

In a special embodiment, the cryoprotectant is trehalose in a concentration from about 0.02 M to about 1 M, such as, e.g., from about 0.05 M to about 0.9 M, from about 0.1 M to about 0.8 M, from about 0.15 M to about 0.7 M, from about 0.2 M to about 0.65 M, from about 0.25 M to about 0.6 M. The concentration of trehalose may preferably be about 0.3 M.

In a another special embodiment the cryoprotectant is sucrose in a concentration from about 0.02 M to about 1 M, such as, e.g., from about 0.05 M to about 0.9 M, from about 0.1 M to about 0.8 M, from about 0.15 M to about 0.7 M, from about 0.2 M to about 0.65 M, from about 0.25 M to about 0.6 M. The concentration of sucrose may preferably be about 0.3 M.

In a further embodiment, the cryoprotectant may be DMSO in a concentration that is at least 2.5 % v/v, such as e.g. from about 2.5% to about 40% v/v, from about 5% to about 35% v/v, from about 7% to about 30% v/v, from about 7% to about 25% v/v, from about 7% to about 20% v/v, from about 15% to about 25% v/v, or from about 5% to about 15% v/v.

In a still further embodiment, the cryoprotectant may be ethylene glycol in a concentration that is at least 2.5 % v/v, such as e.g. from about 2.5% to about 30% v/v, from about 5% to about 25% v/v, from about 5% to about 20% v/v, from about 10% to about 20% v/v, from about 7% to about 10% v/v, or from about 2.5% to about 5% v/v.

The composition may further comprise a viscosity-adjusting agent selected from the group consisting of Ficoll, Percoll, hyaluronic acid, albumin, polyvinyl pyrrolidone, alginic acid, gelatin and glycerol.

In a special embodiment, the viscosity-adjusting agent may be Ficoll in a concentration at the most about 150 mg/ml, such as, e.g., at the most about 100 mg/ml, at the most about 50 mg/ml, at the most about 25 mg/ml, at the most about 15 mg/ml or at the most about 10 mg/ml.

A further component of the composition may be one or more growth factors, e.g. basic fibroblast growth factor (bFGF) in a concentration of about 2 ng/ml, such as about 3 ng/ml, about 4, ng/ml, about 5 ng/ml, about 6 ng/ml or more.

The composition may also comprise one or more essential and/or non-essential amino acids in appropriate concentrations: Examples of essential amino acids are e.g. L-Alanine, L-Arginine, L-Asparagine, L-Aspartic acid, L-Cysteine, L-Glutamic acid, L-Glutamine, Glycine, L-Histidine, L-Isoleucine, L-Leucine, L-Lysine, L-Methionine, L-Phenylalanine, L-Proline, L-Serine, L-Threonine, L-Tryptophan, L-Tyrosine, L-Valine in concentrations raging from about 0.01 mM, such about 0.05 mM, about 0.1 mM, about 0.15 mM, about 0.2 mM or more.

The composition may further include one or more inorganic salts such as Calcium chloride (Anhydrous),Cupric sulfate (CuSO4-5H2O), Ferric sulfate (FeSO4-7H2O), Potassium chloride (KCI), Magnesium chloride (Anhydrous), Sodium chloride (NaCl), Sodium bicarbonate (NaHCO3), Sodium phosphate, dibas (Anhydrous), Zinc sulfate (ZnSO4-7H2O), and/or one vitamins, such as Biotin, D-Calcium pantothenate, Choline chloride, Folic acid, i-Inositol, Niacinamide, Pyridoxine hydrochloride, Riboflavin, Thiamine hydrochloride or Vitamin B12.

The above-mentioned substances may also be available in a commercial mixture e.g., DMEM (Dulbecco's Modified Eagle's Medium), MEM media or RPMI media.

The CMLC were vitrified in closed straws as described in r WO2004 098285 and stored in liquid N₂. A sterile filtered vitrification solution including vitro-PBS (Vitrolife AB) supplemented with 10% ethyleneglycol and 10% DMSO was used, as well as vitro-PBS (Vitrolife AB) supplemented with 0.3 M trehalose, 20% ethyleneglycol and 20% DMSO. The cells were recovered after thawing in culture medium (Knock Out DMEM supplemented with 20% FBS, 1% penicillin-streptomycin, 1% Glutamax, 0.5 mmol/l β-mercaptoethanol, and 1% non-essential amino acids).

In a specific embodiment the CMLC normally has a size of from about 20 µm to about 30 µm. Moreover, 50-80 % of the cells in the cluster express cardiac markers.

A suitable product for shipping contains one or more clusters in KnockOut DMEM medium supplemented with 2-20% FBS, 1% penicillin-streptomycin, 1% Glutamax, 0.5 mmol/l β-mercaptoethanol and 1% non-essential amino acids (all from Invitrogen, Carlsbad, California).

A composition according to the present invention provides a composition wherein the cluster retains at least 95% of its described characteristics after storage at a temperature of at least-80 °C for at least 2 years. Further, at least about 50% or more such as, e.g., about 55% or more, about 60% or more, about 65% or more, about 70% or more, about 75% or more, about 80% or more, about 85% or more, about 90% or more or about 95% or more of the cells are viable after storage of the composition at a temperature of at least -80 °C for at least 2 years.

### Kit

Disclosed herein is a kit for use in testing of cardiotoxicity of a specific substance, wherein the kit comprising
i) one or more cluster of cardiomyocyte-like cells or a composition as described above, and
ii) specific instructions for use of the cluster or composition, whichever is relevant.

In a specific disclosure the kit may comprise a composition of cardiomyocyte-like clusters of nodal-like cells and/or atrial-like cells and/or ventricle-like cells as defined above.

The kit may further comprise
iii) a medium into which the specific substance is dispersed before use of the kit.

In a further disclosure a kit for use in *in vitro* testing during drug discovery of a specific substance is described, wherein the kit comprises
i) one or more clusters of cardiomyocyte-like cells or a composition as described above,
ii) specific instructions for use of the cluster or composition, whichever is relevant.

In a specific disclosure the kit may comprise a composition of cardiomyocyte-like clusters of nodal-like cells and/or atrial-like cells and/or ventricle-like cells as defined above.

The kit may further comprise
iii) a medium into which the specific substance is dispersed before use of the kit.

In a still further disclosure a kit for regenerative medicine is described comprising
i) a composition and/or one or more cardiomyocyte-like cell clusters as described above,and
ii) tools for administration of the composition or the cells to a patient such as, e.g., the cells in an administrative form, such as in a ready-to-use syringe.

In a specific disclosure the kit may comprise a composition of cardiomyocyte-like clusters of nodal-like cells and/or atrial-like cells and/or ventricle-like cells as defined above.

The cryopreservation of the cardiomyocyte-like cell clusters may be performed by using a slow freezing method or by vitrification, using open or closed pulled straws.

### Vitrification solutions

At least one of the vitrification solutions (the first and the second solution) may contain one or more cryoprotectants or mixtures of cryoprotectants. Non-toxic cryoprotectants are of course preferable. Cryoprotectants help minimizing shrinking by reducing the mole fraction of other solutes remaining in the non-frozen water. They inhibit the formation of crystalline ice, and thus depress the freezing point of the water. They may also prevent protein denaturation by hydrogen binding with bound water. As cells cool, solvent water converts to extracellular ice, and the increasing extracellular concentration of non-permeating electrolyte or non-electrolytes damages the cells. When treated with a cryoprotectant, cells do not reach the salt concentrations of non-treated cells until they reach much lower temperatures. Chemical reactions proceed very slowly at such low temperatures and consequently cellular damage is minimized. Usually it is better to use a combination of cryoprotectants since there might be differences between different types. The cryoprotectants may also function as osmotically active agents. Suitable cryoprotectants can be selected from the group consisting of ethylene glycol, propylene glycol, dimethylsulfoxide, glycerol, propanediol, sugars including sucrose, trehalose, maltose, lactose and methyl pentanediol.

A vitrification procedure may comprise the following steps,
i) transfer of the cardiomyocyte-like clusters to a first solution,
ii) optionally incubation of cardiomyocyte-like clusters in the first solution,
iii) transfer the cardiomyocyte-like clusters obtained in step i) or ii) to a second solution,
iv) optionally incubation of the cardiomyocyte-like clusters in the second solution,
v) transfer of the cells obtained from step iii) or iv) into one or more, open, closed at least on one side or fully closed straws, and
vi) vitrification of the one or more open, closed at least on one side, or fully closed straws.

The concentration of the individual agents contained in the first and or the second solution is normally in a range of 5-50% v/v such as, e.g. from about 5% to about 40% v/v such as e.g. from about 5% to about 25% v/v (first solution) and from about 5% to about 30% v/v (second solution). Normally, the total concentration (i.e. calculated as v/v, w/v or M) of the cryoprotectant in the second solution is larger than that in the first solution. The first and the second solution may contain one or more cryoprotectants that are the same or different. The concentration of the one or more cryoprotectants in the first and the second solution can be the same or different, and normally the total concentration of the cryoprotectant in the second solution is larger than that in the first solution.

In a specific disclosure, the cryoprotectant is trehalose. The concentration of trehalose contained in the first and/or the second solution is normally within a range from about 0.02 M to about 1 M, such as, e.g., from about 0.05 M to about 0.9 M, from about 0.1 M to about 0.8 M, from about 0.2 M to about 0.7 M, from about 0.3 M to about 0.65 M, from about 0.4 M to about 0.6 M, from about 0.45 M to about 0.55 M. Usually, sucrose is used in similar applications. Trehalose is a unique, naturally occurring disaccharide and is found in hundreds of plants and animals. Trehalose is an important source of energy and has been shown to be a primary factor in stabilization of organisms during time of freezing. It has been shown that trehalose can depress the phase transition temperature of membranes so that they remain in the liquid-crystal state even when dry. Without being bound to any theory, it is hypothesized that this prevents membrane leakage during rehydration, thereby preserving cellular viability. With respect to proteins, trehalose has been shown to inhibit protein denaturation by exclusion of water from the protein surface when the cells are in the hydrated state.

In another disclosure, the cryoprotectant is sucrose. The concentration of sucrose contained in the first and/or the second solution is normally within a range from about 0.02 M to about 1 M, such as, e.g., from about 0.05 M to about 0.9 M, from about 0.1 M to about 0.8 M, from about 0.2 M to about 0.7 M, from about 0.3 M to about 0.65 M, from about 0.4 M to about 0.6 M, from about 0.45 M to about 0.55 M.

In yet another disclosure, at least one of the first and second solutions comprises a cryoprotectant.

At least one of the first and the second solution may comprise a viscosity-adjusting agent. Suitable viscosity-adjusting agent for use in the present context may be selected from the group consisting of Ficoll, Percoll, hyaluronic acid, albumin, polyvinyl pyrrolidone, alginic acid, gelatin and glycerol. The first and the second solution may contain one or more viscosity-adjusting agents that are the same or different. The concentration of the one or more viscosity-adjusting agents in the first and the second solution may be the same or different.

In a specific disclosure, the viscosity-adjusting agent is Ficoll. The concentration of Ficoll contained in the first and/or the second solution is at the most about 150 mg/ml, such as, e.g., at the most about 100 mg/ml, at the most about 50 mg/ml, at the most about 25 mg/ml, at the most about 15 mg/ml or at the most about 10 mg/ml.

At least one of the first and second solutions may be an aqueous solution.

In one embodiment of the invention, xenofree hBS cell lines and culture methods are applied for therapeutic applications of the cardiomyocyte-like cell clusters.

In one embodiment of the invention, the hBS cell line constitutes a trisomic cell line.

### Improved yield of CMLC from hBS cells

Another aspect the present invention relates to a method for preparing CMLCs from hBS cells. The novel method has shown to be advantageous compared with prior art method using EB formation as the yield of beating clusters are markedly improved. The new method is also more suitable for large scale production. The cell lines used in this method may be obtained from a commercial available cell line.

Thus, the present invention provides a method for the preparation of a cluster comprising cardiomyocyte-like cells, the method comprising the steps of:
i) suspending and dissociating undifferentiated hBS cells in a culture medium, wherein said medium is a cell culture base medium, such as Knock Out Dulbecco Modified Eagles Medium (DMEM) or Modified Eagle Medium (MEM), optionally supplemented with one or more of serum, such as fetal bovine serum, fetal calf serum, human serum or serum replacement, penicillin-streptomycin, GlutaMAX™ -mercaptoethanol and non-essential amino acids,
ii) subjecting the thus dissociated aggregates to forced aggregation,
iii) incubating the thus forced aggregated cell aggregates in said culture medium optionally comprising one of more growth factors to obtain one or more 3D structures,
iv) transferring one or more 3D structures to one or more plates and incubating the 3D structures in said culture medium optionally comprising one or more growth factors to develop them into one or more clusters comprising contracting cells,
   wherein said medium used in step iii) and/or iv) comprises a member GSK-3 inhibitors, Activin A as a member from the transforming growth factor beta superfamily where the concentration of Activin A supplemented to the culture medium is 5-40 ng/ml, and bFGF as a member from the fibroblast growth factor family where the concentration of bFGF supplemented to the culture medium is 5-40 ng/ml.

The method may also comprise a further step of isolating the one or more clusters by removing the one or more clusters from said plate.
In one embodiment, the method for obtaining cardiomyocyte-like clusters, comprises the steps of
1) obtaining a suspension of undifferentiated hBS cells,
2) resuspending said cell suspension in an appropriate medium, optionally containing one or more members from the transforming growth factor beta superfamily and/or one or more members from the fibroblast growth factor family,
3) dissociating the cells in said cell suspension into aggregates,
4) transferring the cell suspension from step 2 or 3 into appropriate culture dish(es), and
5) allowing the cells to develop into cardiomyocyte-like cell clusters.

In the event that these cell lines are propagated on feeder layers, the cells may be detached from the feeder layer by use of mechanical or, preferably, enzymatic treatment, whereby a suspension of primarily undifferentiated hBS cells is obtained. The enzymatic treatment may be performed by use of a proteolytic enzyme, such as a collagenase, for an appropriate time, e.g. 2-20 minutes, 5-15 minutes or 10-15 minutes. However, it is envisaged that any treatment of the cells to obtain a cell suspension can be used in the present invention.

It is envisaged that the cells obtained in the above-mentioned step 1 are mainly undifferentiated, meaning that from about 70% or more, such as e.g. 80 % or more, 85% or more, 90 % or more, 95% or more, or 99% or more of the cells are undifferentiated.

The cell suspension may thereafter be resuspended in an appropriate media which supports the cells, this media can for example be Knock Out DMEM supplemented with FBS (preferably in the range 15-25%, such as 20%). The culture medium can, at various time points during the experiments, be supplemented with different factors such as protein growth factors, chemical compounds, minerals, or other signalling molecules. Examples of such factors are BMP-2, BMP-4, BMP-5, TGF-β1, Activin A, Growth hormone, LIF, and PDGF. It is important to note that the suspension step is relatively short and does not allow for embryoid bodies to develop. In the present method the suspension step is just a transfer step. The resuspended cells are thereafter further dissociated into small aggregates of undifferentiated cells. This dissociation can be performed manually, e.g. by using a pipette, until the cells forms small aggregates of the size 0.2-0.4 mm.

By thereafter transferring said cells to appropriate culture dish(es) in an appropriate medium (e.g. the same as mention above, supplemented with different factors), and incubating the cells for 1-30 days, e.g. 1-15 days, 1-10 days, 1-5 days, or preferably for 3 days, spontaneous clusters of contracting cardiomyocyte-like clusters appeared. The culture dish(es) used may be of any kind that supports the cells, but in a preferred embodiment the culture dish(es) are gelatine coated dishes.

The cell aggregates obtained in step 2) or 3) are subjected to forced aggregation and/or sedimentation before transferring the cells to the culture dish(es) in step 4) and 5). By using this method 3D structures are formed when spun down through forced aggregation. The 3D structures are different from embryoid bodies. The advantage is that a more reliant structure is formed when compared to e.g. embryoid bodies, which have a tendency to fall apart, and therefore the present method gives a higher yield between the number of undifferentiated cells used and the resulting number of cardiomyocyte-like clusters. The method is also advantageous in that it is faster, more reliable and more scalable and useable for automation compared to the prior art EB protocols.

The preferred method for obtaining cardiomyocyte-like clusters, comprises the steps of
1) obtaining a suspension of primarily undifferentiated hBS cells,
2) resuspending said cell suspension in an appropriate medium, optionally, containing one or more members from the transforming growth factor beta superfamily and/or one or more members from the fibroblast growth factor family,
3) dissociating the cells in said cell suspension into aggregates,
4) centrifuge and/or sediment the cells obtained in step 1 or 2,
5) incubate the cells from step 4,
6) transferring the cell suspension from step 2 or 3 into appropriate culture dish(es), and
7) allowing the cells to develop into cardiomyocyte-like cell clusters.

By using forced aggregation approximately 4-6 colonies from step 2) or 3) are transferred to a plate or tube appropriate for centrifugation, e.g. a 96-well v-bottom plate (Corning Incoporated, NY, USA). The cells are then centrifuged for 2-20 minutes at 100-800 x g (e.g. 200-600 x g for 5-10 min or, 300-500 x g for 5-10 min, or at 400 x g for 5 min.). These centrifuged cells may, before the transfer mentioned step 6), further be incubated for 1-10 days (such as 1-7 days, or 1-5 days) for the formation of 3D structures, which structures then are transferred according to step 6) above.

The forced aggregation may additionally be combined, or replaced, by sedimentation of the cell suspension obtained step 2) or 3), for 1-36 hours (such as 2-24 hours or 3-12 hours), before proceeding to step 6).

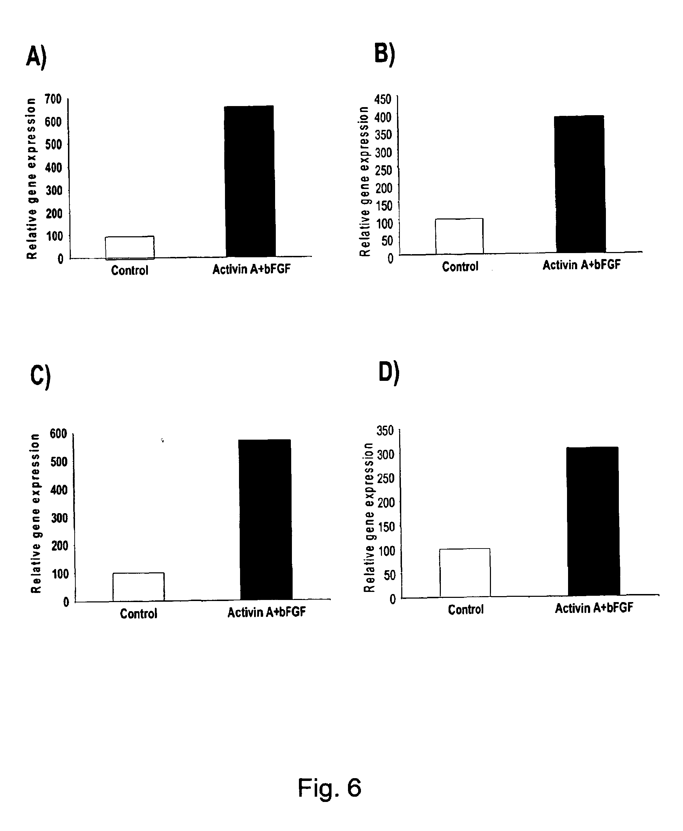

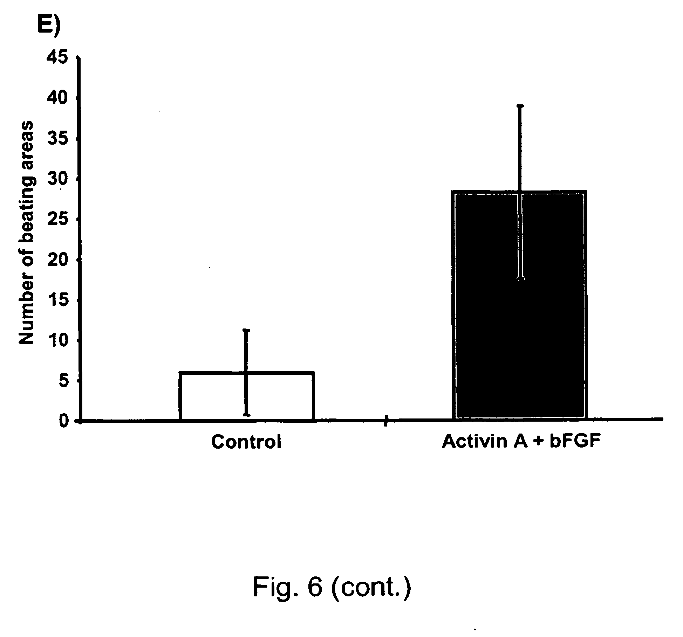

As mentioned above the yield of CMLC from hBS cells can be further improved by supplementing the culture medium used with different factors such as protein growth factors, chemical compounds, minerals, or other signalling molecules. In particular the inventors have found that when the culture medium (e.g. DMEM) comprises a member from the transforming growth factor beta superfamily (e.g. Activin A) and/or a member from the fibroblast growth factor family (e.g. bFGF) in e.g. step 5 and/or step 7 of the preferred method (and/or step 2 of the first embodiment of the method) the yield increases. This yield as shown in figure 6, and in example 9, increases a 4.7 fold compared to controls grown in the same media, but without bFGF or Activin A added.

The concentrations of Activin A supplemented to the culture medium comprises from about 5-40 ng/ml, such as e.g. 8 ng/ml, 9 ng/ml, 10 ng/ml, 11 ng/ml, or 12 ng/ml; and the concentration of bFGF supplemented to the culture medium comprises from about 5-40 ng/ml, such as e.g. 10 ng/ml, 11 ng/ml, 12 ng/ml, 13 ng/ml or 14 ng/ml.

It has also been shown that a concentration of about 20% FBS (e.g. 15-25%) is advantageously added in step 5 and/or 7 of the preferred method to improve the yield.

In a specific embodiment the method for obtaining cardiomyocyte-like clusters, thus comprises the steps of

| Steps | Embodiment 1 | Embodiment 2 | Embodiment 3 | Embodiment 4 |
|---|---|---|---|---|
| Obtaining a suspension of primarily undifferentiated hBS cells | Detach the cells from the feeder layer by collagenase treatment for 10-15 minutes | Detach the cells from the feeder layer by collagenase treatment for 10-15 minutes | Detach the cells from the feeder layer by collagenase treatment for 10-15 minutes | Detach the cells from the feeder layer by collagenase treatment for 10-15 minutes |
| Resuspending said cell suspension in an appropriate medium | The medium comprises Knock out DMEM with 20% FBS, and optionally containing 5-20 ng/ml bFGF 5-20 ng/ml Activin A | The medium comprises Knock out DMEM with 20% FBS, and optionally containing 8-15 ng/ml bFGF 8-15 ng/ml Activin A | The medium comprises Knock out DMEM with 20% FBS, and optionally containing 10-15 ng/ml bFGF 10-15 ng/ml Activin A | The medium comprises Knock out DMEM with 20% FBS, and optionally containing 12 ng/ml bFGF 10 ng/ml Activin A |
| Dissociating the cells in said cell suspension into aggregates | Mechanically using a pipette to obtain aggregates of 0.2-0.4 mm | Mechanically using a pipette to obtain aggregates of 0.2-0.4 mm | Mechanically using a pipette to obtain aggregates of 0.2-0.4 mm | Mechanically using a pipette to obtain aggregates of 0.2-0.4 mm |
| Centrifugation | 200 x g-800x g for 5-10 minutes of 6-8 colonies per well in a 96-well v-bottom plate | 200 x g-800x g for 5-10 minutes of 6-8 colonies per well in a 96-well v-bottom plate | 200 x g - 800x g for 5-10 minutes of 6-8 colonies per well in a 96-well v- bottom plate | 200 x g - 800x g for 5-10 minutes of 6-8 colonies per well in a 96-well v- bottom plate |
| Incubation for 3D formation | 4-10 days at 37 °C in the same medium supplemented with | 4-10 days at 37 °C in the same medium supplemented with | 4-10 days at 37 °C in the same medium supplemented with | 4-10 days at 37 °C in the same medium supplemented with |
| | 5-20 ng/ml bFGF 5-20 ng/ml Activin A | 8-15 ng/ml bFGF 8-15 ng/ml Activin A | 10-15 ng/ml bFGF 10-15 ng/ml Activin A | 12 ng/ml bFGF 10 ng/ml Activin A |
| Transfer to gelatine coated dishes | Incubation for 1-10 days for the formation of CMLC | Incubation for 1-10 days for the formation of CMLC | Incubation for 1-10 days for the formation of CMLC | Incubation for 1-10 days for the formation of CMLC |

As mentioned the invention may also take place by sedimenting the cells instead of centrifugation. Specific embodiments of the invention thus comprises,

| Steps | Embodiment 1 | Embodiment 2 | Embodiment 3 | Embodiment 4 |
|---|---|---|---|---|
| Obtaining a suspension of primarily undifferentiated hBS cells | Detach the cells from the feeder layer by collagenase treatment for 10-15 minutes | Detach the cells from the feeder layer by collagenase treatment for 10-15 minutes | Detach the cells from the feeder layer by collagenase treatment for 10-15 minutes | Detach the cells from the feeder layer by collagenase treatment for 10-15 minutes |
| Resuspending said cell suspension in an appropriate medium | The medium comprises Knock out DMEM with 20% FBS, and optionally containing 5-20 ng/ml bFGF 5-20 ng/ml Activin A | The medium comprises Knock out DMEM with 20% FBS, and optionally containing 8-15 ng/ml bFGF 8-15 ng/ml Activin A | The medium comprises Knock out DMEM with 20% FBS, and optionally containing 10-15 ng/ml bFGF 10-15 ng/ml Activin A | The medium comprises Knock out DMEM with 20% FBS, and optionally containing 12 ng/ml bFGF 10 ng/ml Activin A |
| Dissociating the cells in said cell suspension into aggregates | Mechanically using a pipette to obtain aggregates of 0.2-0.4 mm | Mechanically using a pipette to obtain aggregates of 0.2-0.4 mm | Mechanically using a pipette to obtain aggregates of 0.2-0.4 mm | Mechanically using a pipette to obtain aggregates of 0.2-0.4 mm |
| Sedimentation | 1-36 hours | 2-24 hours | 2-24 hours | 3-12 hours |
| Incubation for 3D formation | 4-10 days at 37 °C in the same medium supplemented with | 4-10 days at 37 °C in the same medium supplemented with | 4-10 days at 37 °C in the same medium supplemented with | 4-10 days at 37 °C in the same medium supplemented with |
| | 5-20 ng/ml bFGF 5-20 ng/ml Activin A | 8-15 ng/ml bFGF 8-15 ng/ml Activin A | 10-15 ng/ml bFGF 10-15 ng/ml Activin A | 12 ng/ml bFGF 10 ng/ml Activin A |
| Transfer to gelatine coated dishes | Incubation for 1-10 days for the formation of CMLC | Incubation for 1-10 days for the formation of CMLC | Incubation for 1-10 days for the formation of CMLC | Incubation for 1-10 days for the formation of CMLC |

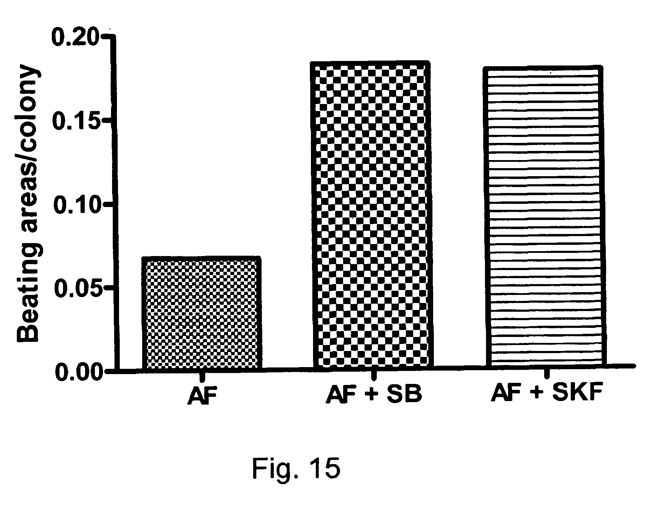

The yield have also been shown to increase (as shown in e.g. figure 15) when the medium used in step 5 and/or 7 of the preferred method is further supplemented (either alone or in combination with Activin A, bFGF and/or FBS) with a member of GSK-3 inhibitors and/or p38 MAP kinase inhibitors such as SB 216763 and SKF-860002, respectively. As shown in Reference example 9 and figure 15, the yield increased by 2-3 fold when Activin A, bFGF and SB 216763 or SKF-860002 are added to the culture medium in step 5 of the preferred method compared to controls where only Activin A and bFGF are added. This medium may be supplemented at any stage of the method, however in one embodiment the medium is supplemented to the cells during their incubation after the centrifugation in step 5) of the preferred method.

The concentration of the GSK-3 inhibitor supplemented to the culture medium may be in the range of from about 1-25 µM, such as e.g. 2-10 µM or 5 µM, and the concentration of the p38 MAP-kinase inhibitor supplemented to the culture medium may be in the range of about 1-25 µM, such as e.g. 2-10 µM or 5 µM.

Further specific embodiments of the invention thus comprises,

| Steps | Embodiment 1 | Embodiment 2 | Embodiment 3 | Embodiment 4 |
|---|---|---|---|---|
| Obtaining a suspension of primarily undifferentiated hBS cells | Detach the cells from the feeder layer by collagenase treatment for 10-15 minutes | Detach the cells from the feeder layer by collagenase treatment for 10-15 minutes | Detach the cells from the feeder layer by collagenase treatment for 10-15 minutes | Detach the cells from the feeder layer by collagenase treatment for 10-15 minutes |
| Resuspending said cell suspension in an appropriate medium | The medium comprises Knock out DMEM with 20% FBS, and optionally containing | The medium comprises Knock out DMEM with 20% FBS, and optionally containing | The medium comprises Knock out DMEM with 20% FBS, and optionally containing | The medium comprises Knock out DMEM with 20% FBS, and optionally containing |
| | | | | |
| | 5-20 ng/ml bFGF 5-20 ng/ml Activin A, and | 8-15 ng/ml bFGF 8-15 ng/ml Activin A | 8-15 ng/ml bFGF 8-15 ng/ml Activin A | 12 ng/ml bFGF 10 ng/ml Activin A |
| | 1-25 µM SB 216763 or 1-25 µM SKF-860002 | 1-10 µM SB 216763 or 1-10 µM SKF-860002 | 5-10 µM SB 216763 or 5-10 µM SKF-860002 | 5 µM SB 216763 or 5 µM SKF-860002 |
| Dissociating the cells in said cell suspension into aggregates | Mechanically using a pipette to obtain aggregates of 0.2-0.4 mm | Mechanically using a pipette to obtain aggregates of 0.2-0.4 mm | Mechanically using a pipette to obtain aggregates of 0.2-0.4 mm | Mechanically using a pipette to obtain aggregates of 0.2-0.4 mm |
| Centrifugation | 200 x g-800x g for 5-10 minutes of 6-8 colonies per well in a 96-well v-bottom plate | 200 x g-800x g for 5-10 minutes of 6-8 colonies per well in a 96-well v-bottom plate | 200 x g - 800x g for 5-10 minutes of 6-8 colonies per well in a 96-well v- bottom plate | 200 x g - 800x g for 5-10 minutes of 6-8 colonies per well in a 96-well v- bottom plate |
| Incubation for 3D formation | 4-10 days at 37 °C in the same medium supplemented with 5-20 ng/ml bFGF 5-20 ng/ml Activin A, and | 4-10 days at 37 °C in the same medium supplemented with 8-15 ng/ml bFGF 8-15 ng/ml Activin A | 4-10 days at 37 °C in the same medium supplemented with 8-15 ng/ml bFGF 8-15 ng/ml Activin A | 4-10 days at 37 °C in the same medium supplemented with 12 ng/ml bFGF 10 ng/ml Activin A |
| | | | | |
| | 1-25 µM SB 216763 or 1-25 µM SKF-860002 | 1-10 µM SB 216763 or 1-10 µM SKF-860002 | 5-10 µM SB 216763 or 5-10 µM SKF-860002 | 5 µM SB 216763 or 5 µM SKF-860002 |
| Transfer to gelatine coated dishes | Incubation for 1-10 days for the formation of CMLC | Incubation for 1-10 days for the formation of CMLC | Incubation for 1-10 days for the formation of CMLC | Incubation for 1-10 days for the formation of CMLC |

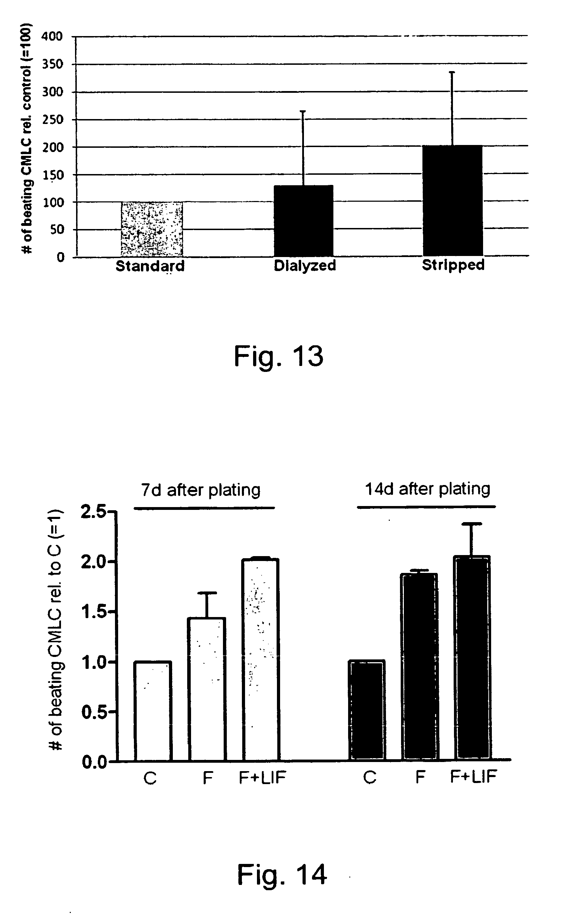

The inventors have also shown that by supplementing the medium in step 7 of the preferred method (or the corresponding step 5 of the first mentioned embodiment of the method) containing Activin A and/or bFGF with LIF, the yield improved by approximately a 2 fold compared to controls that only receive medium, as shown in e.g. figure 14 and example 10. The concentration for LIF comprises between 100-2000 U/ml LIF, such as e.g. between 500-1500 U/ml, or 1000 U/ml. In a specific embodiment, the medium comprising LIF is replaced after 2-8 days, such as after e.g. 4-5 days, with a medium without LIF during the development of cardiomyocyte-like cell clusters in step 7) of the preferred method (or the corresponding step 5 of the first mentioned embodiment of the method).

Further specific embodiments of the invention thus comprises,

| Steps | Embodiment 1 | Embodiment 2 | Embodiment 3 | Embodiment 4 |
|---|---|---|---|---|
| Obtaining a suspension of primarily undifferentiated hBS cells | Detach the cells from the feeder layer by collagenase treatment for 10-15 minutes | Detach the cells from the feeder layer by collagenase treatment for 10-15 minutes | Detach the cells from the feeder layer by collagenase treatment for 10-15 minutes | Detach the cells from the feeder layer by collagenase treatment for 10-15 minutes |
| Resuspending said cell suspension in an appropriate medium | The medium comprises Knock out DMEM with 20% FBS, and optionally | The medium comprises Knock out DMEM with 20% FBS, and optionally | The medium comprises Knock out DMEM with 20% FBS, and optionally | The medium comprises Knock out DMEM with 20% FBS, and optionally |
| | 5-20 ng/ml bFGF 5-20 ng/ml Activin A | 8-15 ng/ml bFGF 8-15 ng/ml Activin A | 8-15 ng/ml bFGF 8-15 ng/ml Activin A | 12 ng/ml bFGF 10 ng/ml Activin A |
| Dissociating the cells in said cell suspension into aggregates | Mechanically using a pipette to obtain aggregates of 0.2-0.4 mm | Mechanically using a pipette to obtain aggregates of 0.2-0.4 mm | Mechanically using a pipette to obtain aggregates of 0.2-0.4 mm | Mechanically using a pipette to obtain aggregates of 0.2-0.4 mm |
| Centrifugation | 200 x g-800x g for 5-10 minutes of 6-8 colonies per well in a 96-well v-bottom plate | 200 x g-800x g for 5-10 minutes of 6-8 colonies per well in a 96-well v-bottom plate | 200 x g - 800x g for 5-10 minutes of 6-8 colonies per well in a 96-well v- bottom plate | 200 x g - 800x g for 5-10 minutes of 6-8 colonies per well in a 96-well v- bottom plate |
| Incubation for 3D formation | 2-6 days at 37 °C with 5-20 ng/ml bFGF and 5-20 ng/ml Activin A | 2-6 days at 37 °C with 8-15 ng/ml bFGFand 8-15 ng/ml Activin A | 3-4 days at 37 °C with 8-15 ng/ml bFGF and 8-15 ng/ml Activin A | 4 days at 37 °C with 12 ng/ml bFGF and 10 ng/ml Activin A |
| Transfer to gelatine coated dishes | 1-4 days at 37 °C with LIF 100-2000 U/ml, | 1-4 days at 37 °C with LIF 500-2000 U/ml, | 1-4 days at 37 °C with LIF 1000 U/ml, thereafter | 1-4 days at 37 °C with LIF 1000 U/ml, thereafter |
| | thereafter replaced with medium without LIF | thereafter replaced with medium without LIF | replaced with medium without LIF | replaced with medium without LIF |
| Incubation | Incubation for 4-14 days for the formation of CMLC | Incubation for 4-14 days for the formation of CMLC | Incubation for 4-14 days for the formation of CMLC | Incubation for 4-14 days for the formation of CMLC |

As mentioned hereinbefore, a cluster according to the invention can be used in *in vitro* studies (e.g. in drug discovery, drug testing, target identification and target validation, e.g. based on electrophysiological measurement on funny channels as described Reference Examples 11 and 14.

### Figure legends

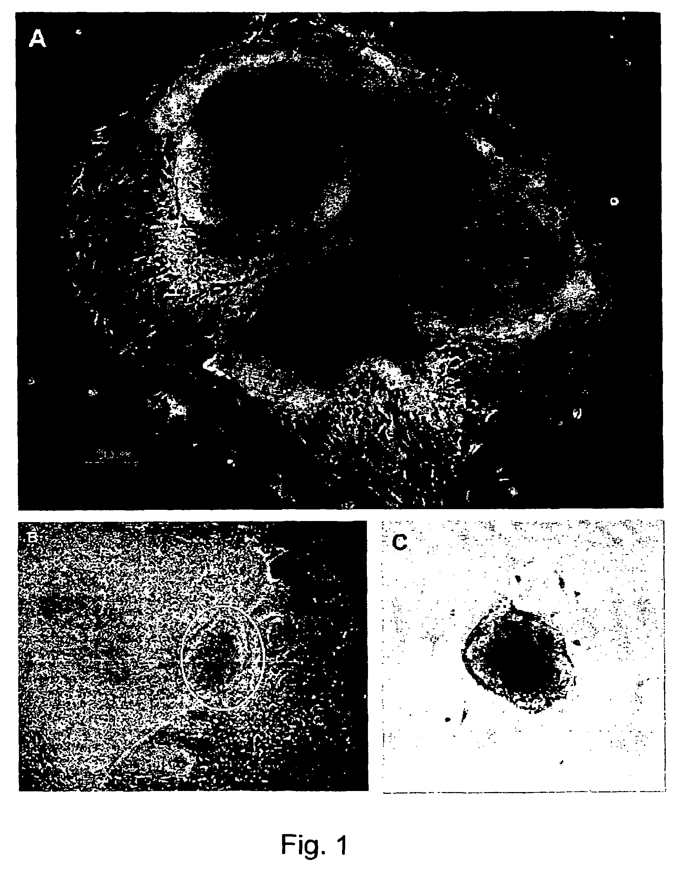

**Figure 1****.** Morphological illustration of CMLC derived from hBS cells. Undifferentiated hBS cells were maintained and differentiated as described in Reference Example 1. The 3D structures were subsequently plated in gelatine coated cell culture dishes leading to attachment and further differentiation of the cells. Panel A shows a 3D cluster 1 day after plating in a culture dish. Panel B shows a spontaneously beating area present in the outgrowth of a 3D structure 3 days after plating (circle). Panel C shows a mechanically isolated beating area sub-cultured for 12 days after isolation in a new culture dish.

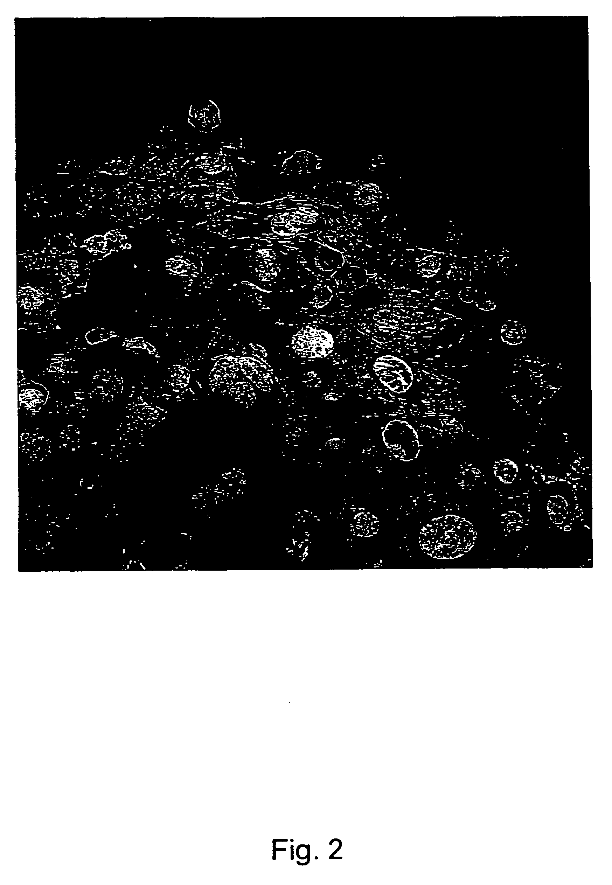

**Figure 2****.** Morphological illustration of CMLC that has been stained to show nuclei (round objects) and cardiac troponin1 (line like structures) (magnification x 600). The CMLC were derived as described in Reference example 1.

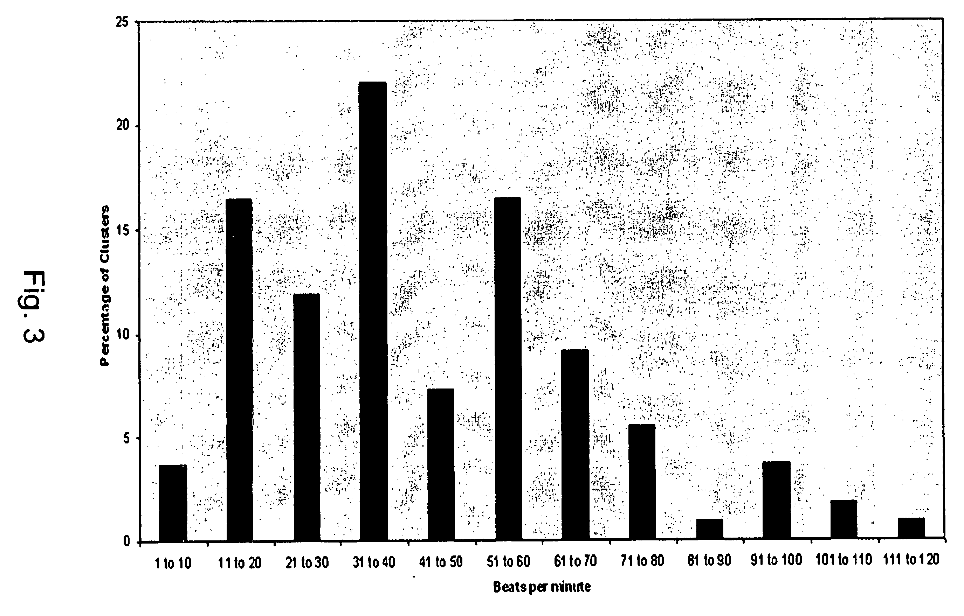

**Figure 3****.** Distribution in beating frequency of CMLC. CMLC were differentiated from hBS cells as described in Reference Example 1, and 109 different spontaneously contracting clusters were evaluated. The figure shows the distribution in beating frequency in the cell preparations.

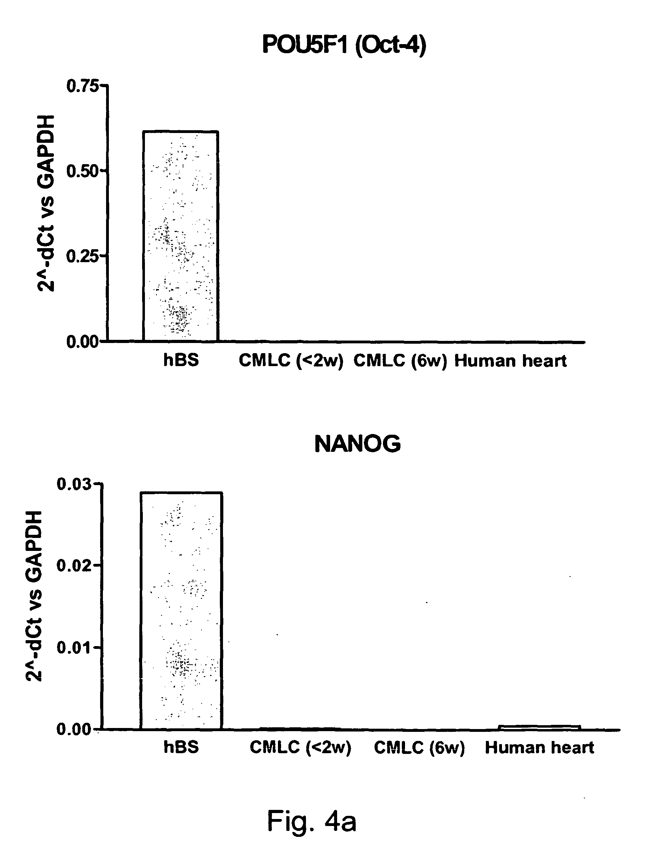

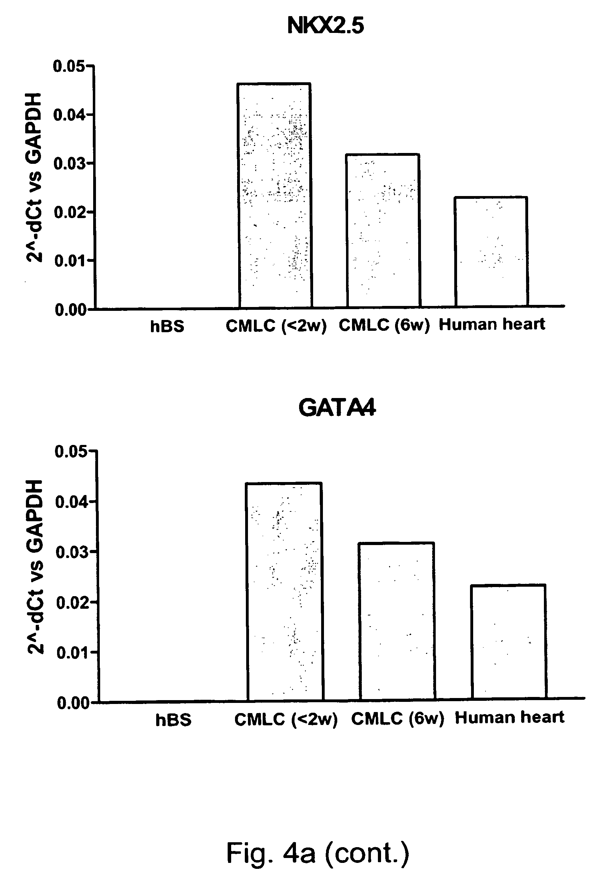

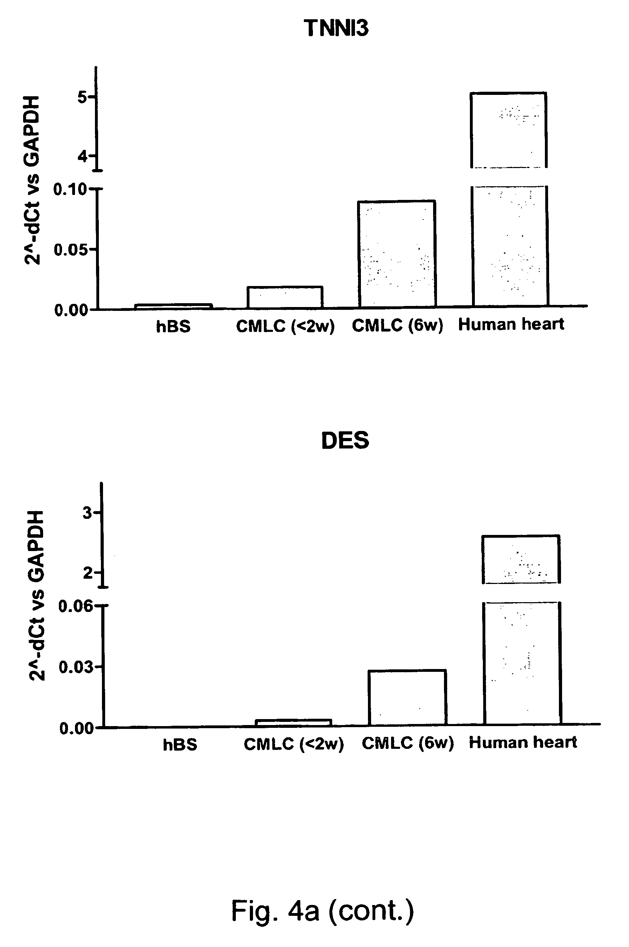

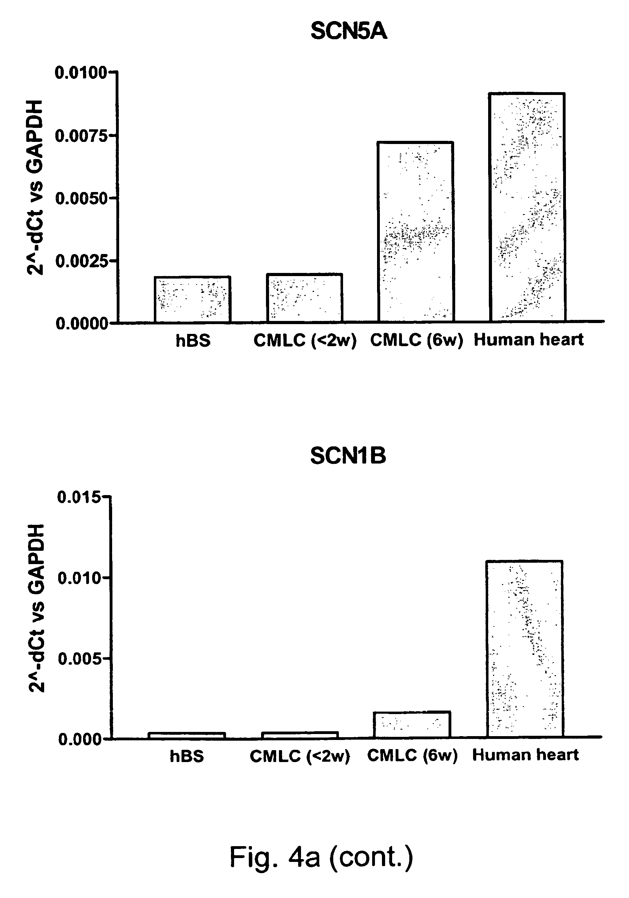

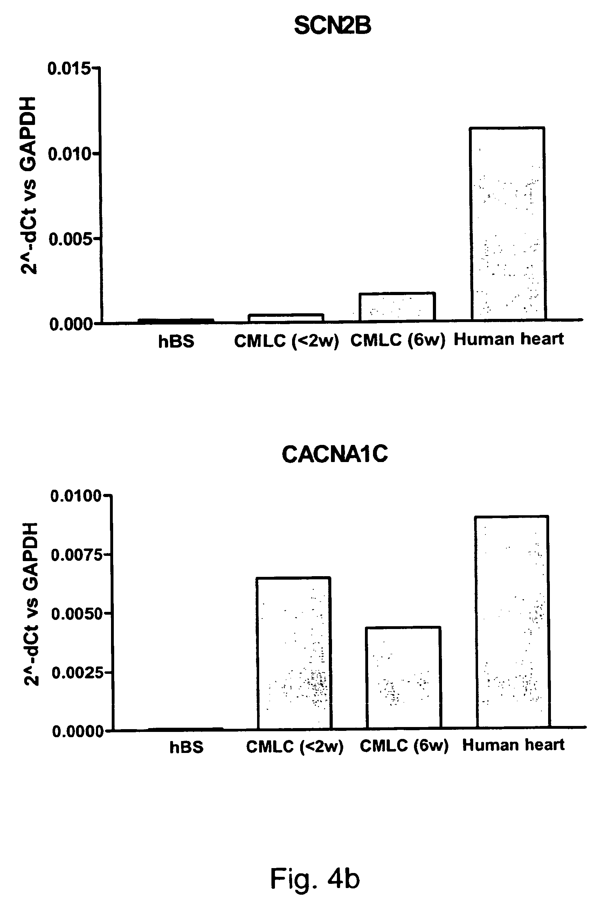

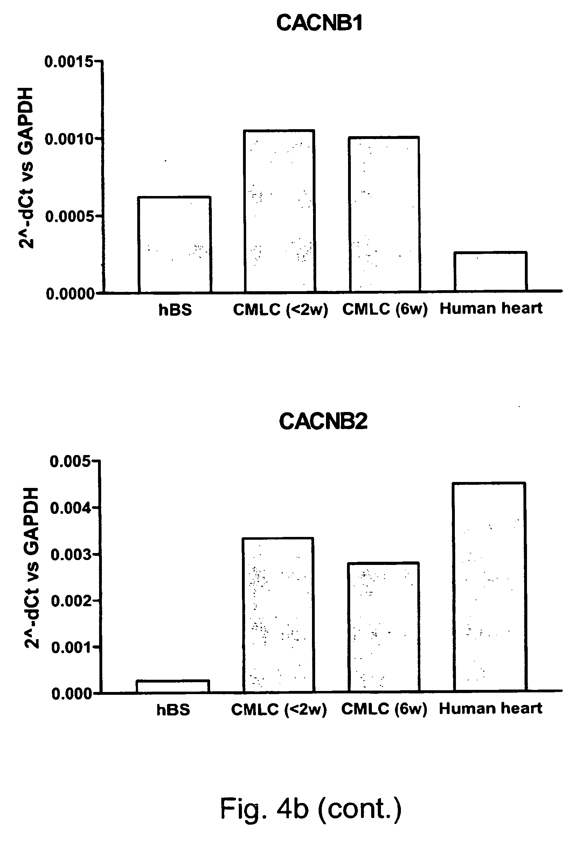

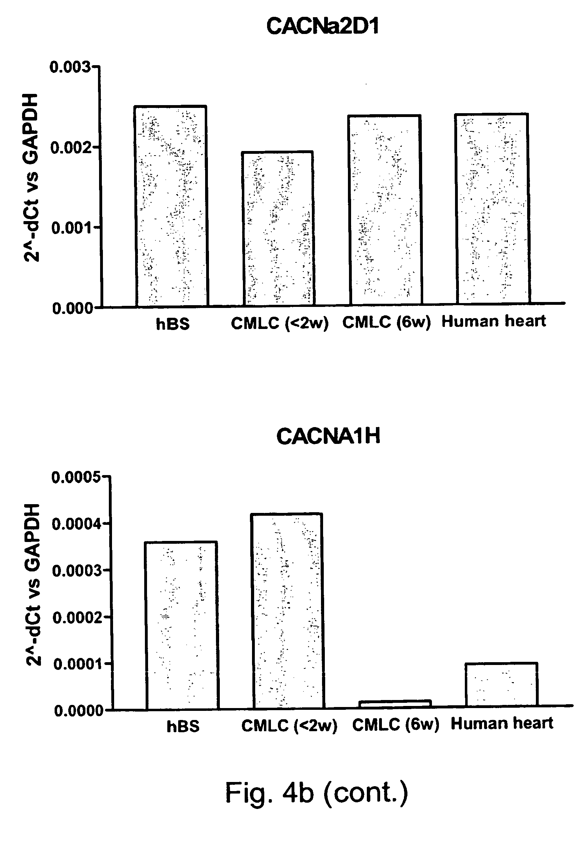

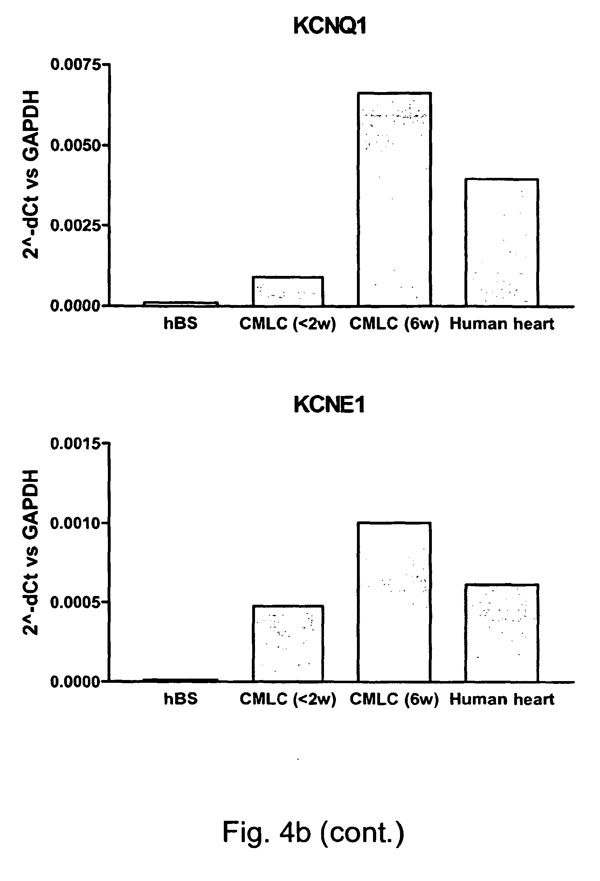

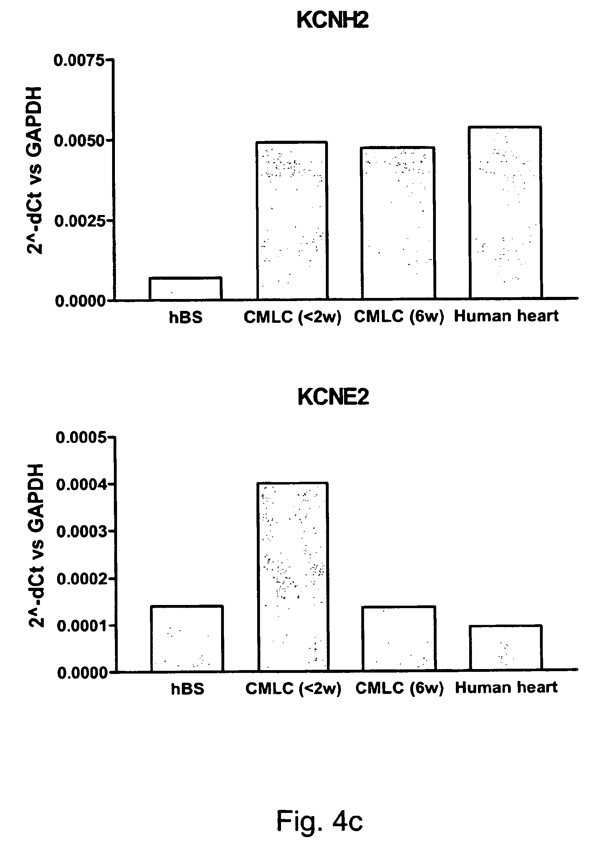

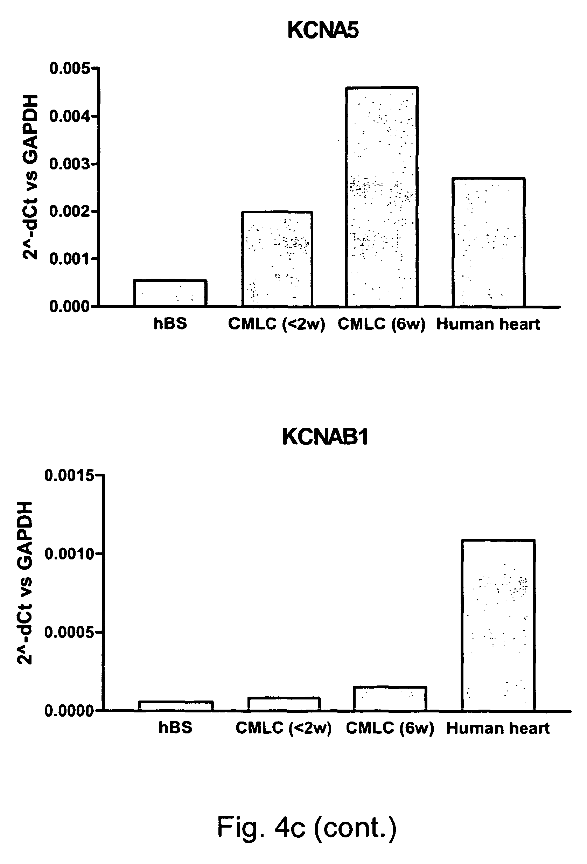

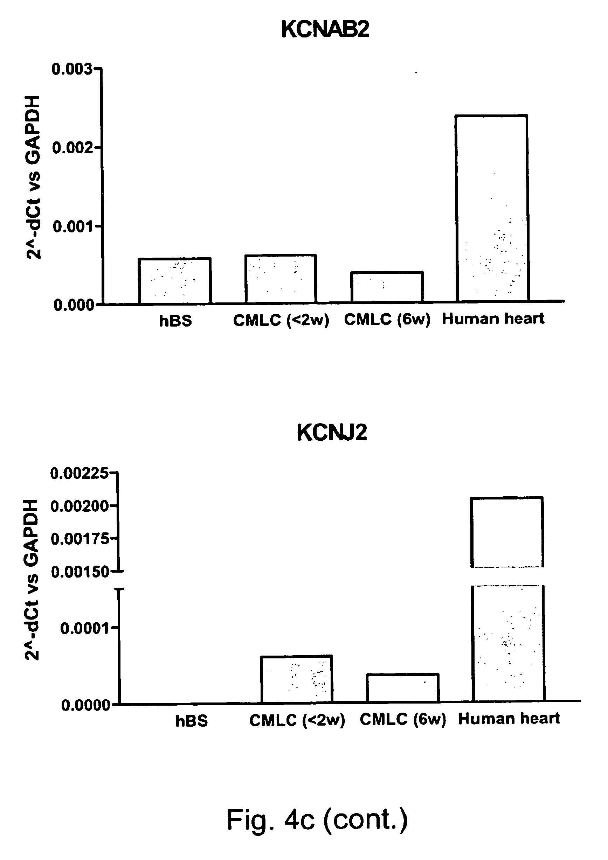

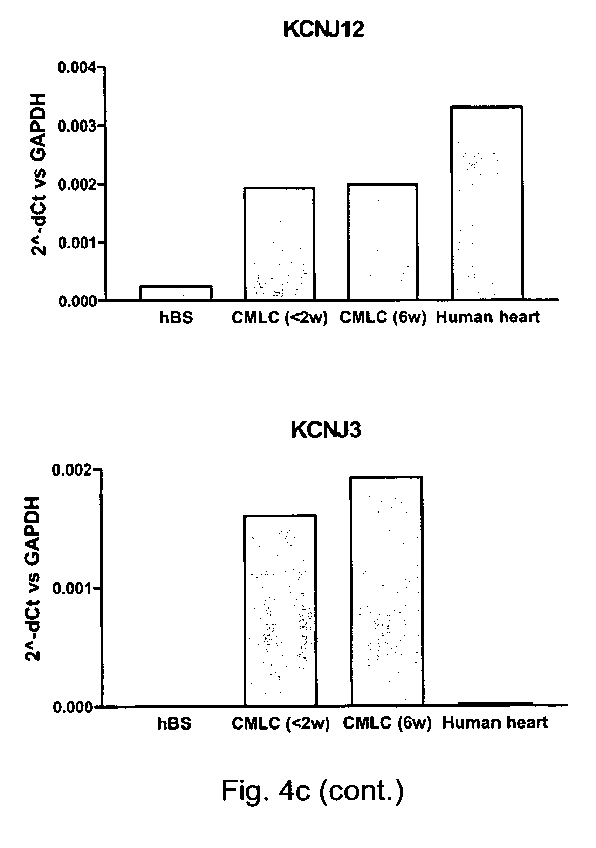

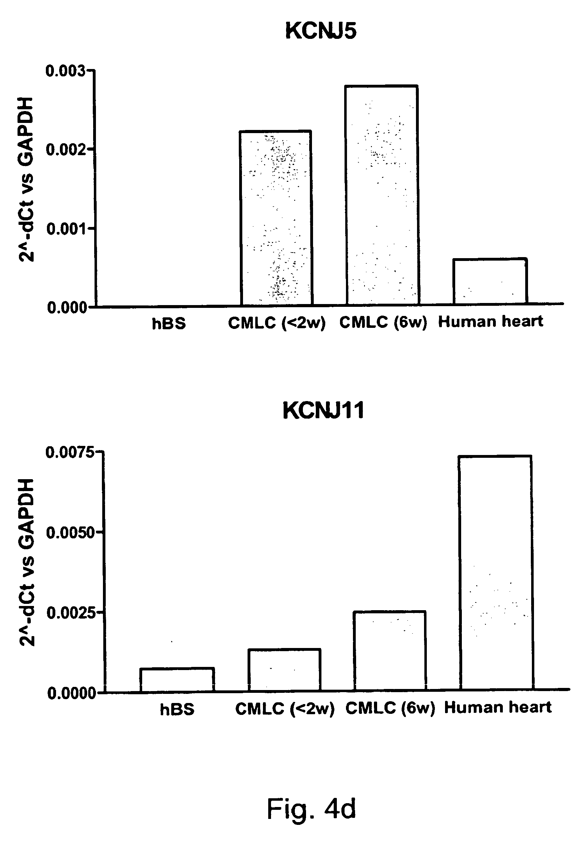

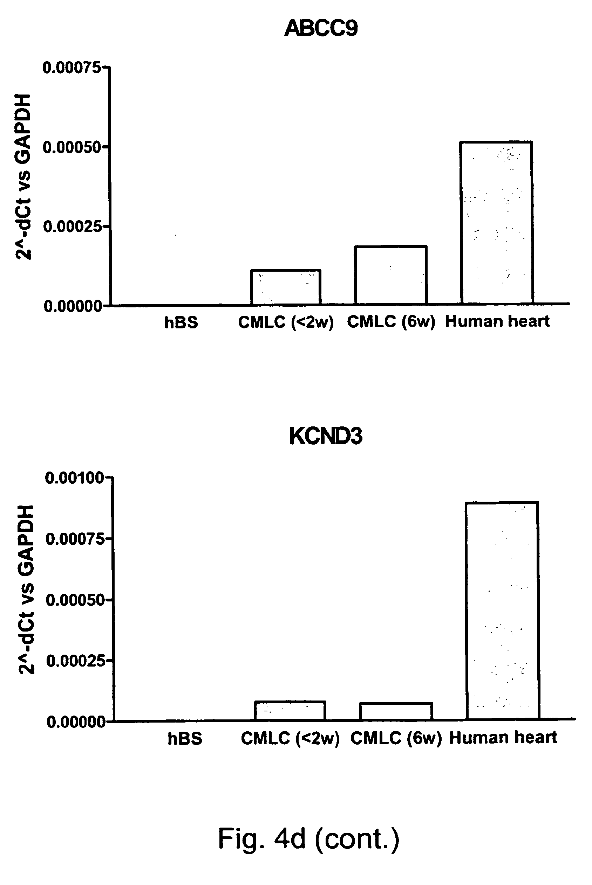

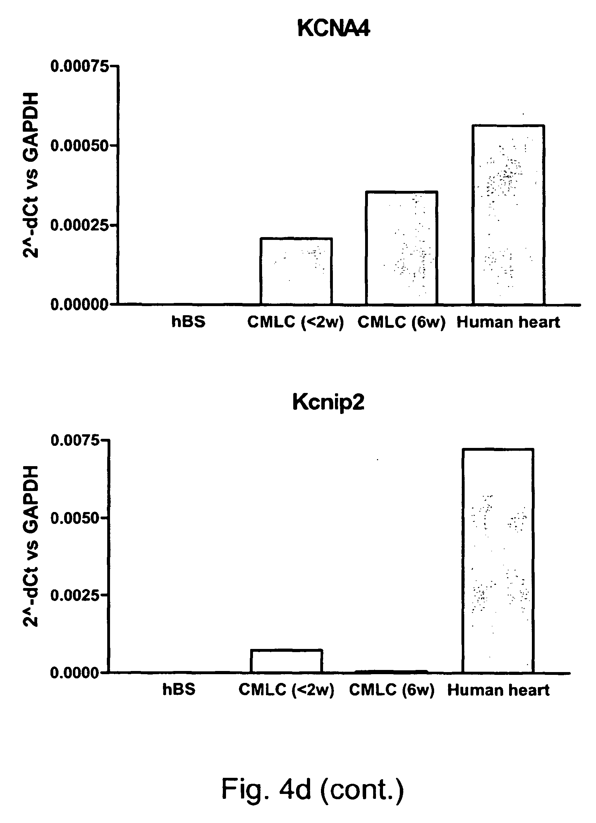

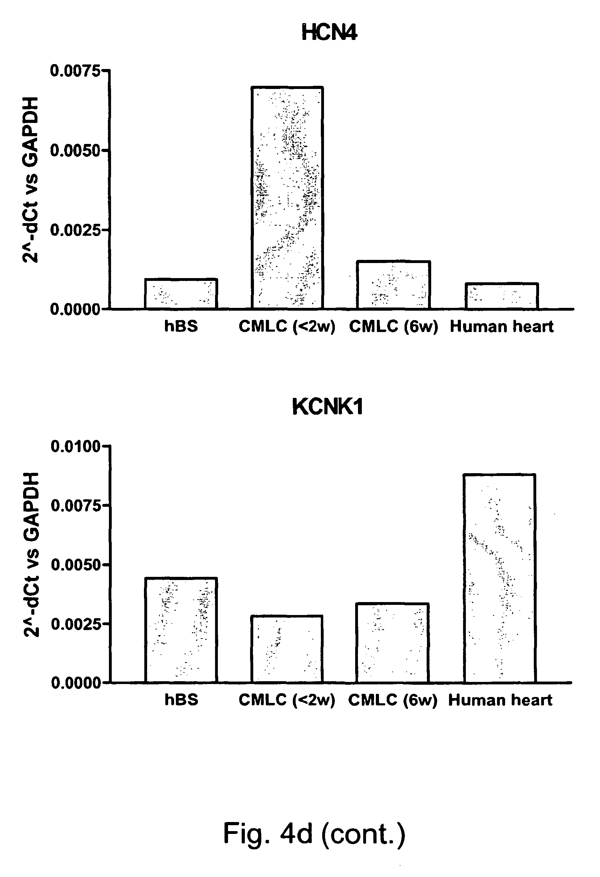

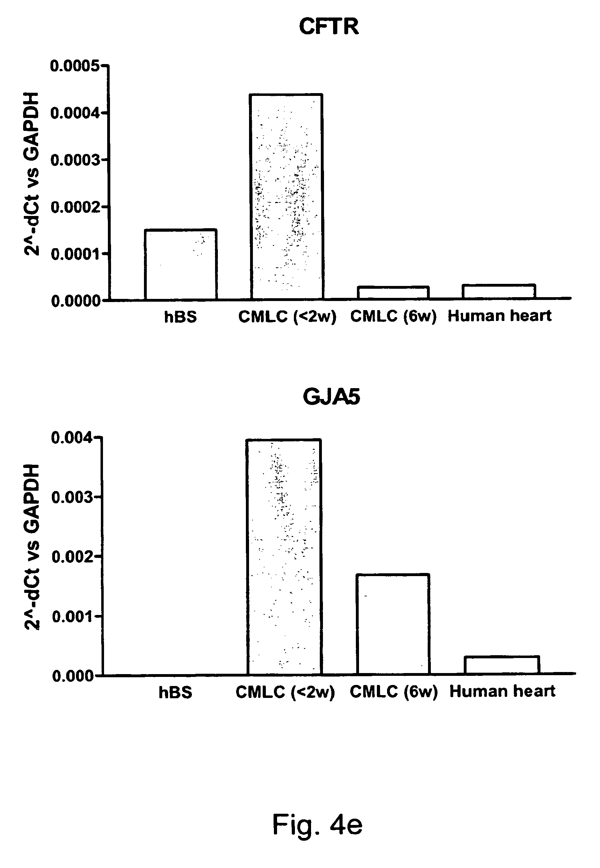

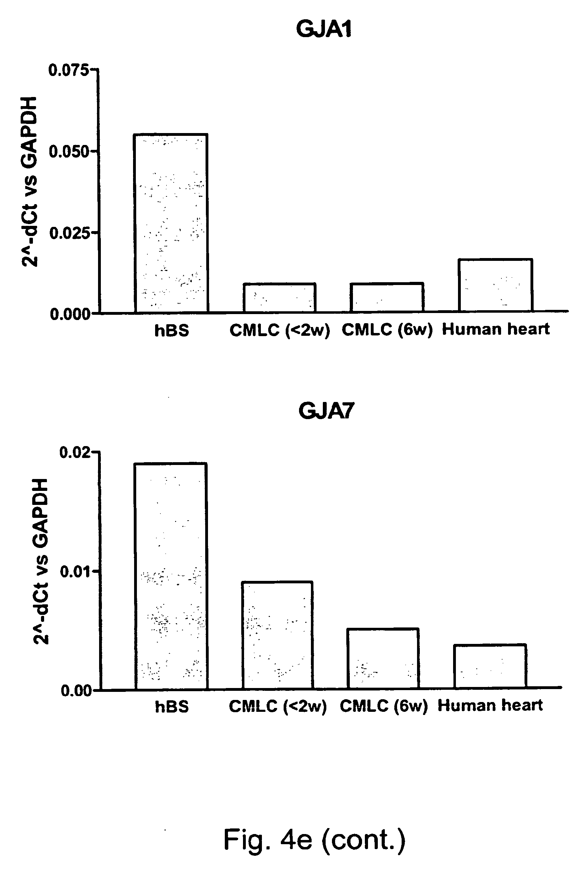

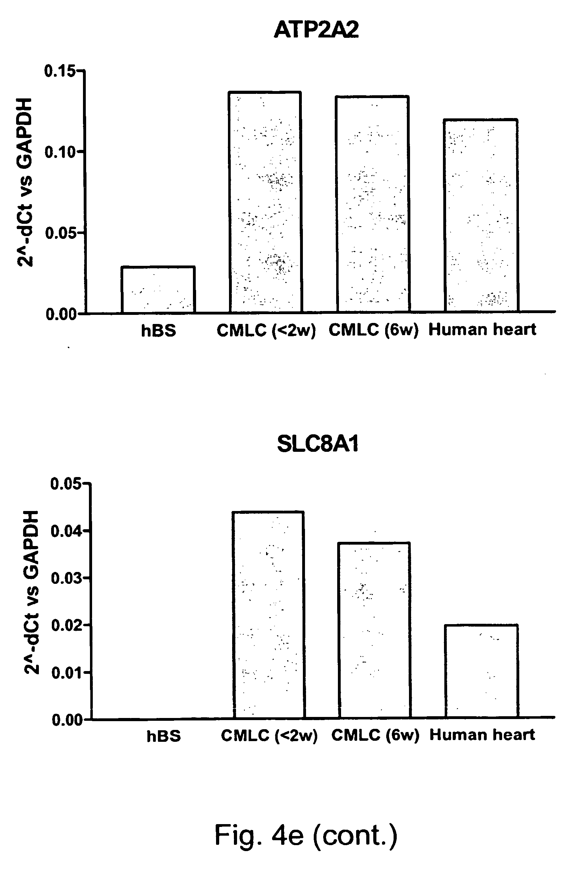

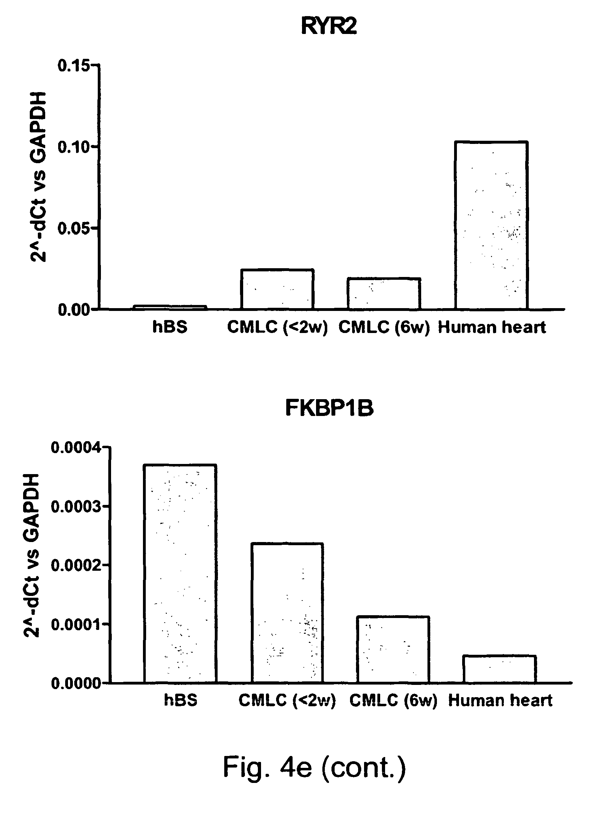

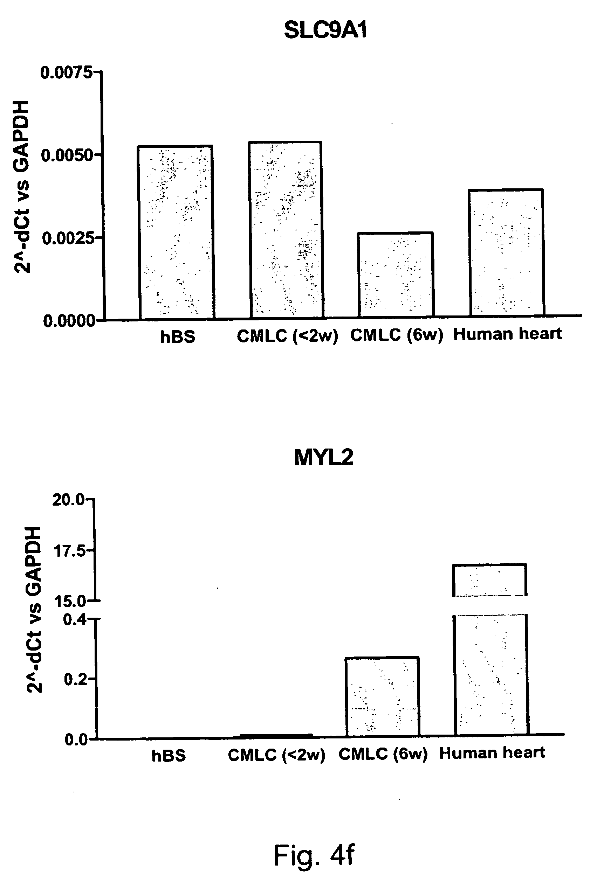

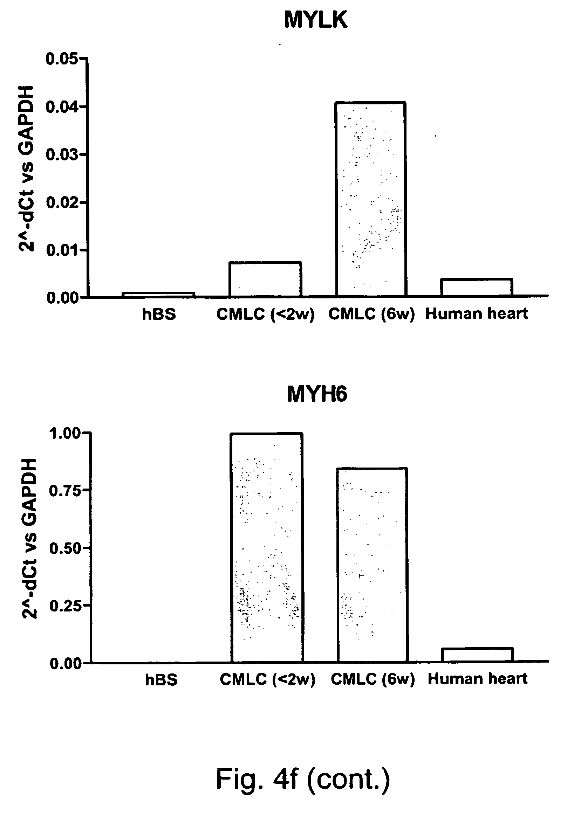

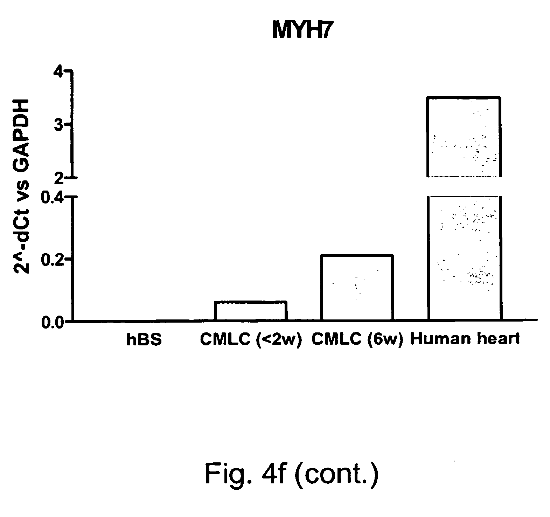

**Figure 4****.** QPCR analysis of gene expression in CMLC in comparison to undifferentiated hBS cells and adult human heart tissue. CMLC were differentiated from hBS cells as described in Reference Example 3. Two different developmental stages of CMLC were prepared; early CMLC (maintained for up to two weeks in culture) and late CMLC (maintained for at least 6 weeks in culture). To investigate the gene expression level of specific ion-channels in the CMLC and undifferentiated hBS cells, TaqMan® low density arrays (384-Well Micro Fluidic Cards, Applied Biosystems) were used. For comparison, commercially available total RNA from human adult normal heart tissue (pool from 6 different male donors) (BioChain®) was analyzed in parallel. QPCR was performed as described in Reference Example 3. Each PCR reaction was run in quadruplicate. The relative gene expression levels were normalized against the expression of GAPDH and calculated according to the ΔΔCₜ-method. Panel A shows the RNA levels for POU5F1 (Oct-4), NANOG, NKX2.5, GATA4, TNNI3, DES, SCN5A, and SCN1B. Panel B shows the gene expression levels for SCN2B, CACNA1C, CACNB1, CACNB2, CACNA2D1, CACNA1H, KCNQ1, and KCNE1. Panel C shows the gene expression levels for KCNH2, KCNE2, KCNA5, KCNAB1, KCNAB2, KCNJ2, KCNJ12, and KCNJ3. Panel D shows the gene expression levels for KCNJ5, KCNJ11, ABCC9, KCND3, KCNA4, Kcnip2, HCN4, and KCNK1. Panel E shows the gene expression levels for CFTR, GJA5, GJA1, GJA7, ATP2A2, SLC8A1, RYR2, and FKBP1B. Panel F shows the gene expression levels for SLC9A1, MYL2, MYLK, MYH6, and MYH7.

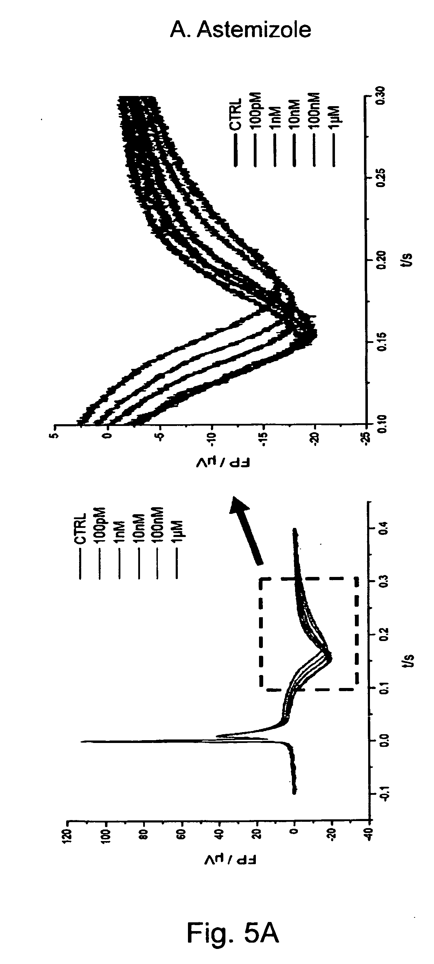

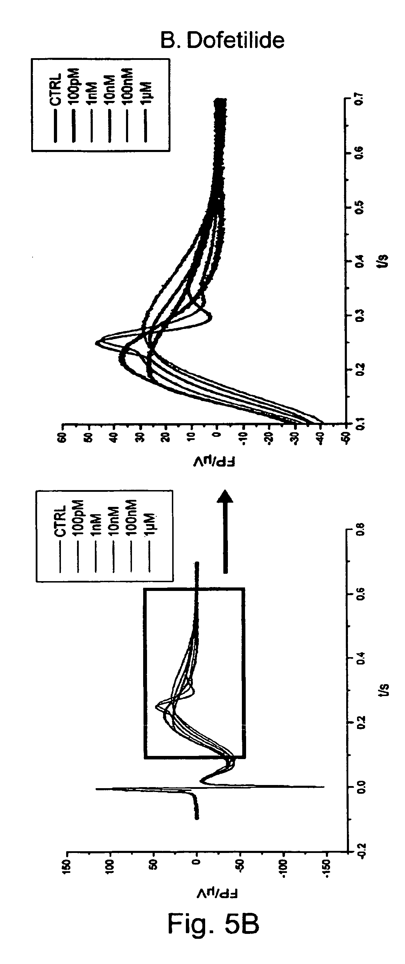

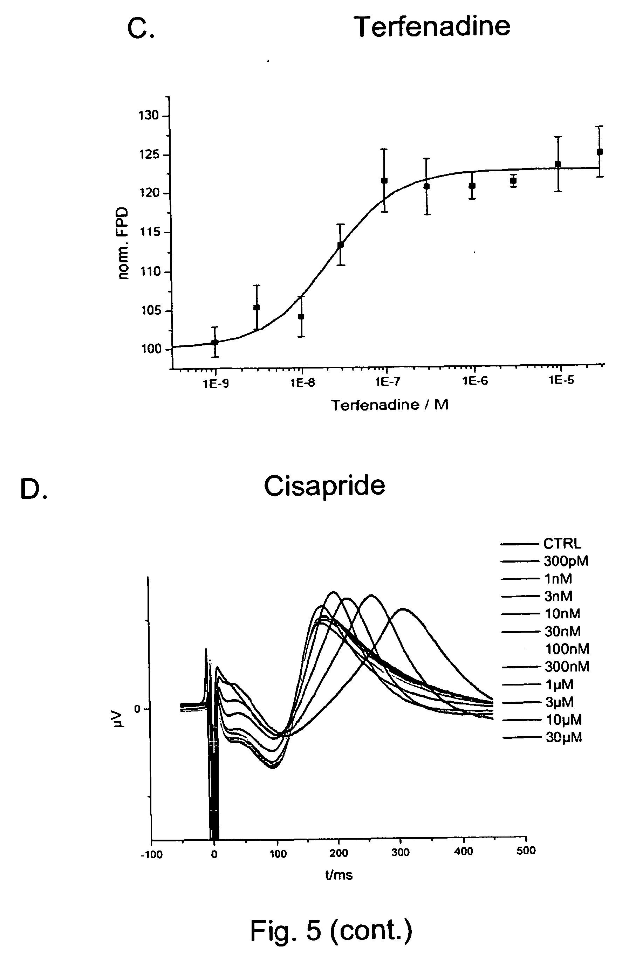

**Figure 5****.** Shows cumulative dose response curves upon increasing drug concentrations (0, 100pM, 1nM, 10nM, 100nM, 1000nM) measured as a delayed repolarisation of the cardiac field potentials after the addition of Astemizol (antihistaminic) and Dofetilide (a class III anti arrhythmic drug). As shown in Figure 5a, Astemizol caused a selective block and sustained prolongation of the repolarizing component at low nanomolar concentrations. Similar effects were observed using Dofetilide (Figure 5b). In addition, Figure 5c shows the dose dependent effect of Terfenadine on the field action potential duration and Figure 5d shows the effect of the potassium channel blocker Cisapride which causes a dose dependent prolongation of the action potential.
**Figure 6****.** CMLC were derived from undifferentiated hBS cells (SA002) as described in Reference example 1. During 3D formation (pre-plating), Activin A (10 ng/ml) and bFGF (12 ng/ml) were supplemented to the medium. The controls received medium with no factors added. Post-plating, medium with 20% FBS but without factors was used for both groups. Data were normalized to an endogenous control and are expressed as relative gene expression with the control set to 100 %. The data shown are the mean of gene expression of the respective gene in two experiments; A) Nxk2.5, B) GATA4, C) Troponin T2 and D) alpha-cardiac actin. Panel E shows the mean value of the number of beating CMLC generated in three experiments. The error bars indicate the S.D.
**Figure 7****.** Poster on global gene expression profiling and characterization of human embryonic stem cell derived cardiomyocyte like clusters. Figure 7a shows the poster and Figure 7b shows a close up of Table 1. Figures 7a1-3 give text details for Fig. 7a.

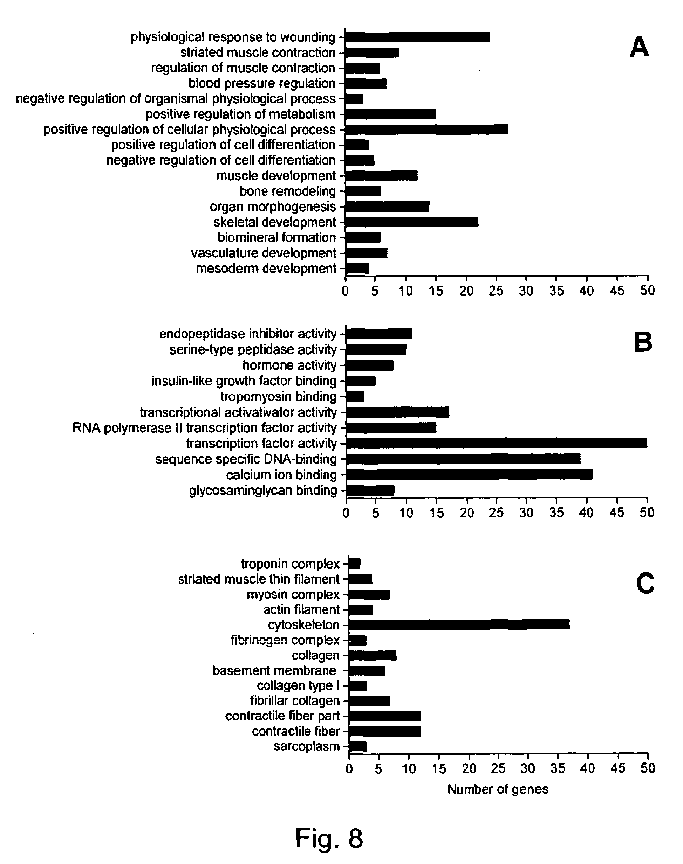

**Figure 8****.** Overrepresented Gene Ontology annotations: Significantly overrepresented Gene Ontology annotations among the 530 up-regulated genes, in hBSC-derived CMCLs. Only significantly overrepresented annotations (p<0.01) are shown in the picture. Panel A represents "Biological process", panel B "Molecular function" and panel C "Cellular component". The X-axis shows the number of genes with a specific annotation.

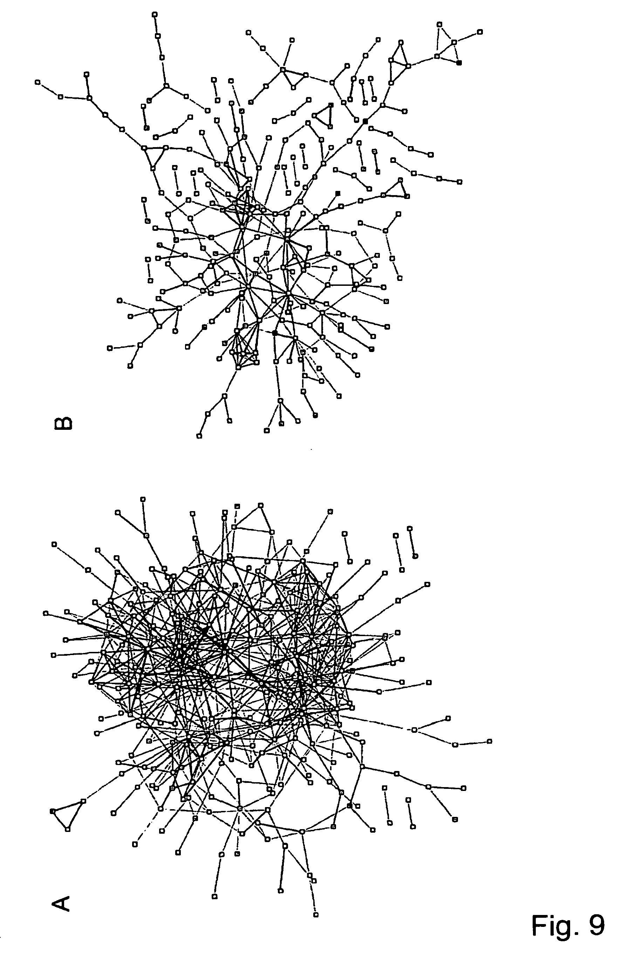

**Figure 9****.** Protein interaction maps: Panel A shows the interaction map of the gene products from 530 significantly up-regulated genes in hBSC-derived CMCLs and panel B shows a corresponding interaction map for one of the randomly generated sets of proteins.

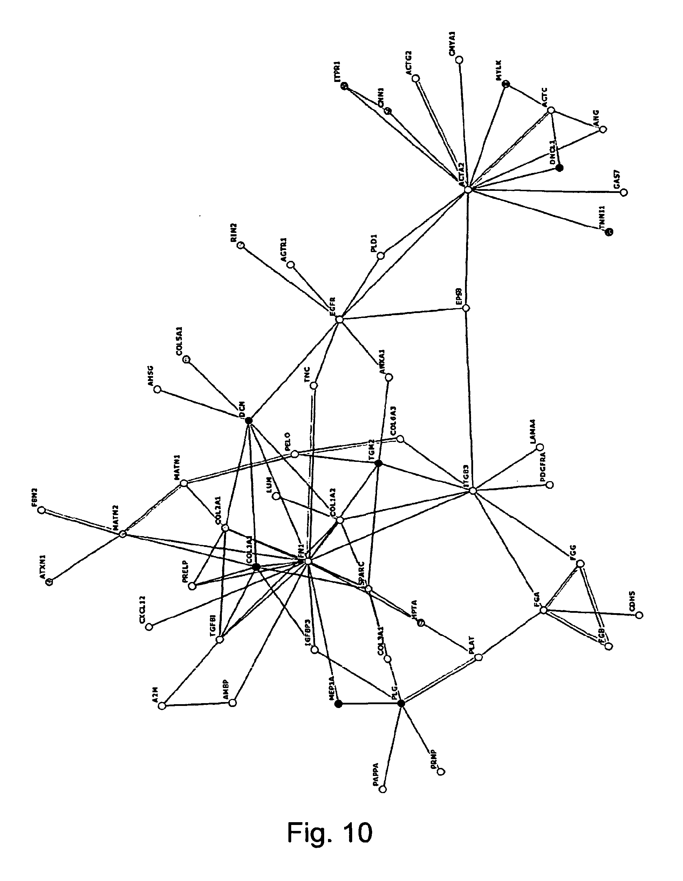

**Figure 10****.** Hub protein network in hBSC-derived CMCLs: Proteins are identified as hubs if they have at least five experimentally determined protein interactions among the products of the up-regulated genes.
**Figure 11****.** A complete list of the 530 genes that were significantly up-regulated in CMCLs compared to undifferentiated hBSs, identified using the SAM algorithm. "Avg FC" column shows the average fold change between the two experiments and next column to the right shows the range of the calculated fold changes across the different samples. The rightmost column represents different categories regarding how these genes have previously been reported to be expressed in heart tissue. Of the 417 genes that were identified by WebGestalt, 331 had been reported as expressed in heart before. The genes that have not been reported as expressed in heart or lack a tissue expression record in WebGestalt (e.g., MYL6) are marked with X (86 genes). Genes that are reported as significantly over-expressed are marked with O (100 genes) and significantly under-expressed genes are marked with U (25 genes). Genes marked with N (206 genes) are reported as expressed in heart but with no significance in expression. Genes marked with dash (-), in total 113 genes represent genes that were significantly up-regulated in CMLC by the WebGestalt toolbox.

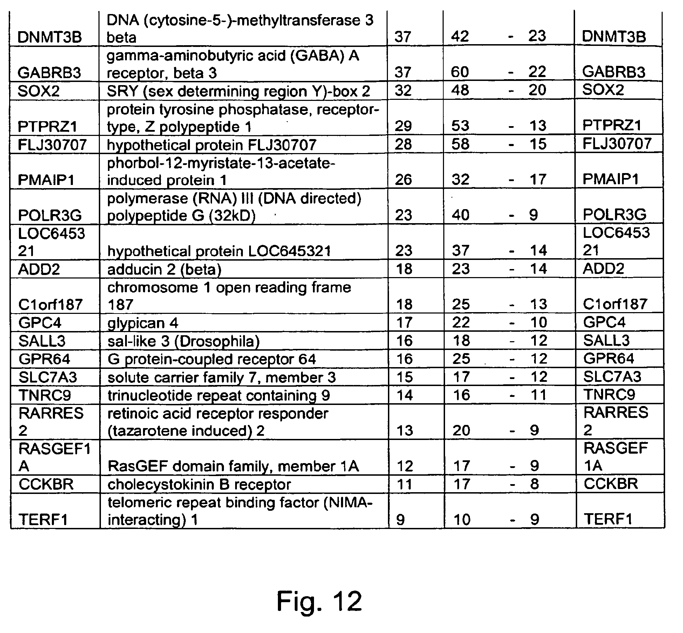

**Figure 12****.** Genes that are significantly down-regulated in hBSC-derived CMCLs compared to undifferentiated hBSCs. Among these 40 genes are well known markers for pluripotency, such as NANOG, POU5F1, SOX2, TDGF1, DPPA4, LEFTY1, and DNMT3B.
**Figure 13****.** CMLC were derived from undifferentiated hBS cells (SA002) as described in Reference example 1. During 3D formation (pre-plating) different types of FBS was supplemented to the medium (20%) as indicated in the graph. The controls received standard FBS and this was compared to dialyzed and charcoal stripped FBS. Post-plating, medium with 20% standard FBS was used for all groups.The number of beating CMLC was counted 14 days post-plating using a light microscope and compared to the control. The data shown are the mean of the number of beating CMLC generated in three experiments. The error bars indicate the S.D.
**Figure 14****.** CMLC were derived from undifferentiated hBS cells (SA002) as described in Reference Example 1. During 3D formation (pre-plating) the culture medium was supplemented with Activin A and bFGF (F). The controls (C) received standard medium only. Post-plating, some groups received medium supplemented with 1000U/ml LIF (F+LIF) while the others received standard medium alone. Four days post-plating all groups received standard medium. The number of beating CMLC was counted at day 7 and day 14 post-plating using a light microscope and compared to the control. The data shown are the mean of the number of beating CMLC generated in three experiments relative to the control set to 1. The error bars indicate the S.D.
**Figure 15****.** Effect of SB 216763 and SKF-86002 on the yield of beating CMLC. CMLC were derived from undifferentiated hBS cells (SA002) as described in Reference Example 8. During 3D formation (pre-plating), SB 216763 (5 µM) or SKF-86002 (5 µM) were supplemented to the medium in addition to Activin A (10 ng/ml) and bFGF (12 ng/ml). The control received medium with only Activin A and bFGF added. Post-plating, standard medium without factors was used for all groups. The graph shows the collective number of beating areas related to the number of hBS cell colonies used in the experiments. AF = Activin A + bFGF, SB = SB 216763, and SKF = SKF-86002.

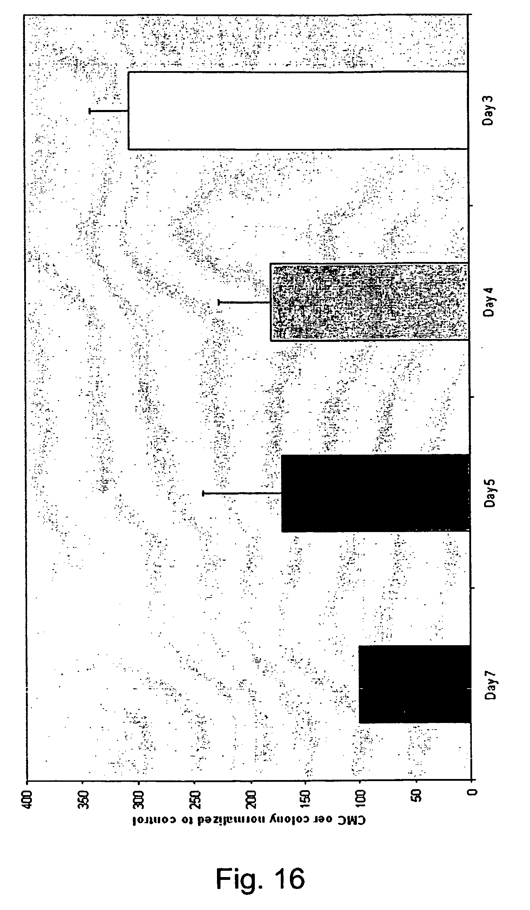

**Figure 16****.** The day at which 3D-aggregates of differentiated hBS cells are transferred from the 96-well plates to new culture dishes significantly affects the number of beating CMLC that can be generated. In these experiments, CMLC were derived from undifferentiated hBS cells (SA002) as described in Reference Example 1. More CMLC are produced from the 3D-aggregates that are transferred earlier when compared to transfer at 7 days. The data shown are the mean of the number of beating CMLC generated relative to the control (7 days) set to 100 at different plating days (5, 4 ,and 3). The error bars indicate the S.D.

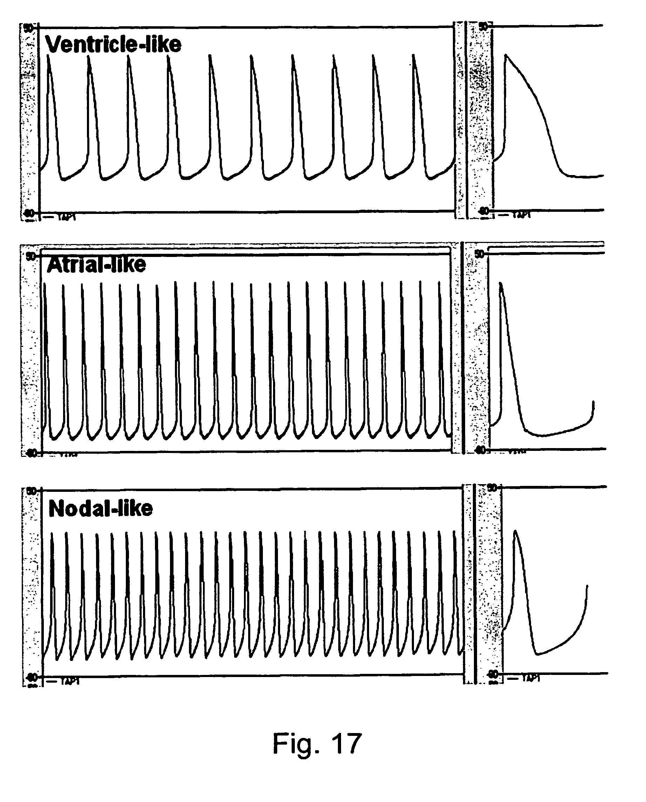

**Figure 17****.** Through microelectrode recordings three different types of action potential morphologies were demonstrated; ventricle-like, atrial-like, and nodal-like.

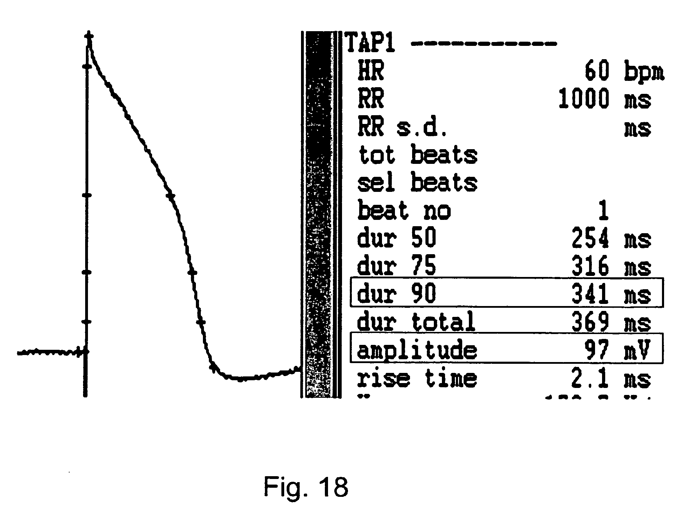

**Figure 18****.** Example of ventricular-like AP with an amplitude of 97 mV and long APD90 of 342 ms.

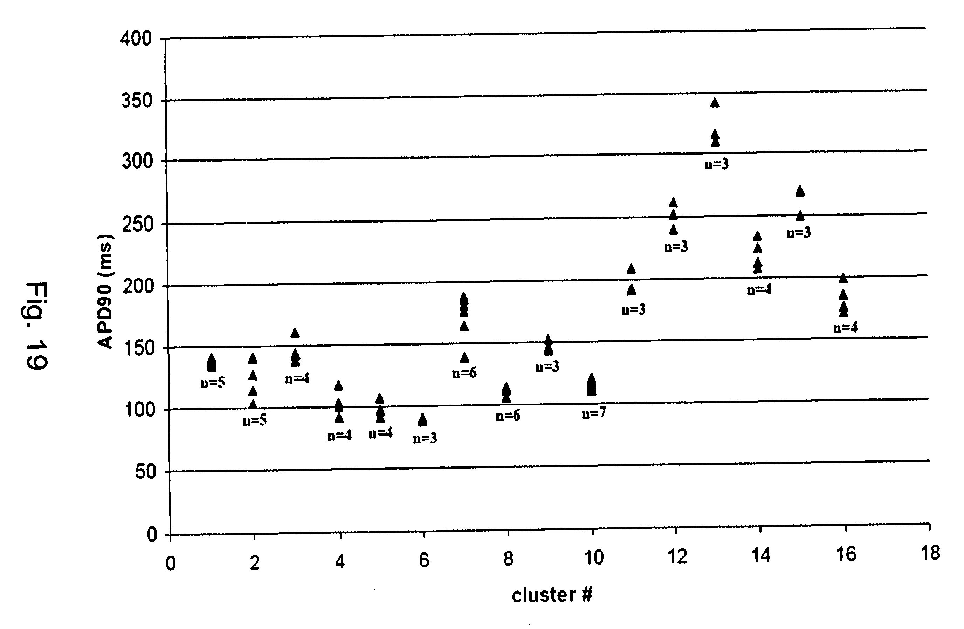

**Figure 19****.** Each CMLC cluster was characterized by a predominant AP morphology. The graph illustrates APD90 in 16 different clusters and they show similar values for repeated impalements within the same cluster.

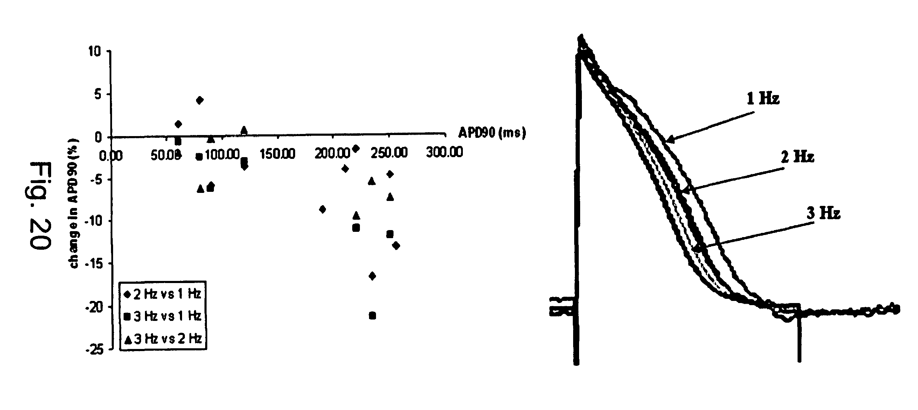

**Figure 20****.** Rate adaptation of CMLC clusters during exposure to field stimulation of 1, 2, and 3 Hz. Left panel: Plot showing that clusters with an initial APD90 over 180 ms showed rate adaptation as pacing frequency increased. Right panel: Overlay plot of AP at 1, 2, and 3 Hz pacing.

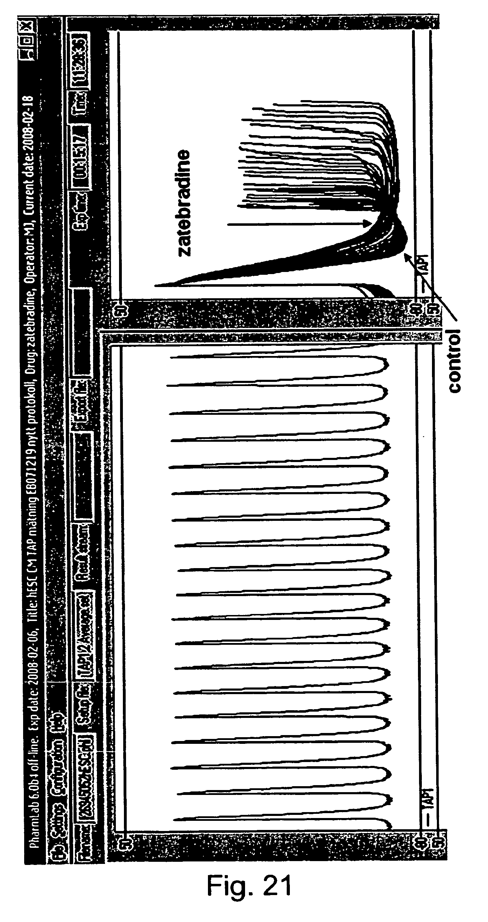

**Figure 21****.** Through microelectrode recordings action potential measurements were preformed during incubation of CMLC clusters with Zatebradine. Right figure shows an overlay plot.

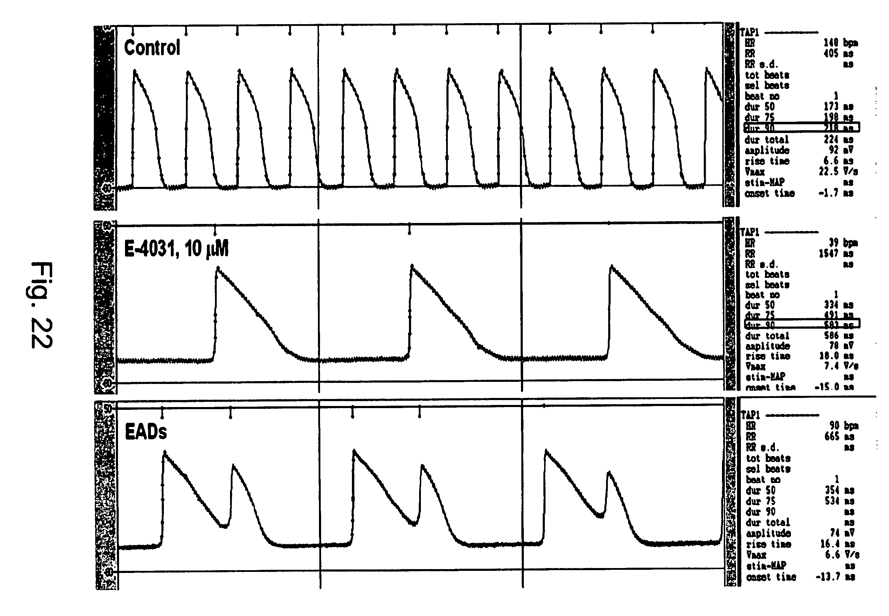

**Figure 22****.** Effect of IKr block (E-4031) on APs. Administration of E-4031 caused APD prolongation, triangulation, and EADs in CMLC clusters.

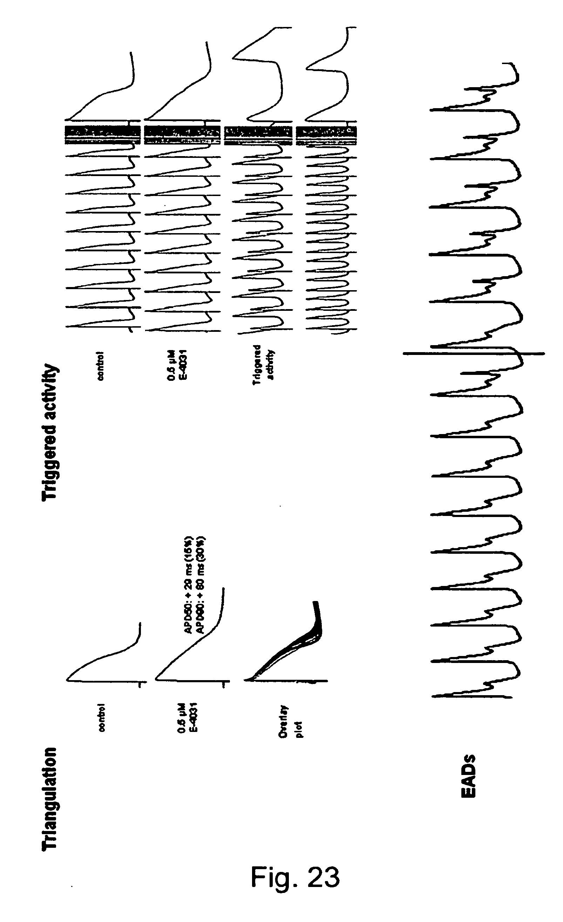

**Figure 23****.** Effect of IKr block on APs. Top left panel: Administration of E-4031 caused prolonged APD in all cases and clear signs of triangulation as seen on the shape of APs and a greater lengthening of APD90 compared to APD50. Top right panel: In a majority of cases, APD prolongation was followed by triggered activity. Bottom panel: Early after depolarizations (EADs) were also recorded.

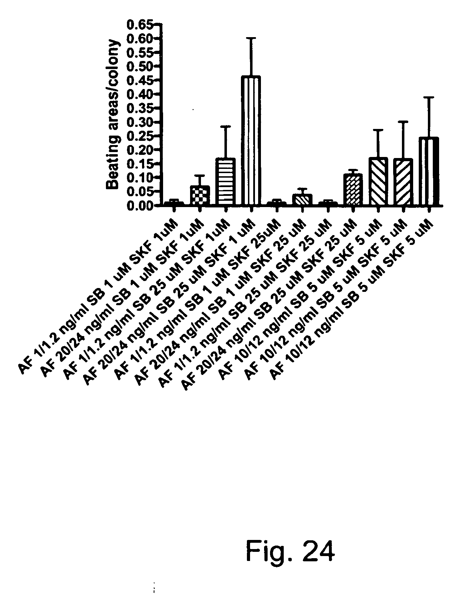

**Figure 24****.** Effect of Activin A, bFGF, SB 216763 and SKF-86002 on the yield of beating. CMLC. CMLC were derived from undifferentiated hBS cells (SA002) as described in Reference Example 1. During 3D formation (pre-plating), different concentrations of Activin A, bFGF, SB 216763 and SKF-86002 were supplemented to the medium in addition. Post-plating, medium without factors was used for all groups. The picture shows the collective number of beating areas related to the number of colonies used in one dish per group. The experiment was repeated three times. AF = Activin A + bFGF, SB = SB 216763, and SKF = SKF-86002.

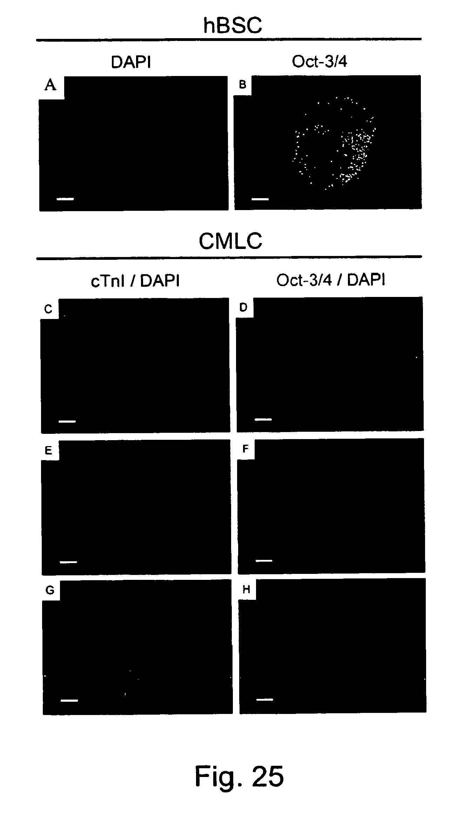

**Figure 25****.** No Oct-3/4 hBS cell marker expression in hBSC derived CMLC. Immunohistochemical examination of stem cell marker Oct-3/4 expression in growing hBS cell colonies (A-B) and in hBS cell derived CMLC (C-H). (A) Nuclei are stained blue with 4',6-diaminidine-2-phenylidole dihydrochloride (DAPI). (B) hBS cell colony showing positive staining with antibody against Oct-3/4 (green). Sections from at least ten CMLC (only three are shown here) were labeled with antibodies against cardiac Troponin I (cTnI; red) and Oct-3/4 (green), as well as with DAPI for the nuclei (blue). The left panel (C, E and G) shows overlay of cTnI (red) and DAPI (blue) staining, while the right panel (D, F and H) shows overlay of Oct-3/4 (green) and DAPI (blue) staining. While all CMLC showed positive staining with cTnI (C, E and G) no detectable staining of the Oct-3/4 hBS cell marker could be observed in the clusters (D, F and H). Scale bars: 100 µm.

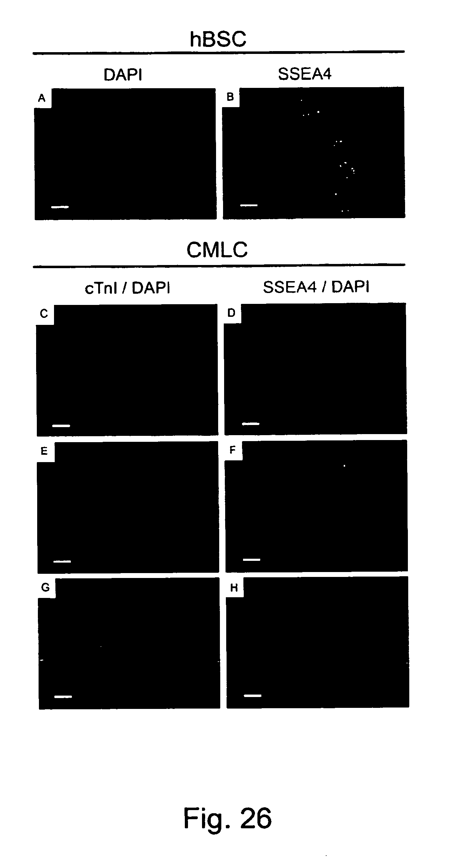

**Figure 26****.** No SSEA-4 hBS cell marker expression in hBS cell derived CMLC. Immunohistochemical examination of stem cell marker SSEA-4 expression in growing hBS cell colonies (A-B) and in hBS cell derived CMLC (C-H). (A) Nuclei are stained blue with 4',6-diaminidine-2-phenylidole dihydrochloride (DAPI). (B) hBS cell colony showing positive staining with antibody against SSEA-4 (green). Sections for at least ten CMLC (only three are shown here) were labeled with antibodies against cardiac Troponin I (cTnI; red) and SSEA-4 (green), as well as with DAPI for the nuclei (blue). The left panel (C, E and G) shows overlay of cTnI (red) and DAPI (blue) staining, while the right panel (D, F and H) shows overlay of SSEA-4 (green) and DAPI (blue) staining. While all CMLC clusters showed positive staining with cTnI (C, E and G) no detectable staining of the SSEA-4 hBSC cell marker could be observed in the clusters (D, F and H). Scale bars: 50 µm.

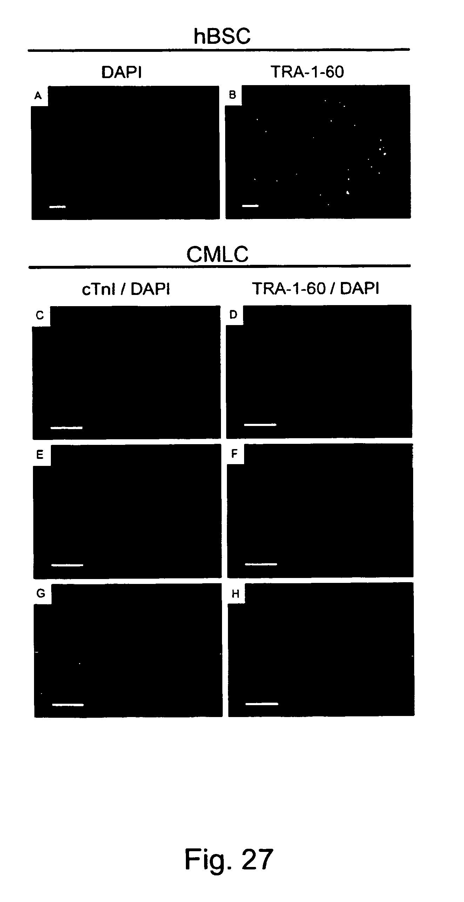

Figure 27. No TRA-1-60 hBS cell marker expression in hBS cell derived CMLC. Immunohistochemical examination of stem cell marker TRA-1-60 expression in growing hBS cell colonies (A-B) and in hBS cell derived CMLC (C-H). (A) Nuclei are stained blue with 4',6-diaminidine-2-phenylidole dihydrochloride (DAPI). (B) hBS cell colony showing positive staining with antibody against SSEA-4 (green). Sections for at least ten CMLC (only three are shown here) were labeled with antibodies against cardiac Troponin I (cTnI; red) and TRA-1-60 (green), as well as with DAPI for the nuclei (blue). The left panel (C, E and G) shows overlay of cTnI (red) and DAPI (blue) staining, while the right panel (D, F and H) shows overlay of TRA-1-60 (green) and DAPI (blue) staining. While all CMLC showed positive staining with cTnI (C, E and G) no detectable staining of the SSEA-4 hBS cell marker could be observed in the clusters (D, F and H). Scale bars: 100 µm.

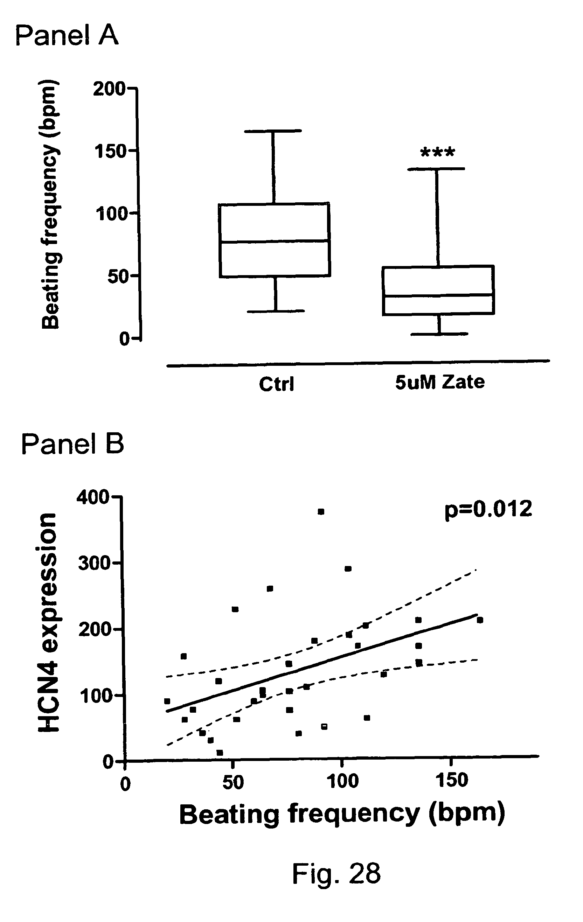

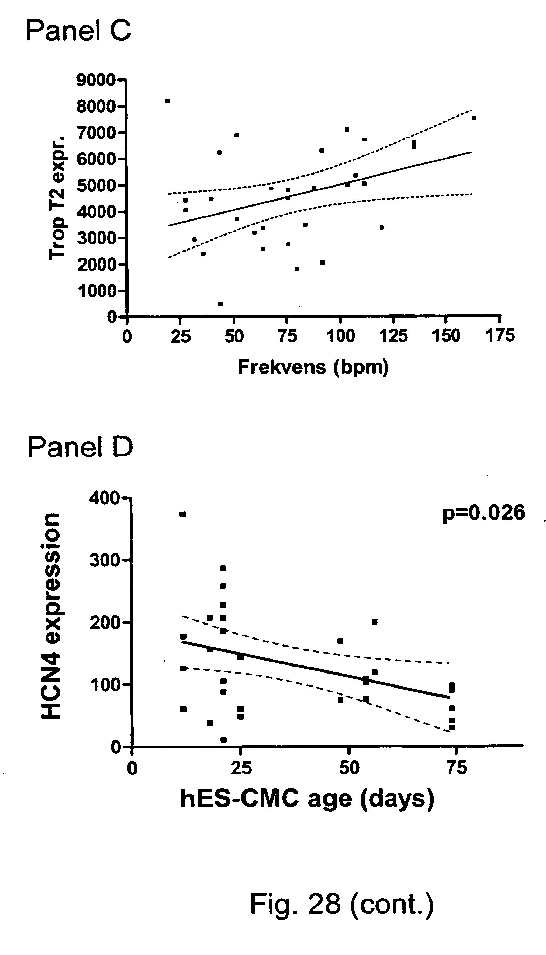

Figure 28. The effect of Zatebradine on the beating frequency of contracting CMLC derived from hBS cells. Panel A shows the results from experiments in which 5µM Zatebradine was administered to 32 individual spontaneously beating CMLC of different ages. The change in beating frequency was calculated by visually registering the contraction rate before and after Zatebradine administration. Values are expressed as beats per minute ± SEM, n = 32, *** = p < 0.001. Statistical significance was calculated using paired t-test. Panel B shows the correlation between beating frequency and mRNA expression of HCN4 (p=0.0116) and Panel C the correlation between beating frequency and mRNA expression of Troponin T2 (p=0.0314). Panel D shows the correlation between HCN4 mRNA expression and age of the CMLC (p=0.0262). The mRNA expression of HCN4 and Troponin T2 was measured using real-time RT quantitative PCR and related to the expression of the endogenous control CREBBP. Values are expressed as relative gene expression as compared to a reference sample set to 100 % (n = 32). Statistical analysis was performed using linear regression analysis.

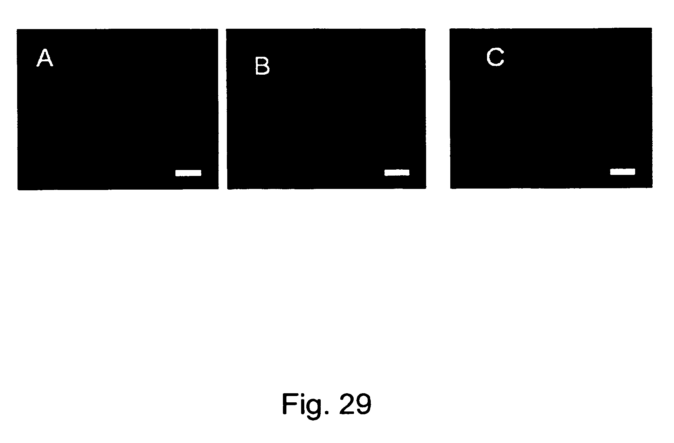

Figure 29. Immunohistochemical analysis of CMLC derived from hBS cells. The figure shows positive staining for antibodies against the hERG ion channel (KCNH2) (panel A), nuclei (DAPI) (panel B), and cardiac troponin I (panel C). Scale bar, 50µM.

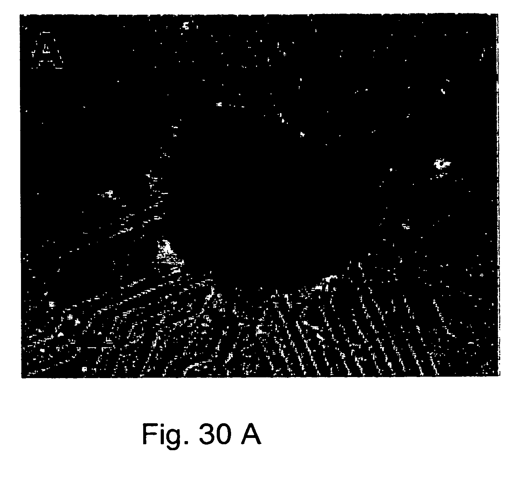

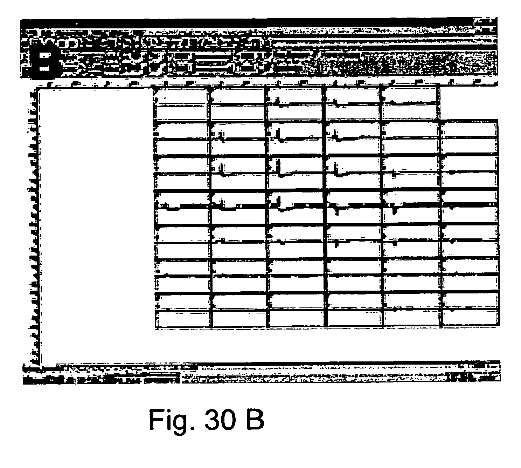

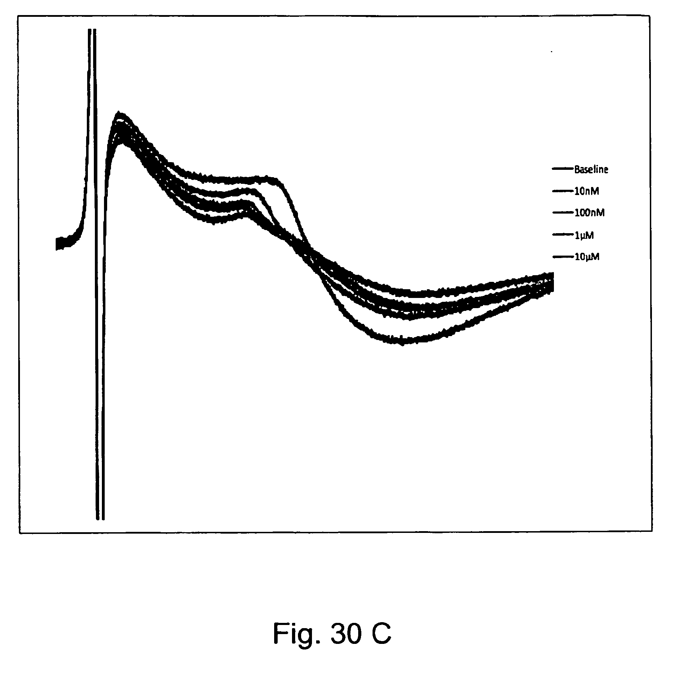

Figure 30. Micro electrode array (MEA) analysis of hES cell derived cardiomyocytes. Panel A shows a hES cell derived cardiomyocyte cluster (hES-CMC™, Cellartis AB) cultured directly on to a MEA (Multichannel Systems, Germany). The MEA technology allows multiple field potential waveforms to be recorded simultaneously, allowing for the analysis of wave propagation and conduction velocity (Panel B). Panel C shows cumulative dose response curves, upon increasing drug concentrations (0, 1nM, 10nM, 100nM, 1 µM, 10 µM), after the addition of Astemizol (an antihistaminic with know QT-prolongation effects). A selective block and sustained prolongation of the repolarizing component at nanomolar concentrations is evident, as measured by delayed repolarization of the cardiac field potential.

The invention is further illustrated in the following non-limiting examples.

### Examples

### Reference Example 1: Differentiation of hBS cells to cardiomyocyte-like cell clusters

Spontaneously contracting cells were derived from undifferentiated hBS cells cultured on MEF cells (Heins et al 2004 Stem Cells). The cell lines used for this experiment could be the hBS cell line SA002, SA121, SA001, SA002.5, SA461 (Cellartis AB, Göteborg, Sweden, http://www.cellartis.com) and they can be propagated as described Heins et al. 2004. These cell lines are listed in the NIH stem cell registry and the UK Stem Cell bank. The hBS cells were detached from the feeder layer by incubation with collagenase IV (200 U/ml), for 10-15 minutes at 37 °C. The cell suspension was transferred to a 15 ml tube, and after the cells had sedimented, the supernatant was removed. The colonies were resuspended in Knock Out DMEM supplemented with 20% FBS, 1% penicillin-streptomycin, 1% Glutamax, 0.5 mmol/l β-mercaptoethanol and 1% non-essential amino acids (all from Invitrogen, Carlsbad, California) and dissociated mechanically into small aggregates of undifferentiated cells (0.2-0.4 mm) using a pipette. This cell suspension was distributed (200 µl/well) into a 96-well v-bottom plate (Corning Incorporated, NY, USA) at a concentration of approximately 4 - 6 colonies per well and centrifuged for 5 min at 400 x g. The plate was then incubated at 37 °C for 3 - 8 days. The 3D structures that formed were then transferred to gelatine coated dishes containing culture medium (same as above). After a couple of days (normally 1-4) of culture, spontaneously contracting areas with cluster of contracting cells (CMLC) occurred and could be visually identified using a light microscope. Medium was changed every second to third day. New spontaneously beating areas continued to appear for up to 20 days post-plating. The clusters are isolated by mechanical dissection under visual inspection using a light microscope from the 3D-structures and subjected to further characterization. Figure 1a, b and c shows representative illustrations of the appearance of the CMLC derived from hBS cells. hBS derived CMLC were differentiated as described above. These were then manually isolated from the surrounding cells and plated onto laminin coated glass. After three days in culture, the clusters were fixed and stained for nuclei and the protein cardiac troponin 1, which is known to be specific for cardiac tissue. These clusters were then examined with confocal microscopy. The micrograph in figure 2 shows a CMLC that has been stained to show nuclei (round objects) and cardiac troponin I (line-like structures) (magnification x 600).

The distribution in beating frequency was evaluated by determining the beats per minute (bpm) of 109 different spontaneously contracting areas. The average beating frequency was 44 bpm ± 24 (Std.dev.). The overall distribution is illustrated in Figure 3.

In order to improve the yield of CMLC from hBS cells, the culture medium can, at various time points during the experiments, be supplemented with different factors such as protein growth factors, chemical compounds, minerals, or other signalling molecules. Examples of such factors are BMP-2, BMP-4, BMP-5, TGF-β1, Activin A, bFGF, Growth hormone, LIF, and PDGF.

### Reference Example 2. Micro array analysis of cardiomyocyte-like cell clusters (CMLC) derived from hBS cells

### Cell culture and differentiation

The hBS cell line SA002 (Cellartis AB, Göteborg, Sweden, http://www.cellartis.com) was propagated as described Heins et al. 2004. This cell line is listed in the NIH stem cell registry and the UK Stem Cell bank. Differentiation of hBS cells was performed as described in Reference Example 1 above. Using light microscopy clusters of spontaneously contracting cardiomyocyte-like cell clusters (CMLC) are frequently observed when hBS cells are differentiated through this protocol. For microarray experiments, three separate samples (biological replicates) of CMLC were collected. For comparison purposes, two independent samples from undifferentiated hBS cells and one sample from a mixed population of differentiated cells (i.e., no contracting CMLC present) were also collected for the analysis. The mixed population was collected as the remaining population of differentiated cells following the selection and removal of the spontaneously contracting CMLCs in the cultures differentiated as described in Reference Example 1. Samples from three independent runs were pooled as indicated below. Using the mixed population as a control therefore represents a differentiated cell population, minus the CMLCs, making subtraction of genes up regulated in all differentiated cells available.
Samples:
1. Undifferentiated hBS cells (pooled from passage 33, 36, 37, 39, and 40)
2. Undifferentiated hBS cells (pooled from passage 35-37)
3. CMLC 1 (pooled from passage 23-25, 29, and 35)
4. CMLC 2 (pooled from passage 39-41)
5. CMLC 3 (pooled from passage 23-28, 32-34, 36-40, and 49)
6. Mixed population of differentiated hBSC (pooled from passage 22, 28, and 32)

### RNA extraction and micro array experiments

Total RNA was extracted from undifferentiated and differentiated hBS cells using the RNeasy® Mini Kit (Qiagen) and subsequently used for microarray experiments employing GeneChip® Human Genome U133 Plus 2.0 (Affymetrix Inc, Santa Clara, CA, USA) targeting 54,675 transcripts. Due to small amounts of RNA, some of the samples in the experiment were amplified using two-cycle in-vitro transcription (IVT). RNA quality and concentration was measured using an Agilent 2100 bioanalyzer and Nanodrop ND-1000 respectively. Total RNA was processed following the GeneChip® Expression 3'-Amplification Reagents Two-cycle cDNA synthesis kit** instructions (Affymetrix Inc, Santa Clara, CA, USA) to produce double-stranded cDNA. This was used as a template to generate biotin-targeted cRNA following manufacturer's specifications. Fifteen micrograms of the biotin-labelled cRNA was fragmented to strands between 35 and 200 bases in length, 10 micrograms of which was hybridised onto the Gene Chip® Human Genome U133 Plus 2.0 (Affymetrix Inc, Santa Clara, CA, USA) overnight in the GeneChip® Hybridisation oven 6400 using standard procedures. The arrays were washed and then stained in a GeneChip® Fluidics Station 450. Scanning was carried out with the GeneChip® Scanner 3000 and image analysis was performed using GeneChip® Operating Software. SCIBLU - Swegene Centre for Integrative Biology at Lund University (http://www.lth.se/sciblu) conducted the quality controls, the RNA processing and the hybridization of the arrays. The samples representing undifferentiated hBS cells, differentiated contracting CMLC and a mixed population of differentiated cells were hybridized to the arrays. The arrays were run in duplicates.

### Data analysis

In order to define the family of genes that are up-regulated in the CMLC the fold change (FC) in gene expression between undifferentiated hBS cells and the three different biological replicates of CMLC was calculated. Notably, in these calculations the amplified and the un-amplified RNA samples were kept separate. Genes that were called as Absent by the MAS software in all samples were filtered and not included in further analyses. In the amplified samples 17,436 probes were called as Absent in all samples and in the un-amplified samples 18,534 probes were called as Absent in all samples and these genes were filtered from the dataset. When the FC-values had been calculated for each of the CMLC samples, the average of the FC-values (both amplified and un-amplified FC-values) were determined and subsequently used when filtering the genes. Genes with an average FC-value < 2 across all CMLC samples when compared to undifferentiated hBS cell samples were filtered from the data set and only the remaining 9,801 genes were considered for further analysis. Table I illustrates the distribution of up-regulated genes and summarizes the number of genes at specific FC levels (2, 3, 5, and 10).

**Table I. No. of remaining genes after applying different filters on the dataset.**

| **Filter** | **Experiment with amplified RNA** | **Experiment with non-amplified RNA** |
|---|---|---|
| Total number of genes on the array | 54,675 genes | 54,675 genes |
| Present call in at least one sample | 37,239 genes | 36,141 genes |
| FC undiff hBS - average CMLC >2 | 9,801 genes | |
| FC undiff hBS - average CMLC >3 | 6,090 genes | |
| FC undiff hBS - average CMLC >5 | 3,410 genes | |
| FC undiff hBS - average CMLC >10 | 1,607 genes | |

To further refine the selection method, a population of "mixed differentiated hBS cells" was included as control material to distinguish genes up regulated in all types of differentiated cells from genes specifically up regulated in cardiomyocyte-like cell clusters. The "mixed differentiated hBS cells" thus represent a population of spontaneously differentiated hBS cells where cells from all three germ layers mesoderm, endoderm and ectoderm may be present.

To identify genes that were specifically up-regulated in CMLC and not in the mixed differentiated hBS cell population, the following parameters were calculated:
1) Fold-change (FC) in gene expression between CMLC and undifferentiated hBS cells (FC_{CMLC})
2) FC in gene expression between the mixed differentiated hBS cells and undifferentiated hBS cells (FC_{MC})

For all calculations, the average of the three replicates of CMLC was used. The following criteria were used to sort out only genes that were specifically up-regulated in the CMLC:
1) Genes with Expression values below 500 in the CMLC were removed
2) Genes with FC_{CMLC} below 10 were removed
3) Genes with a FC_{CMLC} / FC_{MC} ratio below 10 were removed.

The remaining 56 genes are listed in Table II and these represent suitable marker genes for the CMLC.

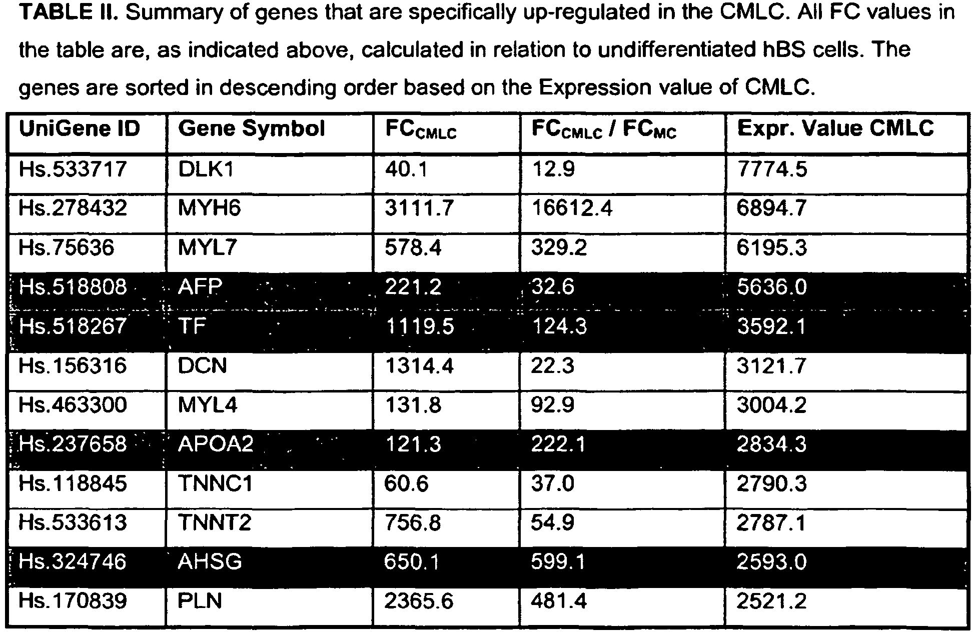

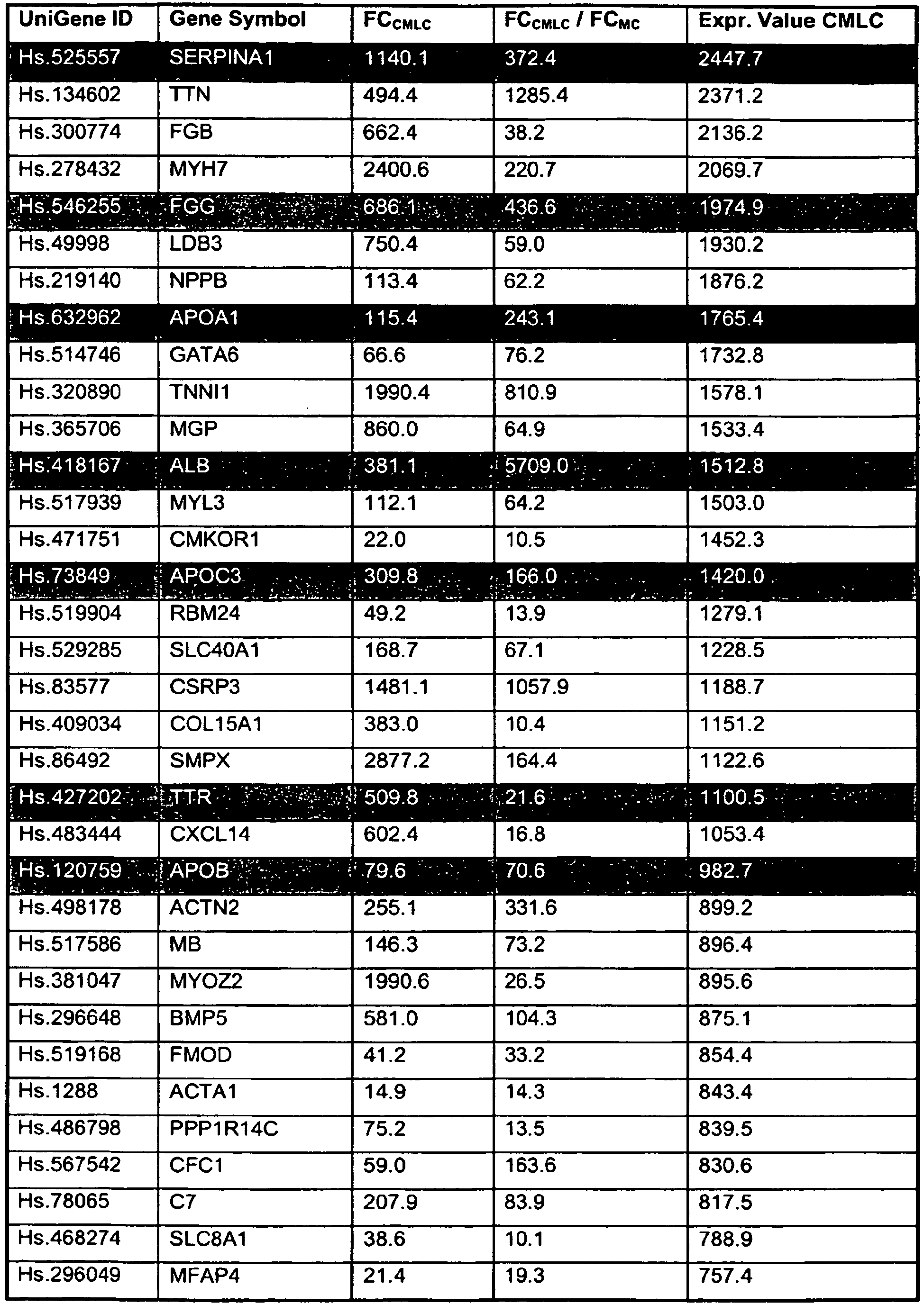

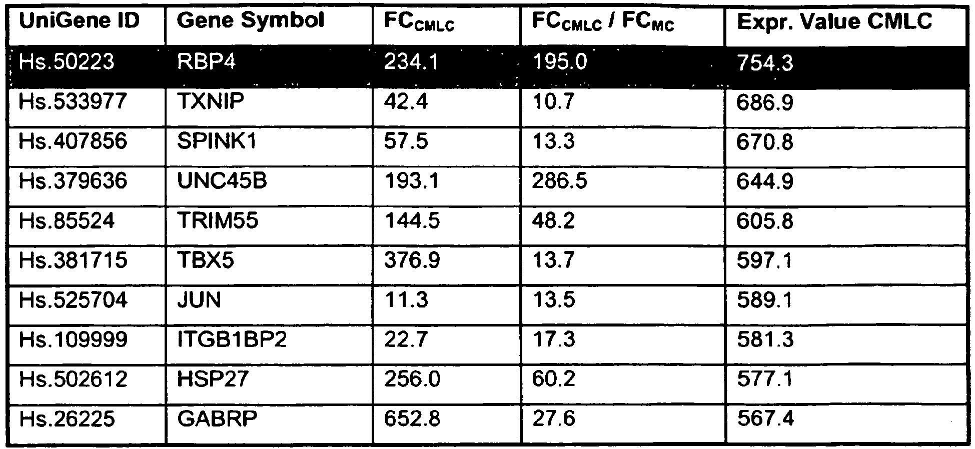

Of particular note is that the majority of the genes in Table II can be classified into two different groups. The first group consists of genes previously associated with cardiac cells and the second of genes previously associated with endodermal cells, such as hepatocytes. Examples of genes belonging to the first group are (marked in light grey in table II): MYH6, MYL7, MYL4, TNNC1, TNNT2, PLN, TTN, MYH7, LDB3, NPPB, GATA6, MYL3, CSRP3, ACTN2, MB, MYOZ2, TBX5, and HSP27. Examples of genes belonging to the second group are (marked in dark grey in table II): AFP, TF, APOA2, AHSG, SERPINA1, APOA1, ALB, APOC3, TTR, APOB, and RBP4.

To get an even stricter selection of genes specifically up-regulated in CMLC the criteria were narrowed to:
iv) Genes with Expression values below 2000 in the CMLC were removed
v) Genes with FC_{CMLC} below 100 were removed
vi) Genes with a FC_{CMLC} FC_{MC} ratio below 100 were removed.

The remaining 9 genes are listed in Table III and these represent suitable marker genes for the CMLC.

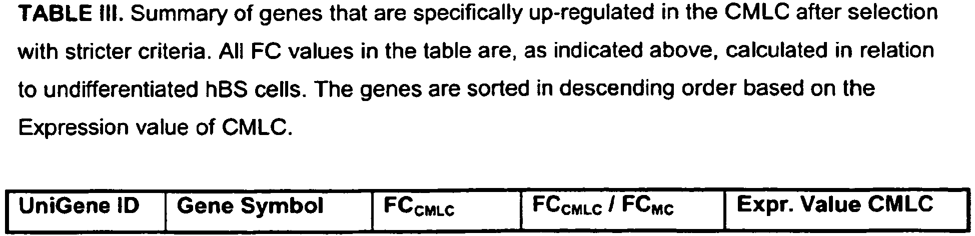

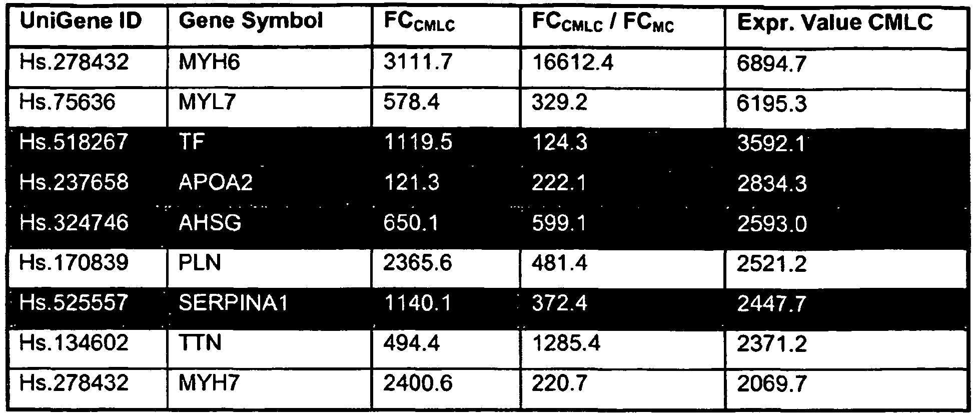

The selection shows an even distribution between endodermal and mesodermal genes expressed in the clusters. The mesodermal genes and the endodermal are marked in, light grey and dark grey respectively in Table III.

### Microarray analysis of cardiomyocyte-like clusters using significance analysis of microarrays (SAM) algorithm

By using the differentiation method and RNA extraction method described above, RNA were pooled at various time points (<22 days) after the onset of contraction.

Undifferentiated hBS were harvested at day 4-5 after passage for RNA extraction or differentiated to obtain CMLC using cells in passage 23-41. Spontaneously contracting clusters were identified by visual inspection using light microscopy and harvested by mechanical dissection. Specific care was taken only to harvest the beating areas with a minimum of surrounding non-contracting cells.

For each experiment, the material consisted of one pooled sample of undifferentiated hBS and two different biological replicates of pooled clusters.

### Data analysis

### Identification of differentially expressed genes

Genes that were significantly up- or down-regulated in the hBS-derived clusters compared to undifferentiated hBS were identified using the SAM algorithm (Tusher VG, Tibshirani R, Chu G. Significance analysis of microarrays applied to the ionizing radiation response. Proc Natl Acad Sci USA. 2001;98:5116-5121). Briefly, the algorithm assigns a score to each gene based on differences in expression between conditions relative to the standard deviation of repeated measurements. The false discovery rate (FDR) is determined by using permutations of the repeated measurements to estimate the percentage of genes identified by chance. The algorithm was applied to each of the data sets from the two experiments separately using FDR<0.04. Subsequently, only the genes marked as significantly up- or down-regulated in both data sets were considered as differentially expressed in CMLC compared to undifferentiated hBSs.

### Results

Using the SAM algorithm, and FDR<0.04, 530 genes were identified that were significantly up-regulated in clusters compared to undifferentiated hBS, figure 11. The fold change (FC_{CMLC}) given in figure 11 are equivalent to the results obtained in the above microarray analysis (Table II and III). Any differences in the numerical value of the fold change are due to the differences in statistical methods used for calculation. For a few of the genes, the FC values differ between the results presented in Table II and Table III. The figures have been calculated using the same raw data but the approach for calculating the average FC was not identical. For the genes in Table II, the individual probes on the GeneChips corresponding to each gene were used separately and only the probes that reached the inclusion criteria were subsequently used for the calculation of the average FC values. On the other hand, for the values in Table III all probes were used in order to first calculate the average of the probes corresponding to one gene and subsequently use that value for further calculations

In addition, we also identified a smaller group of 40 genes that were significantly down-regulated in the hBSC-derived CMLC compared to undifferentiated hBSC, figure 12. Among these are several genes that are associated with pluripotent hBSs, such as NANOG, TDGF1, POU5F1, LEFTY1, DPPA4, DNMT3B, and SOX2, demonstrating that CMLC represent a differentiated cell population. To explore if the up-regulated genes were previously known to be over-expressed in human heart tissue the Tissue Expression analysis in WebGestalt was used (as described in Zang et al., 2005). Of the 530 genes in Figure 11, 417 were identified by WebGestalt and 331 were marked as "expressed" in human heart tissue. One hundred of these genes (24%) are designated as significantly over-expressed in heart tissue (marked as category O Figure 11).In addition, 25 genes (6%) were designated as significantly under-expressed in heart tissue (marked as category U Figure 11). Interestingly, some of these genes (e.g., TF, FGG, TTR, and SERPINA1) were recorded as significantly over-expressed in human liver by WebGestalt, suggesting the presence of endodermal derivatives in the CMLCs. Of particular note, is that the gene MYH6 in was not identified by WebGestalt due to lack of a tissue expression record. For this reason, and in order to maintain consistency, MHY6 is not marked as significantly over-expressed in human heart in either of these tables. However, MYH6 has been reported to be a cardiac specific gene in other studies.

To characterize the family of up-regulated genes in hBSC-derived CMLCs, we performed a Gene Ontology analysis (GO) analysis. Gene Ontology (GO) annotations were used to group the genes according to biological process, molecular function, and cellular component. By comparing with a reference list, overrepresentation of annotations among sets of genes can be calculated as O/E where O is the observed number of genes with a specific annotation and E is the expected number of genes with that annotation. E is calculated as E = R * I /R where R is the number of reference genes and I is the number of interesting genes. All genes represented on the arrays were used as the reference list. Significantly overrepresented annotations (p<0.01) among the up-regulated genes from all three annotation categories at level 5 in the GO annotation database were identified using the hypergeometric test.

Notably, several of the significantly overrepresented GO annotation terms shown in Figure 8 are associated with cardiomyocyte properties and functions. For example, about 10% of the up-regulated genes were annotated as "calcium ion binding" in the Molecular function category. In addition, the results in the Biological process and Cellular component categories also support the presence of cardiac lineage in the hBSC-derived CMLC s. For instance, the up-regulated genes are typically associated with processes such as "muscle contraction", "cell differentiation", and "development". In addition, the cellular component annotations have a predominance of "cytoskeletal compartments" and "myofibrillar structures" typical for the contractile apparatus active in cardiomyocytes.

To investigate the possible interactions among proteins from the significantly up-regulated genes in CMLC, the search tool STRING (http://string.embl.de/) was used to mine for recurring instances of neighbouring genes (Snel B, Lehmann G, Bork P, et al. STRING: a webserver to retrieve and display the repeatedly occurring neighbourhood of a gene. Nucleic Acids Res. 2000;28:3442-3444..STRING aims to collect, predict and unify various types of protein-protein associations, including direct (physical) and indirect (functional) associations. Using the list of up-regulated genes in CMLC as input to STRING, 431 matches were made and potential interactions among products from these genes were investigated further. A protein interaction map was created from these gene products and compared with protein interaction maps from 10 different randomly generated sets of genes, all of equal size. For each protein, an interaction score was calculated as n*2/N, where n is the number of interactions (edges in the map) for the protein in question and N is the total number of input proteins. Furthermore, the number of hub proteins was determined. Following Han et al., we designated a protein as a hub if it had ≥ 5 interactions with other proteins (Han JD, Bertin N, Hao T, et al. Evidence for dynamically organized modularity in the yeast protein-protein interaction network. Nature. 2004;430:88-93. A distinction between "party hubs", which interact with most of their partners simultaneously, and "date hubs", which interact with their partners at different times or locations has also been made. Since our data sets do not include time series data we identified only "party hubs". As default STRING utilizes four different sources; Genomic context, High-throughput experiments, Co-expression, and Previous knowledge, to derive protein interaction maps. However, we restricted our analysis to include only experimentally determined protein interactions, excluding for example text mining, to increase the validity of the results.

As shown in Figure 9, the protein interactions are substantially more complex between proteins coded by genes that are up-regulated in hBSC-derived CMLCs than what is observed in randomly generated sets of proteins of equal size. For example, considerably more hub proteins (characterized by ≥ 5 interactions with other proteins) were identified and all the hub proteins interacted directly, or indirectly, with each other which resulted in a fully connected interaction network (Figure 10). In the interaction networks obtained from the randomly generated sets of genes, we typically observed a couple of smaller sub-networks that lacked interaction to each other.

In addition to identifying protein interaction networks among the differentially expressed genes we also here report significant up-regulation of a number of cellular pathways. Strikingly, 23 genes in the Focal adhesion pathway are significantly up-regulated in hBSC-derived CMLCs. The Focal adhesion pathway has been implicated in a diverse array of cellular processes, including tissue remodeling, cell migration, embryogenesis, growth factor signaling, cell cycle progression, and cell survival (Parsons et al 2003, Petit et al 2000). In addition, the role of focal adhesions in mechanotransduction in cardiomyocytes has recently been highlighted (Samarel et al 2005). Interestingly, besides affecting the beat-to-beat regulation of cardiac performance, mechanotransduction also influences the proliferation, differentiation, growth, and survival of the cellular components that comprise the human myocardium. Furthermore, in neonatal rat ventricular myocytes it has been reported that focal adhesion kinase regulates the activation of the MEF2 and JNK/c-Jun pathways, which have important roles in the early activation of the hypertrophic genetic program by mechanical stress in cardiomyocytes (Nadruz et al 2005).

Notably, 16 genes in the Calcium signaling pathway are also significantly up-regulated in the hBSC-derived CMLCs. This observation is not unexpected since Ca²⁺, as an intracellular messenger, is an important component for the initiation and regulation of cardiac contraction (Ferrier GR, Howlett SE. Cardiac excitation-contraction coupling: role of membrane potential in regulation of contraction. Am J Physiol Heart Circ Physiol. 2001;280:H1928-1944.). In addition, Ca²⁺ has been shown to be important already at the beginning of life to mediate the process of fertilization and later on it regulates some of the cell cycle events during early development (Berridge et al 1997). In this regard, the Hedgehog signaling pathway, also known to be critical in a plethora of developmental processes (Ingham et al 2006) is significantly up-regulated in the hBSC-derived CMLCs. Specifically, Sonic hedgehog appears to be a critical signaling factor for cardiac development in P19 cells (Gianakopoulos et al 2005). In addition, experimental mice lacking hedgehog signaling show a delay in the expression of Nkx2.5 (Gianakopoulos et al 2005).

### Reference Example 3. Real-time QPCR of CMLC derived from hBS cells

### Cell culture and RNA extraction

Spontaneously contracting CMLC were derived from undifferentiated hBS cells (SA002) (see Reference Example 1). The hBS were detached from the MEF feeder layer by incubation with collagenase IV (200 U/ml), for 10 - 15 minutes at 37 °C. The suspension was transferred to a 15 ml tube, and after the cells had sedimented, the supernatant was removed. The colonies were re-suspended in culture medium and dissociated mechanically into small aggregates of undifferentiated cells using a pipette. This cell suspension was distributed (200 µl/well) into a 96 well plate (Costar, Corning, untreated, v-bottom) and centrifuged for 5 min at 400 x g. The plate was then incubated at 37 °C for 6 - 8 days. The 3D structures that formed were then transferred to gelatine coated dishes containing culture medium. After a couple of days spontaneously contracting clusters of cells (CMLC) were observed. Some of these clusters were harvested after 0-2 weeks ("early CMLC") after onset of contraction while others were maintained in culture for at least 6 weeks ("late CMLC") before harvest. In parallel, undifferentiated hBS cells were harvested for subsequent RNA extraction.
1. Undifferentiated hBS (passage 35-37)
2. "Early CMLC" (passage 23-28, 32-34, 36-40, and 49)
3. "Late CMLC" (passage 35, 36, 39, 40, 43, 46 and 48)

Total RNA was extracted from undifferentiated hBS cells and CMLC using the RNeasy® Mini Kit with on-column DNase treatment (Qiagen).

### Quantitative real-time PCR

To investigate the gene expression level of specific ion-channels in the CMLC and undifferentiated hBS cells, TaqMan® low density arrays (384-Well Micro Fluidic Cards, Applied Biosystems) were used. For comparison, commercially available total RNA from human adult normal heart tissue (pool from 6 different male donors) (BioChain®) was analyzed in parallel. cDNA synthesis was performed using Superscript 3 (cat no 18080-051 Invitrogen). Each PCR reaction was run in quadruplicate. The relative gene expression levels were normalized against the expression of GAPDH and calculated according to the ΔΔC₁-method. The following genes were analyzed: POU5F1 (Oct-4), NANOG, NKX2.5, GATA4, TNNI3, DES, SCN5A, SCN1B, SCN2B, CACNA1C, CACNB1, CACNB2, CACNA2D1, CACNA1H, KCNQ1, KCNE1, KCNH2, KCNE2, KCNA5, KCNAB1, KCNAB2, KCNJ2, KCNJ12, KCNJ3, KCNJ5, KCNJ11, ABCC9, KCND3, KCNA4, Kcnip2, HCN4, KCNK1, CFTR, GJA5, GJA1, GJA7, ATP2A2, SLC8A1, RYR2, FKBP1B, SLC9A1, MYL2, MYLK, MYH6, and MYH7. The results from the PCR analysis are summarized in Figure 4. The panel of genes is focused on ion channel expression which plays an important role in cardiomyocyte function. For example, drugs specific for a cardiac ion channel can be tested on CMLC. Indicators such as action potential, beating frequency, and morphological appearance can be analyzed.

### Reference Example. 4. Cryopreservation and thawing of CMLC using vitrification

Cardiomyocyte-like cell clusters (CMLC) were derived from undifferentiated hBS cells as described in Reference Example 1. The clusters of spontaneously contracting cells were manually dissected and further divided into smaller clusters (about 0.2 - 0.3 mm in diameter). The CMLC were vitrified in closed straws as described in Patent application "Vitrification" publication number WO2004 098285 and stored in liquid N₂. A sterile filtered vitrification solution including vitro-PBS (Vitrolife AB) supplemented with 10% ethyleneglycol and 10% DMSO was used, as well as vitro-PBS (Vitrolife AB) supplemented with 0.3 M trehalose, 20% ethyleneglycol and 20% DMSO. The cells were recovered after thawing in culture medium (Knock Out DMEM supplemented with 20% FBS, 1% penicillin-streptomycin, 1% Glutamax, 0.5 mmol/l β-mercaptoethanol, and 1% non-essential amino acids) and the results summarized below.

| | |
|---|---|
| Experiment 1: | 10 CMLC were vitrified |
| | 7 CMLC were recovered from the straw |
| | 5 CMLC regained their spontaneous contraction |
| | → 70% recovery rate |
| | |
| Experiment 2: | 12 CMLC were vitrified |
| | 8 CMLC were recovered from the straw |
| | 6 CMLC regained their spontaneous contraction |
| | → 75% recovery rate |
| Experiment 3: | The results are summarized in Table IV below. |

**Table IV. Results from cryopreservation and thawing of CMLC derived from hBS cells**

| **Straw** | **# of vitrified CMLC** | **# of recovered CMLC** | **# of beating CMLC** | **Recovery rate** |
|---|---|---|---|---|
| No. 1 | 7 | 7 | 6 | 86% |
| No. 2 | 7 | 7 | 6 | 86% |
| No. 3 | 11 | 8 | 3 | 38% |
| No. 4 | 11 | 9 | 7 | 78% |
| No. 5 | 11 | 8 | 6 | 75% |
| No. 6 | 9 | 7 | 7 | 100% |

Additional experiments were also performed and are summarized below. The table below reports the collected data from four separate vitrification experiments. This data shows the percentage of cardiomyocyte clusters that are attached and those that are attached and spontaneously contracting at three, seven or 14 days after thawing.

| | **Day 3** | | **Day 7** | | **Day 14** | |
|---|---|---|---|---|---|---|
| | **Attached Clusters** | **Attached and Contracting Clusters** | **Attached Clusters** | **Attached and Contracting Clusters** | **Attached Clusters** | **Attached and Contracting Clusters** |
| **Average** | 90 | 52 | 90 | 57 | 84 | 59 |
| **St. Dev** | 21 | 37 | 12 | 37 | 20 | 36 |
| **# of Straws** | 32 | 32 | 27 | 27 | 32 | 32 |

### Example 1. Cluster characteristics

Colonies of undifferentiated hBS cells are separated from their feeder cells, using collagenase (200 U/ml, in DMEM/F12 medium). Colonies are collected either through passive sedimentation or via centrifugation. The colonies are then slightly broken up by gentle pipetting, and resuspended to give a final concentration of 4 average-sized colonies per 200 µl medium (Medium 1). Colonies are then centrifuged in a 96 well, v-bottom, uncoated plate (Corning article number: 3896) for 5 minutes at 400xg, with 200 µl (4 colonies) per well. The plates are then stored in an humidified incubator at 37 °C, with 5%CO₂ to allow 3D structures containing differentiated cells to form over a 3-8 day period. After this period, the 3D structures are transferred to gelatine coated Petri dishes and cultured for a period of two weeks in medium 2, below, with medium changes every two/three days.

### Medium 1 for 3D structure formation:

39.4 ml DMEM with glutamine and 1% penicillin/streptomycin
500 µl non essential amino acid
100 µl ß-mercapoethanol
10 ml foetal bovine serum

*Medium 2 for plating and culturing of 3D structures to allow for spontaneous differentiation:* as above, but with either 20% or 2% FBS to give a better differentiation.

### Cluster characteristics

When 3D structures are plated at age 7 days, on gelatine in medium containing 20% foetal bovine serum, clusters with contracting cells (CMLC) have the following characteristics (n= 55):
Beat frequency
   average beats per minute: 45.4 ±25.9
   median beats per minute: 40
Length
   average: 619 ± 440 µm
   median: 500 µm
Width
   average: 330 ± 298 µm
   median: 260 µm

The distribution of beating frequency is affected by both the plating age of the 3D structures from which the cardiomyocytes-like cell clusters are obtained, and the culturing conditions after the plating of the 3D structures.

### Reference Example 5. Cryopreservation of cardiomyocyte-like cell clusters using slow freezing

Cardiomyocyte-like cell clusters (CMLC) were derived from undifferentiated hBS cells as described in Reference Example 1. The clusters of spontaneously contracting cells were manually dissected and further divided into smaller clusters (about 0.2 - 0.3 mm in diameter). In some experiments, the cell clusters were cryopreserved and in others the clusters were dissociated into single cell suspensions using trypsin/EDTA (15 min at 37°C). The cell suspensions or cell clusters were transferred to cryopreservation media. The following different media compositions have been tested with comparable results regarding recovery of viable cells post-thawing:
1. (KO-DMEM, NEAA, β-MeOH, Glutamax, PEST, 20 % FBS) + 10 % DMSO 2.90 % FBS + 10 % DMSO
3. 95 % FBS + 5 % DMSO
4. 45 % KO-DMEM + 45 % FBS + 10 % DMSO
5. 50 % KO-DMEM + 45 % FBS + 5 % DMSO
6. 50 % Trypsin/EDTA + 40 % FBS + 10 % DMSO
7. 20 % KO-DMEM + 50 % Trypsin/EDTA + 20 % FBS + 10 % DMSO

### Reference Example 6. Use of cardiomyocyte-like cell clusters for in vitro pharmacological testing

Cardiomyocyte-like cell clusters (CMLC) were derived from undifferentiated hBS cells as described in Reference Example 1 and cryopreserved as described in Reference Example 4. The CMLC were thawed and pharmacological test were performed using different platforms, such as microelectrode arrays, voltage-clamp analysis, transmembrane action potential (TAP) measurements, and QT-screen system. The use of the functional clusters is further illustrated in figure 30.

### Reference Example 7: Analysis of hERG channel functionality in CMLC

Spontaneously beating CMLC were derived and isolated from hBS cells as described in Reference Example 1. The CMLC were plated on microelectrode arrays (MEA). After adhesion of the CMLC to the MEA surface, the electrical activity was recorded as extracellular field potential by the substrate integrated electrodes of the MEA. Cardiac field potential properties have been studied before and their correlation with cardiac action potential data has been well described (Banach et al Am. J. Physiol. 2003, 284 (6): H2114 -H2123). The addition of the QT-prolonging drug E-4031 causing hERG channel blockade was measured as a delayed repolarisation of the cardiac field potentials. Increasing drug concentrations (0, 100pM, 1 nM, 10nM, 100nM, 1000nM) was added to obtain cumulative dose response curves. Notably E-4031 did not affect beating frequency over the experiments but caused a sustained prolongation in the low nanomolar concentrations of the cardiac field potential. This is indicative for a hERG channel block known to be caused by this compound. This example is illustrated in figure 2b of the poster, see figure 7a and b.

In addition, similar experiments were performed using other substances such as Astemizol (antihistaminic) and Dofetilide (a class III anti arrhythmic drug). As shown in Figure 5a, Astemizol caused a selective block and sustained prolongation of the repolarizing component at low nanomolar concentrations. Similar effects were observed using Dofetilide (Figure 5b). Furthermore, Terfenadine (an antihistamine, withdrawn from the US market in 1997 due to QT prolongation effects) and Cisapride (a serotonin receptor agonist, withdrawn from the US market in 2000 due to QT prolongation effects) also demonstrated dose dependent prolongation of the action potential (indicator of QT prolongation due to a ventricular repolarization disturbance) (Figure 5c and 5d).

### Reference Example 8. Effect of combined treatment with Activin A and bFGF on cardiomyocyte-like cell clusters

CMLC were derived from undifferentiated hBS cells (cell line SA002, LOTAL002, passage 21, 22, 44 and 51) essentially as described in Reference Example 1. In order to improve the yield of CMLC from hBS cells, the culture medium used before plating of the 3D structures (pre-plating) was supplemented with Activin A (10 ng/ml) and bFGF (12 ng/ml).

The number of contracting CMLC was visually determined at day 6 or 7 after plating of formed 3D structures. In three experiments, there was on average 4.7 times more beating CMLC in cultures that had been treated with Activin A and bFGF during the pre-plating period compared to control cultures not receiving the treatment (Figure 6E). In one experiment, 3D structures were plated in CM medium supplemented with 2% FBS instead of 20% FBS. In this experiment, the yield of contracting CMLC 7 days after plating was increased 2.5-fold by the combined pre-plating treatment with Activin A and bFGF (data not shown). The effect of the treatment on beating area characteristics was determined in terms of distribution in beating frequency, length, width, calculated area and structure in two experiments; one using plating medium with 20% FBS and one with plating medium containing 2% FBS. The experiment set ups and characteristics of contracting CMLC are described in the table below (n = 7 - 14).

| **Group** | **Control, 20% FBS** | **Activin A+bFGF, 20% FBS** | **Control, 2% FBS** | **Activin A+bFGF, 2% FBS** |
|---|---|---|---|---|
| **Medium pre-plating** | CM medium (w 20% FBS) | CM medium (w 20% FBS), Activin A+bFGF | CM medium (w 2% FBS) | CM medium (w 2% FBS), Activin A+bFGF |
| **Medium post-plating** | CM medium (w 20% FBS) | CM medium (w 20% FBS) | CM medium (w 2% FBS) | CM medium (w 2% FBS) |
| **Beat frequency (BPM)** | 44 ± 26 | 51 ± 14 | 61 ± 17 | 49 ±20 |
| **Length (µm)** | 200 ± 89 | 279 ± 133 | 171 ± 95 | 228 ± 112 |
| **Width (µm)** | 317 ± 103 | 416 ± 234 | 229 ± 119 | 356 ± 178 |
| **Area (µm² x 100)** | 700 ± 497 | 1281 ± 897 | 457 ± 421 | 947 ± 1015 |

Total RNA was isolated from mixed cultures, including CMLC, from the above mentioned experiments on day 7 after plating and transcribed into cDNA. Quantitative real-time PCR was performed using the Taqman labelling system and data were normalized to the endogenous control CREBBP. The expression of genes involved in early (Nkx2.5 and GATA4) and late (Troponin T2 and alpha-cardiac actin) cardiac differentiation was increased by adding Activin A and bFGF to the medium during the pre-plating period in combination with plating in CM medium supplemented with both 20% FBS (Figure 6A - D) and 2% FBS (data not shown). This indicates that the number of cardiomyocytes was increased by the Activin A and bFGF treatment. The gene expression of Flk1, CD31 and alpha-actin was also increased by the combined treatment with Activin A and bFGF in these experiments (data not shown).

### Reference Example 9. Effects of medium composition, supplements, and plating day on CMLC formation from hBS cells.

By modifying the basic protocol described in Reference Example 1 it is possible to increase the yield of CMLC. In this example some of these opportunities are illustrated.

The serum component used has an effect on the yield of beating CMLC from hBS cells. In a set of experiments different FBS preparations were used; standard FBS (as in Reference Example 1) (Invitrogen, art nr 10108-165), dialyzed FBS (Invitrogen, art nr 26400-036), and charcoal stripped FBS (Invitrogen, art nr 12676-011). The cells were maintained in the presence of the different FBS (20%) during culture in the 96-well plates. The aggregates formed were plated after 3 days in new dishes coated with gelatine and subsequent culture was done in the presence of standard FBS (20%). The number of beating CMLC was determined 14 days after plating and the results are shown in Figure 13. The results indicate that there is a trend of increased yield by using the stripped FBS and by screening a large number of FBS batches it should be possible to identify an optimal batch.

In addition to modulating the initial phase of differentiation (i.e., in the 96-well plates) it is also possible to stimulate later stages of differentiation to increase the yield of contracting CMLC from hBS cells. In this regard, hBS cells were differentiated in the absence or presence of Activin A and bFGF as described in Reference Example 8. The differentiated cells (3D-aggregates) were plated at day 7 in medium supplemented with 1000U/ml LIF. After 4 days the medium was changed to medium without LIF. The number of beating CMLC was counted at day 7 and day 14 after plating and the results are shown in Figure 14. Notably, treatment with Activin A and bFGF increased the yield of CMLC and the additional treatment with LIF further increased the generation of beating CMLC, especially at the early time point (7 days).

As an alternative to using protein growth factors for modulation of the differentiation of hBS cells it is possible to use low molecular weight compounds. Undifferentiated hBS cells were treated essentially as described in Reference Example 8. In order to improve the yield of CMLC from hBS cells, the culture medium used before plating was supplemented with 5 µM SB 216763 (GSK-3 inhibitor) or 5 µM SKF-86002 (p38 MAP-kinase inhibitor) in addition to Activin A (10 ng/ml) and bFGF (12 ng/ml). The number of contracting CMLC was visually determined at day 7 after plating of formed 3D structures and the results are shown in Figure 15. The yield of beating CMLC was almost 3 times higher in cultures that had been treated with either SB 216763 or SKF-86002 during the pre-plating period compared to control cultures not receiving additional factors. These results show that both SB 216763 and SKF-86002 have a positive effect on the number of beating CMLC derived from hBS cells. It is also possible that a combination of the two compounds can add further to the yield of CMLC from hBS cells.

The day at which the 3D-aggregates are being plated after onset of differentiation also affect the yield of beating CMLC. In a series of experiments, 3D-aggreates of differentiating hBS cells were generated as described in Reference Example 1. The 3D-aggregates were subsequently plated at day 7 (control) or earlier (day 3, 4, or 5) in new culture dishes. The number of spontaneously beating CMLC was visually determined using a light microscope and the data are summarized in Figure 16. Plating earlier than day 7 significantly increases the final yield of CMLC.

### Reference Example 10. Transmembrane action potential measurements: Pharmacology and safety testing

### Methods

Transmembrane action potentials (TAP) were recorded by using a microelectrode filled with 3 M KCI (resistance 10 to 20 MΩ) connected via an Ag/AgCl junction to a high impedance amplifier (Intracellular Electrometer IE-210, Warner Instrument Corporation). Sharp microelectrodes were prepared using a glass, and a micromanipulator was used to lower the electrode into the bath and enter the cluster. The same impalement was kept for as long as possible. The action potentials were recorded on a PC (sampling frequency 1 and 20 kHz) and the different action potential characteristics were analyzed using custom-designed software (Pharm-Lab v6.0b4, AstraZeneca) to determine AP duration at 50%, 70% and 90% of repolarization (dur50, dur70, dur90), AP amplitude (amp), membrane resting potential (MRP), and maximum rate of rise of the AP upstroke (Vmax). Steady-state recordings of action potentials at spontaneous frequency or during electric stimulation of 1-4 Hz were made. All calculations were made on the average of 10 (consecutive) action potentials. The bath was continuously perfused with Tyrode's solution consisting of (in mmol/L) 130 NaCl, 4 KCI, 0.5 MgSO4•7H20, 1.8 NaH2PO4xH2O, 18 NaHCO3, 5.5 glucose, 1.8 CaCl2 and kept at 37°C.

CMLC were derived from hBS cells as described in Reference Example 1 and 8 and the clusters were grown on gelatine coated silicon pieces and on cover glasses, which allowed for easy transfer into the bath, and eliminated the problem of keeping the cluster at the same position. Electrical stimulation of the CMLC was done using field stimulation by means of a mobile electrode.

0.5-1 µM E-4031 was used for testing the effect of blocking the rapid rectifier potassium current IKr. Change in APD was calculated and used to determine prolongation and assess triangularization.

To investigate the role of the funny current (If) in spontaneously contracting CMLC, 10 µM zatebradine was administered.

### Results

The cells were characterized as either nodal-like, atrial-like, or ventricle-like based on the results of transmembrane action potential (TAP) recordings. The characteristics of action potentials recorded from beating CMLC are shown in Figure 17 and are summarized in the table below. Data are ± SD. n indicates number of clusters of that type; bpm, beats per minute; RR, distance between APs, APD50/APD75/APD90, duration measured at 50%, 75%, or 90% repolarization; amp, AP amplitude; Vmax, maximum rate of depolarizing upstroke velocity.

**Table 2**

| | | **HR (bpm)** | **RR (ms)** | **dur 60 (ms)** | **dur 75 (ms)** | **dur 90 (ms)** | **amp (mV)** | **Vmax (V/s)** |
|---|---|---|---|---|---|---|---|---|
| **Ventricle** | n=42 | 81.10±30.74 | 894.72±440.96 | 147.30+36.97 | 194.82±47.29 | 220.00±50.80 | 60.51±17.45 | 14.40±8.99 |
| **Artia** | n=63 | 151.13±39.98 | 447.46±186.61 | 69.72±15.47 | 96.44±17.47 | 114.71±20.34 | 68.66±13.69 | 21.46±13.04 |
| **Nodal** | n=21 | 130.34±46.71 | 533.54±201.99 | 58.46±15.99 | 81.70±17.17 | 99.03±18.70 | 53.13±13.83 | 11.16±9.34 |

Characteristics of action potentials recorded from beating CMLC derived from hBS cells.

Although there is variability between individual CMLC, examples of close to fully mature ventricular APs were found. As an example, Figure 18 shows a ventrical action potential with a dur 90 of 341ms and an amplitude of 97mV. Out of recordings from 145 different clusters, 24 showed APD(90) above 200ms.

Repeated recordings were made at different locations in the same beating CMLC cluster, showing a low variability within the clusters (Figure 19).

In the native heart, cardiomyocytes respond to increased beating frequency by shortening of APD. This rate adaptation is fundamental and can be impaired in some diseases. To test the ability of hBS-cell derived CMLC to respond equally to a change in beating frequency, clusters were paced by means of electrical field stimulation and APD90 was compared at different frequencies. Figure 20 shows that the APD90 is frequency dependent and that clusters with an initial APD90 over 180 ms displayed rate adaptation as pacing frequency increased (1-3Hz). Clusters with a short APD90 were only limitedly affected by a change in pacing frequency.

Spontaneous contractile activity in CMLC clusters is due to funny currents (If). If can be blocked by Zatebradine. Application of 10 µM of Zatebradine slowed beating frequency and increased duration as shown in Figure 21 The results are also summarized in the table below.

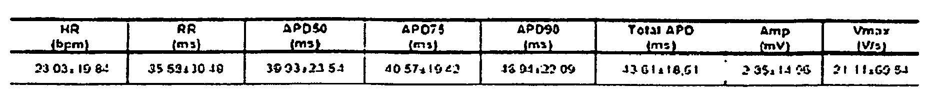

Change in % after administration of 10 µM of If blocker Zatebradine on atrial-like hESC-CM clusters (n=4).

The proarrhytmic potential was assayed using E-4031 which is a potent IKr blocker. E-4031 induced concentration dependent action potential prolongation in all CMLC phenotypes as summarized in the table below. It also caused triangularization as an effect of the drug, triggered activity, and in some cases EADs were detected (Figure 22 and 23).

**Table 5**

| **Table 5** | **0.5 µM E-4031** | | | **1 µM E-4031** | | |
|---|---|---|---|---|---|---|
| | **Spontaneous f** | **1 Hz** | **2 Hz** | **Spontaneous f** | **1 Hz** | **2 Hz** |
| **Ventricle-like** | 16.67±10.64 (5) | 34.89±9.83 (4) | 27.92±9.23 (2) | | 59.33±31.28 (2) | 38.14±36.85 (3) |
| **Atrial-like** | 27.27±7.61 (7) | | | 70.08±25.91 (2) | | |
| **Nodal-like** | 43.16±21.00(4) | | | | | |

Average change in percent of APD90 ± SD (n) for ventricular-, atrial-, and nodal-like CMLC APs

### Reference Example 11

### Addition of Activin A, bFGF, SB 216763 and SKF-86002 during 3D aggregate formation

3D aggregate cultures were derived from undifferentiated hBS cells essentially as described in Reference Example 1. The cell line used for derivation was SA002, LOTAL002 (passage 25, 62 and 68). In order to improve the yield of CMLC from hBS cells, the culture medium used before plating was supplemented with different combinations of 1 - 25 uM SB 216763 (GSK-3 inhibitor), 1 - 25 uM SKF-86002 (p38 MAP-kinase inhibitor) in addition to 1 - 20 ng/ml Activin A and 1.2 - 24 ng/ml bFGF. Three concentrations in each range were tested according to the scheme below. The number of contracting CMLC was visually determined (Figure 24), and expression of the heart related genes Troponin T2, Nkx2.5 and GATA4 was measured by means of real-time RT quantitative PCR, at day 5 - 7 after plating of formed 3D structures. The experiment was repeated three times.

### Experimental set up

| Group | Activin A (ng/ml) | bFGF (ng/ml) | SB 216763 (µm) | SKF-86002 (µm) |
|---|---|---|---|---|
| 1 | 1 | 1.2 | 1 | 1 |
| 2 | 20 | 24 | 1 | 1 |
| 3 | 1 | 1.2 | 25 | 1 |
| 4 | 20 | 24 | 25 | 1 |
| 5 | 1 | 1.2 | 1 | 25 |
| 6 | 20 | 24 | 1 | 25 |
| 7 | 1 | 1.2 | 25 | 25 |
| 8 | 20 | 24 | 25 | 25 |
| 9 | 10 | 12 | 5 | 5 |
| 10 | 10 | 12 | 5 | 5 |
| 11 | 10 | 12 | 5 | 5 |

The combination of factors that produced the highest number of beating areas related to the amount of start material was 20 ng/ml Activin A, 24 ng/ml bFGF, 25 µM SB 216763 and 1 µM SKF-86002 (group 4, Figure 24). However, other concentrations of these factors might also have an equal positive effect or be even more potent. The gene expression of Troponin T2, Nkx2.5 and GATA4 was closely related to the number of beating areas per colony (data not shown).

### Reference Example 12. Immunohistochemical analysis of CMLC derived from hBS cells.

Spontaneously contracting CMLC were derived from undifferentiated hBS cells (SA002) essentially as described in Reference Example 1. The hBS cells were detached from the MEF feeder layer by incubation with collagenase IV (200 U/ml), for 10 - 15 minutes at 37 °C. The suspension was transferred to a 15 ml tube, and after the cells had sedimented, the supernatant was removed. The colonies were re-suspended in culture medium and dissociated mechanically into small aggregates of undifferentiated cells using a pipette. This cell suspension was distributed (200 µl/well) into a 96 well plate (Costar, Coming, untreated, v-bottom) and centrifuged for 5 min at 400 x g. The plate was then incubated at 37 °C for 3-4 days. The 3D structures that formed were then transferred to gelatine coated dishes containing culture medium. After a couple of days spontaneously contracting clusters of cells (CMLC) were observed and 4 to 6 weeks thereafter all CMLC clusters were harvested for cryosectioning, i.e. they were manually isolated, washed two times in 1xDPBS (GIBCO) buffer, incubated for 10 minutes in 10% sucrose, embedded in Tissue-Tek (Histolab) and finally frozen in -80 °C. Cryosections (18 µm) of 4 - 6 weeks CMLC clusters, as well as undifferentiated hBS cells on IVF plates, were fixed in 4% PFA and blocked with 1% BSA/0.01% Triton X-100 in 1xDPBS (GIBCO) buffer. The primary antibodies were diluted in blocking buffer and incubated over night at 4°C. Three 5 minutes washes in 1xDPBS buffer were followed by two hours incubation of diluted secondary antibody at room temperature. Nuclei were visualized using blue-fluorescent 4',6-diamindino-2-phenylindole (DAPI, 1:1000). After three times washes with DPBS, slides were mounted in DakoCytomation Fluorescent Mounting Medium (DAKO) and imaged with a Nikon E400 fluorescent microscope. Sections from at least 10 - 15 different CMLC clusters were analysed for each marker and the results were consistent across all the samples. All antibodies were diluted as fallows: rabbit monoclonal anti cardiac Troponin I (Abcam, ab52862) 1:250, mouse-IgG2b anti Oct-3/4 (Santa Cruz, SC-5279) 1:500, mouse-IgG3 anti SSEA-4 (Santa Cruz, SC-21704) 1:200, and mouse-IgM anti TRA-1-60 (Santa Cruz, SC-21705) 1:200. Secondary antibodies were diluted as fallows; donkey anti-rabbit AlexaFluor594 1:500 and donkey anti-mouse AlexaFluor488 1:500 both bought from Molecular Probe Inc (Oregon, USA).

In conclusion, no expression of the three well defined hBS cell markers (Oct-3/4, SSEA-4, and TRA-1-60) could be detected in hBS cell derived CMLC (Figure 25-27). As a positive control for the hBS cell markers, undifferentiated colonies of hBS cells were stained under the same conditions, using the same protocols and these cells stained strongly positive for all the markers. We also used an antisera against the specific cardiac cell marker cardiac Troponin I (cTnI) in order to characterize the CMLC.

### Reference Example 13. The effect of Zatebradine on CMLC.

CMLC were derived from undifferentiated hBS cells essentially as described in Reference Example 1 using cell line SA002. Zatebradine is a substance known to block so called funny channels, e.i. hyperpolarization-activated, cyclic nucleotide-gated (HCN) channels 1-4, which are involved in the regulation of cardiomyocyte contraction and cardiac rate. In order to investigate the effect of Zatebradine on CMLC derived from hBS cells and to correlate this effect to age and gene expression, Zatebradine was administered at a concentration of 5 µM to 32 spontaneously beating clusters of different ages. The contraction rate was registered visually every other minute until the effect of the substance had declined. Gene expression was quantified using real-time RT quantitative PCR. The beating of some clusters was completely blocked by Zatebradine administration. On average, Zatebradine decreased the beating frequency of CMLC about 50 % (Figure 28). Of the four known mammalian HCN isoforms, HCN4 is the one most highly expressed in the sinoatrial node and is therefore considered the most important in generating and determining pacemaker activity. The gene expression of HCN4, as well as Troponin T2, was positively correlated to the beating frequency, i.e. the more HCN4 or Troponin T2 expression the higher contraction rate (Figure 28). Moreover, the expression of HCN4 decreased with the age of the hES-CMC (Figure 28), while there was no correlation between Troponin T2 and age (data not shown). As expected, the gene expression of HCN4 was positively related to the Troponin T2 expression (data not shown).

### Reference Example 14. Immunohistochemical analysis of CMLC derived from hBS cells.

Cryosections of CMLC clusters were prepared and used for immunohistochemcial analysis as described in Reference Example 12. Nuclei were visualized using blue-fluorescent 4',6-diamindino-2-phenylindole (DAPI). The primary antibodies used were: rabbit polyclonal anti KCNH2 (Abcam, ab32585) and rabbit monoclonal anti cardiac Troponin I (Abcam, ab52862). The results are shown in Figure 29 and positive staining was obtained using the anti-hERG (KCNH2) antibody in areas of the CMLC clusters that co-express the specific cardiac cell marker cardiac Troponin I (cTnI).

### Example 2. Functional receptors

To test whether the cell clusters has functional receptors, the following method described in Norström et.al. 2006 can be used. The following pharmacological agents are used: noradrenaline and adrenaline (Apoteket, Umeå, Sweden); phenylephrine and forskolin (Sigma Chemicals, St.Louis, Mo,USA); phenoxybenzamine and acetylcholine (KeLab, Göteborg, Sweden); atenolol and atropin (NM Pharma AB, Täby, Sweden); labetalol (Glaxo Smith Kline, Mölndal, Sweden); verapamil (Abbot Scandinavia AB, Solna, Sweden).

Phenylephrine, an α1-adrenoceptor agonist, is administered to contracting areas at increasing concentrations in 30 min intervals. Contractile activity is stimulated in a dose-dependent manner (see Table 1 in Norström et al.). The stimulatory effect is more pronounced in specimens with low basal contraction frequency. It is noted how many beating areas that are non-responsive to phenylephrine. Phenylephrine (10-7 M), administered to arrested areas, initiates contractile activity promptly. The stimulatory effect of phenylephrine is counteracted by phenoxybenzamine, a blocker of α1-and α2- receptors. Irregular contractions can occasionally be observed at maximal stimulation by phenylephrine but normally returns to regular beats following inhibition by phenoxybenzamine. If primarily administrated to contracting clusters of cells, phenoxybenzamine, is expected to reduce the contraction frequency. This effect is immediate and may be followed by a successive restitution of contraction frequency. The inhibition can be reversed by phenylephrine.

Adrenaline, exhibiting predominantly ß1-adrenoceptor agonistic effects in the myocardium, can be used to test stimulated contractile activity. The stimulatory effect is registered within minutes and maximal stimulation occurs at a certain. In specimens exhibiting low contraction frequency (<20bpm) an increase of frequency may be observed at certain concentrations. The stimulatory effect is reduced within minutes following administration of atenolol, a ß-receptor blocker. Likewise, labetolol, a ß- and α1-receptor blocker can be used to show a reduction in the stimulation of contraction frequency exerted by adrenaline. Atenolol (10-7 M), may reduced spontaneous contractile activity within 2 min.

Noradrenaline, an α- and ß1- adrenoceptor agonist, stimulates contractile activity normally in a concentration-response manner. Maximal stimulation is reached within 10 min and this response can be reversed by phenoxybenzamine. Noradrenaline (10-7 M) administered to an arrested cell can initiate contractile activity within minutes.

Acetylcholine, exerting muscarinic effect in the myocardium, reduces the contractile activity. Following administration of 10-4-10-3 M acetylcholine total inhibition may be observed and it may persist for hours. Atropin, which competes with acetylcholine for a common binding site on the muscarinic receptor, counteracts the inhibition by acetylcholine. Primarily administered atropin (10-6 M) stimulates spontaneous contractile activity.
Verapamil, a blocker of the trans-membranous flow of Ca2+ ions and inhibitor of mobilization of intracellular Ca 2+, reduces or abolishes the contractile activity, depending on the concentration primarily administered. Spontaneous contractions of basal frequency can be reestablished following washing and exchange of medium within 30 min.

Forskolin is generally recognized as a stimulator of adenylate-cyclase and formation of cyclic adenosine monophosphate (cAMP). A stimulatory response may be registered after 30 min of treatment with 10-10-10-6 M of forskolin as compared with baseline frequency. Two beating areas are exposed to pico-molar concentrations of forskolin. In these areas the contractile activity may be inhibited at 10-12 M during registration between 5 and 30 min but stimulated at higher concentrations of forskolin, though an initial but transient inhibition may be observed 5 min after administration.

Based on the variation in spontaneous contraction frequency of cell clusters obtained from different EBs it is likely to assume that the cells represent atrial, ventricular as well as nodal cardiac muscle cells. Nevertheless, their response to chronotropic agents was principally similar although, generally, in cell clusters exhibiting low beat frequency, the response to adrenoceptor agonists was more pronounced. Thus, our results (Table 1) support the notion of the development of functioning both adrenergic and cholinergic mechanisms as an early event in embryonic cardiomyocyte differentiation as indicated previously (3, 4, 6). The endogenous spontaneous contractile activity and the response to pharmacological agents appeared unaffected by repeated experiments since the basal contractile activity returned after washing and the pharmacological response could be repeated after weeks in culture. Exceptionally, contracting clusters did not respond to any chronotropic agent which may imply an impediment of receptor differentiation. It has previously been shown that the response to adrenoceptor agonists is time-dependent and better recognized in more differentiated cardiomyocytes (4).

### References

Anderson D, Self T, Mellor IR, et al. Transgenic Enrichment of Cardiomyocytes From Human Embryonic Stem Cells. Mol Ther. 2007
Ameen C, Strehl R, Bjorquist P, Lindahl A, Hyllner J, Sartipy P., Human embryonic stem cells: Current technologies and emerging industrial applications., Crit Rev Oncol Hematol. 2007 Aug 3
Burridge PW, Anderson D, Priddle H, Barbadillo Munoz MD, Chamberlain S, Allegrucci C, Young LE, Denning C. , Improved human embryonic stem cell embryoid body homogeneity and cardiomyocyte differentiation from a novel V-96 plate aggregation system highlights interline variability. Stem Cells. 2007 Apr;25(4):929-38.
Berridge MJ. Elementary and global aspects of calcium signalling. J Physiol. 1997;499 (Pt 2):291-306.
Gianakopoulos PJ, Skerjanc IS. Hedgehog signaling induces cardiomyogenesis in P19 cells. J Biol Chem. 2005;280:21022-21028.
He et al Human embryonic stem cells develop into multiple types of cardiac myocytes: action potential characterization. Circ Res, 93: 32-39, 2003
Heins N, Englund MCO, Sjöblom C et al. Derivation, characterization, and differentiation of human embryonic stem cells. STEM CELLS 2004;22:367-76.
Ingham PW, Placzek M. Orchestrating ontogenesis: variations on a theme by sonic hedgehog. Nature reviews. 2006;7:841-850.
Itskovitz-Eldor J, Schuldiner M, Karsenti D, Eden A, Yanuka O, Amit M, Soreq H, Benvenisty N., Differentiation of human embryonic stem cells into embryoid bodies compromising the three embryonic germ layers., Mol Med. 2000 Feb;6(2):88-95.
Kehat et al Human embryonic stem cells can differentiate into myocytes with structural and functional properties of cardiomyocytes. J Clin Invest, 108: 407- 5 414, 2001
Keller G., Embryonic stem cell differentiation: emergence of a new era in biology and medicine. Genes Dev. 2005 May 15;19(10):1129-55. Review.
MacLennan DH, Kranias EG. Phospholamban: a crucial regulator of cardiac contractility. Nat Rev Mol Cell Biol. 2003;4:566-577
Mummery et al Differentiation of human embryonic stem cells to cardiomyocytes: role of coculture with visceral endoderm-like cells. Circulation, 107: 2733-2740, 2003
Nadruz W, Jr., Corat MA, Marin TM, et al. Focal adhesion kinase mediates MEF2 and c-Jun activation by stretch: role in the activation of the cardiac hypertrophic genetic program. Cardiovasc Res. 2005;68:87-97.
Norstrom A, Akesson K, Hardarson T, Hamberger L, Bjorquist P, Sartipy P. Molecular and pharmacological properties of human embryonic stem cell-derived cardiomyocytes. Exp Biol Med (Maywood). 2006 Dec;231(11):1753-62.
Parsons JT. Focal adhesion kinase: the first ten years. J Cell Sci. 2003;116:1409-1416.
Passier R, Mummery C., Cardiomyocyte differentiation from embryonic and adult stem cells. Curr Opin Biotechnol. 2005 Oct; 16(5):498-502. Review
Petit V, Thiery JP. Focal adhesions: structure and dynamics. Biol Cell. 2000;92:477-494.
Reubinoff BE, Pera MF, Fong CY, Trounson A, Bongso A., Embryonic stem cell lines from human blastocysts: somatic differentiation in vitro., Nat Biotechnol. 2000 Apr; 18(4):399-404. Erratum in: Nat Biotechnol 2000 May; 18(5):559.
Samarel AM. Costameres, focal adhesions, and cardiomyocyte mechanotransduction. Am J Physiol Heart Circ Physiol. 2005;289:H2291-2301.
Singh AM, Terada N. Bypassing heterogeneity: the road to embryonic stem cell-derived cardiomyocyte specification. Trends Cardiovasc Med. 2007;17:96-101
Sjögren-Jansson E, Zetterström M, Moya K, Lindqvist J, Strehl R, Eriksson PS. Largescale propagation of four undifferentiated human embryonic stem cell lines in a feeder-free culture system, Dev Dyn. 2005 Aug;233(4):1304-14.
Snir M, Kehat I, Gepstein A, Coleman R, Itskovitz-Eldor J, Livne E, Gepstein L., Assessment of the ultrastructural and proliferative properties of human embryonic stem cell-derived cardiomyocytes., Am J Physiol Heart Circ Physiol. 2003 Dec; 285(6):H2355-63.
Stenoien DL, Knyushko TV, Londono MP, et al. Cellular trafficking of phospholamban and formation of functional sarcoplasmic reticulum during myocyte differentiation. Am J Physiol Cell Physiol. 2007;292:C2084-2094.
Thomson JA, Itskovitz-Eldor J, Shapiro SS, Waknitz MA, Swiergiel JJ, Marshall VS, Jones JM., Embryonic stem cell lines derived from human blastocysts., Science. 1998 Nov 6;282(5391):1145-7. Erratum in: Science 1998 Dec 4; 282(5395):1827.
Xu et al Characterization and enrichment of cardiomyocytes derived from human embryonic stem cells Circ Res, 91: 501-508, 2002
Xue T, Cho HC, Akar FG, Tsang SY, Jones SP, Marban E, Tomaselli GF, Li RA. Functional integration of electrically active cardiac derivatives from genetically engineered human embryonic stem cells with quiescent recipient ventricular cardiomyocytes: insights into the development of cell-based pacemakers, Circulation. 2005 Jan 4;111(1):11-20.
Zhang B, Kirov S, Snoddy J. WebGestalt: an integrated system for exploring gene sets in various biological contexts. Nucleic Acids Res. 2005;33:W741-748.
Junying Yu, Maxim A. Vodyanik, Kim Smuga-Otto, Jessica Antosiewicz-Bourget, Jennifer L. Frane, Shulan Tian, Jeff Nie, Gudrun A. Jonsdottir, Victor Ruotti, Ron Stewart, Igor I. Slukvin, James A. Thomson. Induced Pluripotent Stem Cell Lines Derived fromHuman Somatic Cells, Published 20 November 2007 on Science Express.
Yoon BS, Yoo SJ, Lee JE, You S, Lee HT, Yoon HS., Enhanced differentiation of human embryonic stem cells into cardiomyocytes by combining hanging drop culture and 5-azacytidine treatment. Differentiation. 2006 Apr;74(4):149-59. Erratum in: Differentiation. 2006 Jul;74(6):322
   - WO2004098285, Cryopreservation of human blastocyst stem cells by use of a closed straws vitrification method
   - WO03055992, A method for the establishment of a pluripotent human blastocyst-derived stem cell line, Cellartis AB
   - WO2004098285, Cryopreservation of human blastocyst stem cells by use of a closed straws vitrification method
   - WO2004099394, A method for the efficient transfer of human blastocystderived stem cells from a feeder-supported to a feeder-free culture system
   - WO2005/012510, Method for the isolation and expansion of cardiac stem cells from biopsy
   - WO2006/052925, Cardiac stem cells

### Specific embodiments are as follows:

### Item

1. A method for the preparation of a cluster comprising cardiomyocyte-like cells, the method comprising the steps of:
   i) suspending and dissociating undifferentiated hBS cells in a culture medium, wherein said medium is a cell culture base medium, such as Knock Out Dulbecco Modified Eagles Medium (DMEM) or Modified Eagle Medium (MEM), optionally supplemented with one or more of serum, such as fetal bovine serum, fetal calf serum, human serum or serum replacement, penicillin-streptomycin, GlutaMAX™ -mercaptoethanol and non-essential amino acids
   ii) subjecting the thus dissociated aggregates to forced aggregation,
   iii) incubating the thus forced aggregated cell aggregates in said culture medium optionally comprising one of more growth factors to obtain one or more 3D structures,
   iv) transferring one or more 3D structures to one or more plates and incubating the 3D structures in said culture medium optionally comprising one or more growth factors to develop them into one or more clusters comprising contracting cells, wherein said medium used in step iii) and/or iv) comprises a member GSK-3 inhibitors, Activin A as a member from the transforming growth factor beta superfamily where the concentration of Activin A supplemented to the culture medium is 5-40 ng/ml, and bFGF as a member from the fibroblast growth factor family where the concentration of bFGF supplemented to the culture medium is 5-40 ng/ml.
2. A method according to item 1 further comprising the step of isolating one or more clusters by removing one or more clusters from said plate.
3. A method according to item 1 or 2, wherein the forced aggregation is performed by centrifugation, optionally performed at 100-800 x g for 2-20 minutes, such as at 200-600 x g for 5-10 minutes, or 300-500 x g for 5-10 minutes, or at 400 x g for 5 minutes.
4. A method according to item 1, wherein forced aggregation is performed by sedimentation for about 1-36 hours, such as for about 2-24 hours or for about 3-12 hours.
5. A method according to any of items 3-5, wherein the cells obtained after sedimentation and/or centrifugation are incubated for 1-10 days in step iii), such as 1-7 days or 1-5 days, for the formation of 3D structures.
6. A method according to any of the preceding items, wherein the development of clusters in step iv) is for 1-30 days, such as 1-15 days, 1-10 days, or 1-5 days, until the formation of cardiomyocyte-like cell clusters.
7. A method according to any of the preceding items, wherein the dissociation in step i) is performed mechanically.
8. A method according to item 1, wherein the concentration of each of the one or more growth factors is from about 5 to about 40 ng/ml.
9. A method according to item 1 or 8, wherein the concentration of Activin A supplemented to the culture medium is about from 5 to 30 ng/ml, such as from 5 to 20 ng/ml, from 5 to 15 ng/ml such as 8 ng/ml, 9 ng/ml, 10 ng/ml, 11 ng/ml, or 12 ng/ml, and wherein the concentration of bFGF supplemented to the culture medium is from 5 to 30 ng/ml, such as from 5 to 20 ng/ml, from 5 to 15 ng/ml such as 10 ng/ml, 11 ng/ml, 12 ng/ml, 13 ng/ml or 14 ng/ml.
10. A method according to any of the preceding claims, wherein said medium used in step i) and/or step ii) and/or iii) of item 1 comprises Activin A, bFGF and/or FBS.
11. A method according to any of the preceding items, wherein said medium used in step iii) and/or iv) of claim 1 comprises a p38 MAP kinase inhibitor and wherein the member of the GSK-3 inhibitor family is SB 216763 and the member of the p38 MAP-kinase inhibitor family is SKF-860002, wherein the concentration of the GSK-3 inhibitor supplemented to said medium is from 1 to 25 µM, such as from 2 to 10 µM or 5 µM and/or the concentration of the p38 MAP-kinase inhibitor supplemented to the culture medium is from 1 to 25 µM, such as from 2 to 10 µM, or 5 µM.
12. A method according to any of the preceding items, wherein said medium used in step iii) and/or step iv) of claim 1 further comprises between 100-2000 U/ml LIF, such as between 500-1500 U/ml, or 1000 U/m and wherein the medium comprising LIF is replaced after 2-8 days of incubation, such as after 4-5 days, with a medium without LIF.
13. A method according to any of the preceding items, wherein the plate(s) in step iii) of item 1 has a gelatine coated surface.
14. A cluster comprising cardiomyocyte-like cells obtainable by the method defined in any of items 1-13, wherein the cluster has:
   i) contracting cells,
   ii) cells that are electrically connected,
      and expresses
   iii) cardiac markers including Nkx.2.5, troponin and myosin,
   iv) markers for functional adrenergic receptors,
   v) markers for functional muscarinic receptors, vi) markers for functional ion-channels including hERG, Na+, Ca2+ and K+ channels,
   vii) one or more endodermal markers selected from the group consisting of AFP, TF, APOA2, AHSG, SERPINA1, APOA1, APOC3, TTR, APOB, and RBP4.
   viii) absence of expression of one or more of the following markers for undifferentiated cells: OCT-3/4, SSEA-4, TRA-1-60, and
   the cluster comprising genes that are up-regulated and have,
   i) expression values of 500 or more,
   ii) a fold change in gene expression between cardiomyocyte-like cells and undifferentiated hBS cells (FC_{CMLC}) of 10 or more.
   iii) a ratio between FC_{CMLC} and FC_{MC} (i.e. the fold change between mixed differentiated hBS cells and undifferentiated hBS cells) of 10 or more, and the cluster comprises cells expressing all of the following genes: 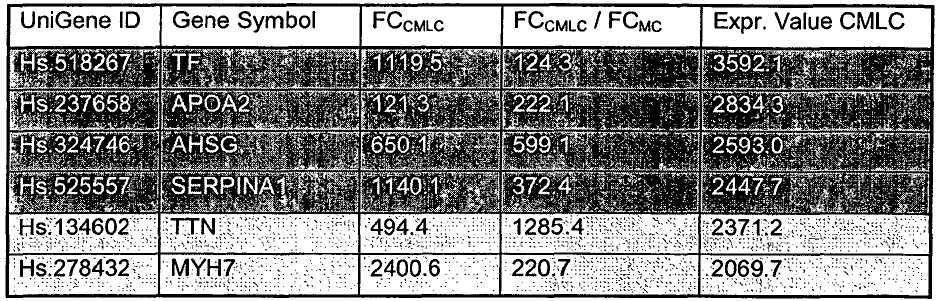
15. A cluster according to item 14, wherein the cluster comprises cells expressing at least 10, at least 15, at least 20, at least 25 or all of the following genes: 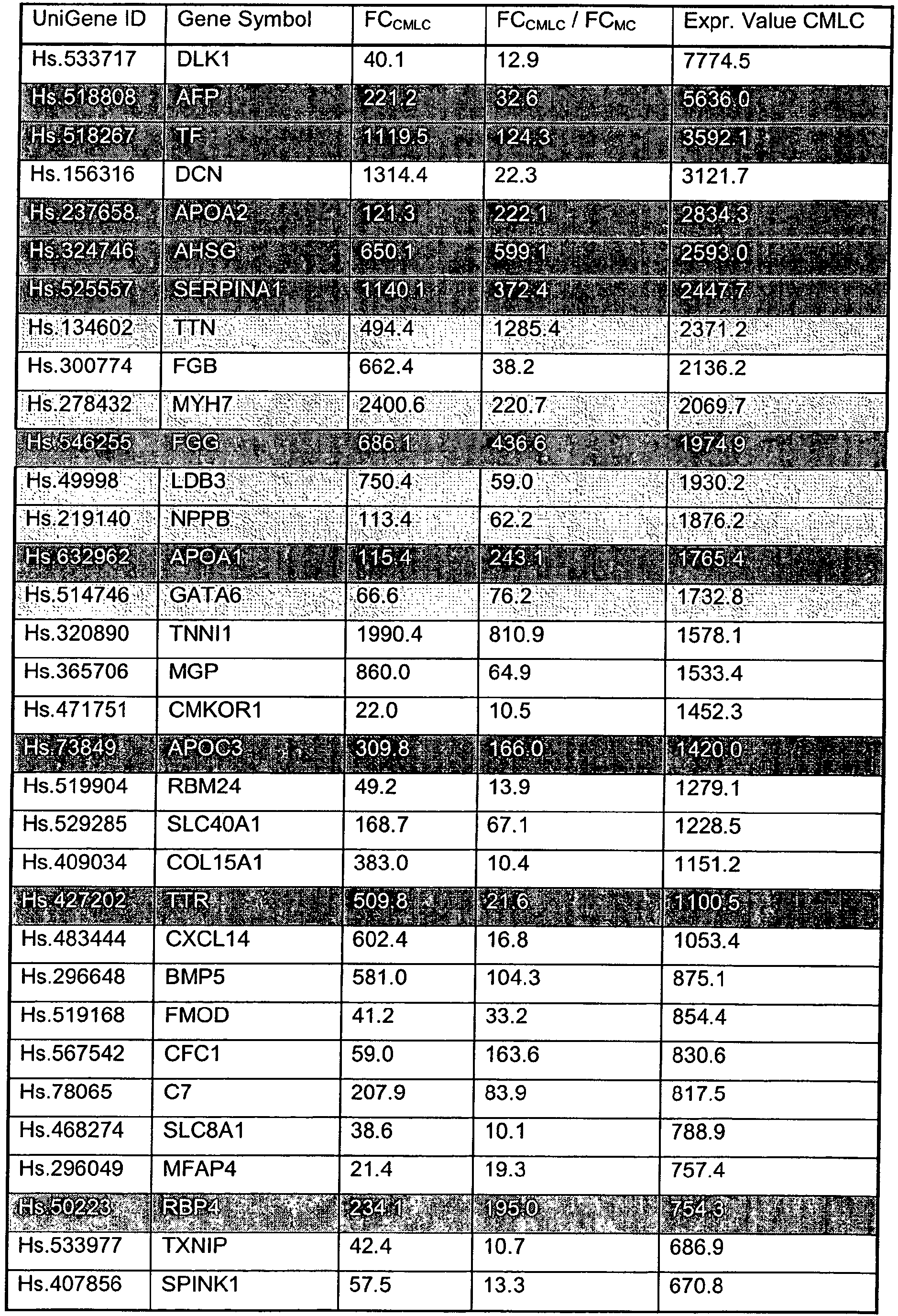 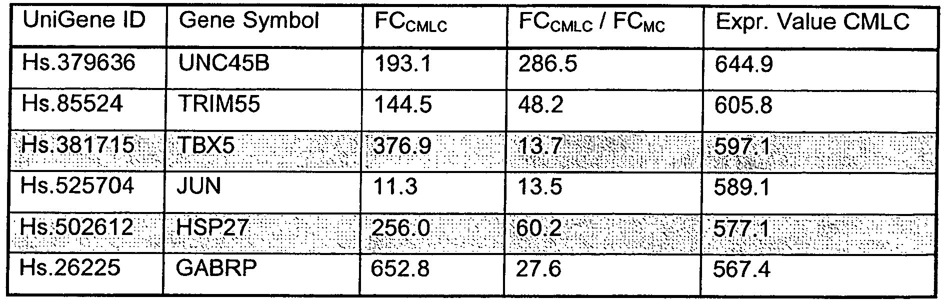
16. A cluster according to any of items 14-15, derived from hBS cells, with one or more of the following properties:
   i) the derived hBS cells are trisomic hBS cells carrying an extra chromosome 13,
   ii) the derived hBS cells are xeno-free hBS cells, or the cluster derived from hBS cells is xeno-free,
   iii) the cluster contains from about 10 to about 5000 cells.
17. A cluster according to any of items 14-16, with one or more of the following genetic properties:
   i) 2 or more such as, e.g., 4 or more, 6 or more, 8 or more, 10 or more, 12 or more or 16 or more of the up-regulated genes are genes associated with cardiac cells (described in Table II herein).
   ii) 2 or more such as, e.g., 4 or more, 6 or more, 8 or more of the up-regulated genes are genes associated with endodermal cells (described in Table II herein).
   iii) 2 or more such as, e.g., 4 or more, 6 or more, 8 or more of the up-regulated genes are genes associated with non cardiac or non endodermal cells, described in Table II herein.
   iv) the up-regulated genes comprise 10 or more such as, e.g., 20 or more, 30 or more, 40 or more, 50 or more, 55 or more or all genes listed in Table II herein.
18. A composition of cardiomyocyte-like clusters according to any of items 14-17, wherein the clusters contain a mixture of nodal-like cells, atrial-like cells ventricle-like cells.
19. A composition of cardiomyocyte-like clusters according to item 18, wherein the ratio between the number of nodal-like cells and the number of clusters containing atrial-like cells is in a range of from about 1:100 to about 50:100 such as from about 10:100 to about 40:100, from about 20:100 to about 40:100, from about 30:100 to about 40:100 or about 33:100-34:100 or wherein the ratio between the number of nodal-like cells and the number of ventricle-like cells is in a range of from about 1:100 to about 80:100 such as from about 10:100 to about 70:100, from about 30:100 to about 70:100, from about 45:100 to about 55:100 or about 50:100 or wherein the ratio between the number of ventricle-like cells and the number atrial-like cells is in a range of from about 1:100 to about 90:100 such as from about 40:100 to about 80:100, from about 50:100 to about 75:100 or about 66:100.
20. A composition of cardiomyocyte-like clusters according to item 18, wherein the ratio between the nodal-like cells, the atrial-like cells and the ventricle-like cells is 17:50:33.

## Claims

1. A method for the preparation of a cluster comprising cardiomyocyte-like cells, the method comprising the steps of:
i) suspending and dissociating undifferentiated human embryonic stem (hES) cells in a culture medium, wherein said medium is a cell culture base medium, such as Knock Out Dulbecco Modified Eagles Medium (DMEM) or Modified Eagle Medium (MEM), optionally supplemented with one or more of serum, such as fetal bovine serum, fetal calf serum, human serum or serum replacement, penicillin-streptomycin, GlutaMAX™-mercaptoethanol and non-essential amino acids
ii) subjecting the thus dissociated aggregates to forced aggregation,
iii) incubating the thus forced aggregated cell aggregates in said culture medium optionally comprising one of more growth factors to obtain one or more 3D structures,
iv) transferring one or more 3D structures to one or more plates and incubating the 3D structures in said culture medium optionally comprising one or more growth factors to develop them into one or more clusters comprising contracting cells, wherein said medium used in step iii) and/or iv) comprises a GSK-3 inhibitor, Activin A as a member from the transforming growth factor beta superfamily where the concentration of Activin A supplemented to the culture medium is 5-40 ng/ml, and bFGF as a member from the fibroblast growth factor family where the concentration of bFGF supplemented to the culture medium is 5-40 ng/ml.

2. A method according to claim 1 further comprising the step of isolating one or more clusters by removing one or more clusters from said plate.

3. A method according to claim 1 or 2, wherein the forced aggregation is performed by centrifugation, optionally performed at 100-800 x g for 2-20 minutes, such as at 200-600 x g for 5-10 minutes, or 300-500 x g for 5-10 minutes, or at 400 x g for 5 minutes.

4. A method according to claim 1, wherein forced aggregation is performed by sedimentation for about 1-36 hours, such as for about 2-24 hours or for about 3-12 hours.

5. A method according to any of claims 3-5, wherein the cells obtained after sedimentation and/or centrifugation are incubated for 1-10 days in step iii), such as 1-7 days or 1-5 days, for the formation of 3D structures.

6. A method according to any of the preceding claims, wherein the development of clusters in step iv) is for 1-30 days, such as 1-15 days, 1-10 days, or 1-5 days, until the formation of cardiomyocyte-like cell clusters.

7. A method according to any of the preceding claims, wherein the dissociation in step i) is performed mechanically.

8. A method according to claim 1, wherein the concentration of each of the one or more growth factors is from about 5 to about 40 ng/ml.

9. A method according to claim 1 or 8, wherein the concentration of Activin A supplemented to the culture medium is about from 5 to 30 ng/ml, such as from 5 to 20 ng/ml, from 5 to 15 ng/ml such as 8 ng/ml, 9 ng/ml, 10 ng/ml, 11 ng/ml, or 12 ng/ml, and wherein the concentration of bFGF supplemented to the culture medium is from 5 to 30 ng/ml, such as from 5 to 20 ng/ml, from 5 to 15 ng/ml such as 10 ng/ml, 11 ng/ml, 12 ng/ml, 13 ng/ml or 14 ng/ml.

10. A method according to any of the preceding claims, wherein said medium used in step i) and/or step ii) and/or iii) of claim 1 comprises Activin A, bFGF and/or FBS.

11. A method according to any of the preceding claims, wherein said medium used in step iii) and/or iv) of claim 1 comprises a p38 MAP kinase inhibitor and wherein the member of the GSK-3 inhibitor family is SB 216763 and the member of the p38 MAP-kinase inhibitor family is SKF-860002, wherein the concentration of the GSK-3 inhibitor supplemented to said medium is from 1 to 25 µM, such as from 2 to 10 µM or 5 µM and/or the concentration of the p38 MAP-kinase inhibitor supplemented to the culture medium is from 1 to 25 µM, such as from 2 to 10 µM, or 5 µM.

12. A method according to any of the preceding claims, wherein said medium used in step iii) and/or step iv) of claim 1 further comprises between 100-2000 U/ml LIF, such as between 500-1500 U/ml, or 1000 U/m and wherein the medium comprising LIF is replaced after 2-8 days of incubation, such as after 4-5 days, with a medium without LIF.

13. A method according to any of the preceding claims, wherein the plate(s) in step iii) of claim 1 has a gelatine coated surface.

14. A cluster comprising cardiomyocyte-like cells obtainable by the method defined in any of claims 1-13, wherein the cluster has:
i) contracting cells,
ii) cells that are electrically connected,
and expresses
iii) cardiac markers including Nkx.2.5, troponin and myosin,
iv) markers for functional adrenergic receptors,
v) markers for functional muscarinic receptors, vi) markers for functional ion-channels including hERG, Na+, Ca2+ and K+ channels,
vii) one or more endodermal markers selected from the group consisting of AFP, TF, APOA2, AHSG, SERPINA1, APOA1, APOC3, TTR, APOB, and RBP4.
viii) absence of expression of one or more of the following markers for undifferentiated cells: OCT-3/4, SSEA-4, TRA-1-60, and
the cluster comprising genes that are up-regulated and have,
i) expression values of 500 or more,
ii) a fold change in gene expression between cardiomyocyte-like cells and undifferentiated hES cells (FC_{CMLC}) of 10 or more,
iii) a ratio between FC_{CMLC} and FC_{MC} (i.e. the fold change between mixed differentiated hES cells and undifferentiated hES cells) of 10 or more, and the cluster comprises cells expressing all of the following genes:

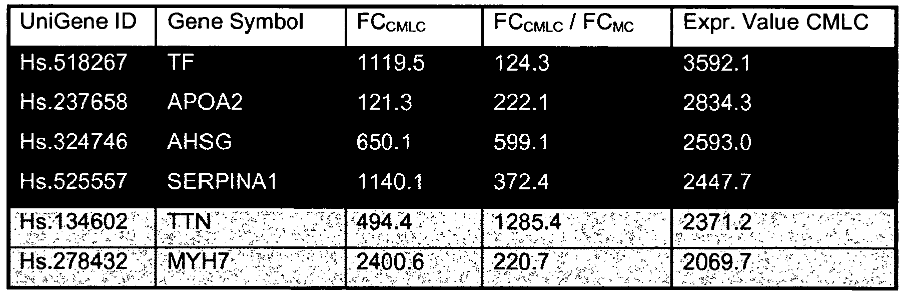

15. A cluster according to claim 14, wherein the cluster comprises cells expressing at least 10, at least 15, at least 20, at least 25 or all of the following genes:

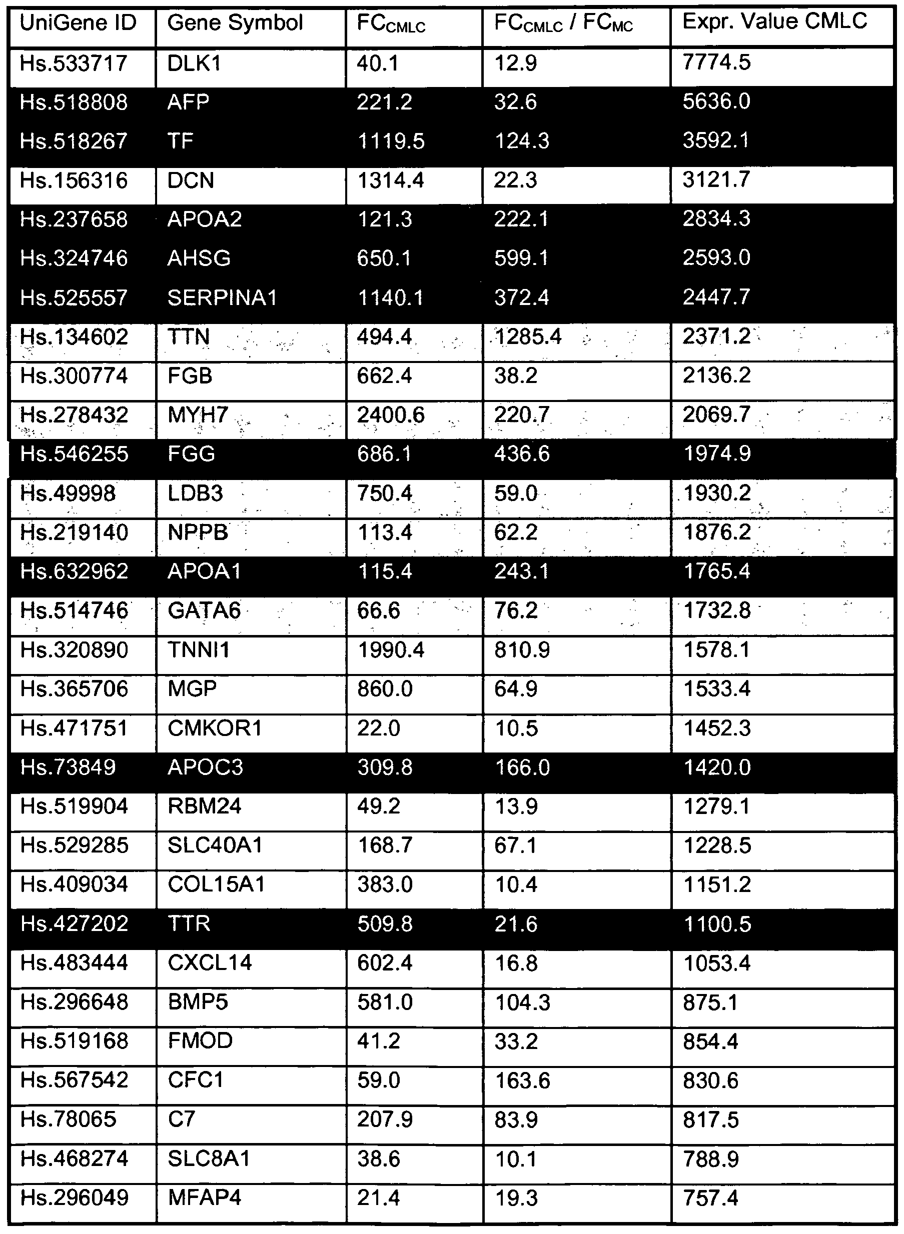

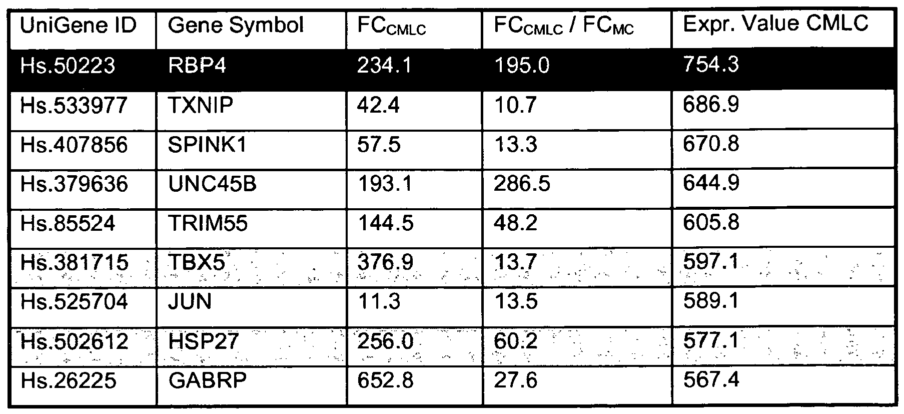

16. A cluster according to any of claims 14-15, derived from hES cells, with one or more of the following properties:
i) the derived hES cells are trisomic hES cells carrying an extra chromosome 13,
ii) the derived hES cells are xeno-free hES cells, or the cluster derived from hES cells is xeno-free,
iii) the cluster contains from about 10 to about 5000 cells.

17. A cluster according to any of claims 14-16, with one or more of the following genetic properties:
i) 2 or more such as, e.g., 4 or more, 6 or more, 8 or more, 10 or more, 12 or more or 16 or more of the up-regulated genes are genes associated with cardiac cells (described in Table II herein);
ii) 2 or more such as, e.g., 4 or more, 6 or more, 8 or more of the up-regulated genes are genes associated with endodermal cells (described in Table II herein);
iii) 2 or more such as, e.g., 4 or more, 6 or more, 8 or more of the up-regulated genes are genes associated with non cardiac or non endodermal cells, described in Table II herein;
iv) the up-regulated genes comprise 10 or more such as, e.g., 20 or more, 30 or more, 40 or more, 50 or more, 55 or more or all genes listed in Table II herein.

18. A composition of cardiomyocyte-like clusters according to any of claims 14-17, wherein the clusters contain a mixture of nodal-like cells, atrial-like cells ventricle-like cells.

19. A composition of cardiomyocyte-like clusters according to claim 18, wherein the ratio between the number of nodal-like cells and the number of clusters containing atrial-like cells is in a range of from about 1:100 to about 50:100 such as from about 10:100 to about 40:100, from about 20:100 to about 40:100, from about 30:100 to about 40:100 or about 33:100-34:100 or wherein the ratio between the number of nodal-like cells and the number of ventricle-like cells is in a range of from about 1:100 to about 80:100 such as from about 10:100 to about 70:100, from about 30:100 to about 70:100, from about 45:100 to about 55:100 or about 50:100 or wherein the ratio between the number of ventricle-like cells and the number atrial-like cells is in a range of from about 1:100 to about 90:100 such as from about 40:100 to about 80:100, from about 50:100 to about 75:100 or about 66:100.

20. A composition of cardiomyocyte-like clusters according to claim 18, wherein the ratio between the nodal-like cells, the atrial-like cells and the ventricle-like cells is 17:50:33.

## Patentansprüche

1. Verfahren zur Herstellung eines Clusters, umfassend kardiomyozytenähnliche Zellen, welches Verfahren die folgenden Schritte umfasst:
i) Suspension und Dissoziation undifferenzierter humaner embryonaler (HES) Stammzellen in einem Kulturmedium, wobei das Medium ein Zellkultur-Basismedium wie etwa Knock Out Dulbecco modifiziertes Eagles Medium (DMEM) oder modifiziertes Eagle Medium (MEM) ist, eventuell ergänzt mit einem oder mehreren von Serum wie etwa fetalem Rinderserum, fetalem Kälberserum, humanem Serum oder Serumersatz, Penicillin-Streptomycin, GlutaMAX™ -Mercaptoethanol und nicht-essentiellen Aminosäuren
ii) Unterziehen der so dissoziierten Aggregate einer erzwungenen Aggregation,
iii) Inkubieren der so erzwungenen aggregierten Zellaggregate in das Kulturmedium eventuell umfassend einen von mehreren Wachstumsfaktoren zur Erhaltung einer oder mehrerer 3D-Strukturen,
iv) Übertragen einer oder mehrerer 3D-Strukturen an eine oder mehrere Platten und Inkubieren der 3D-Strukturen in das Kulturmedium eventuell umfassend einen oder mehrere Wachstumsfaktoren, um sie zu einem oder mehreren Clustern zu entwickeln, umfassend kontrahierende Zellen, wobei das im Schritt iii) und/oder iv) verwendete Medium einen GSK-3-Hemmer, Aktivin A als ein Mitglied der Superfamilie des transformierenden Wachstumsfaktors beta, wobei die dem Kulturmedium zugegebene Konzentration von Aktivin A 5-40 ng/ml beträgt, und bFGF als ein Mitglied der Familie des Fibroblastenwachstumsfaktors umfasst, wobei die dem Kulturmedium zugegebene Konzentration von bFGF 5-40 ng/ml beträgt.

2. Verfahren nach Anspruch 1 zusätzlich umfassend den Schritt des Isolierens eines oder mehrerer Cluster durch Entfernen eines oder mehrerer Cluster von der Platte.

3. Verfahren nach Anspruch 1 oder 2, wobei die erzwungene Aggregation durch Zentrifugation durchgeführt wird, die eventuell bei 100-800 x g für 2-20 Minuten wie etwa bei 200-600 x g für 5-10 Minuten oder 300-500 x g für 5-10 Minuten oder bei 400 x g für 5 Minuten durchgeführt wird.

4. Verfahren nach Anspruch 1, wobei die erzwungene Aggregation durch Sedimentation für ungefähr 1-36 Stunden wie etwa für ungefähr 2-24 Stunden oder für ungefähr 3-12 Stunden durchgeführt wird.

5. Verfahren nach einem der Ansprüche 3-5, wobei die nach der Sedimentation und/oder Zentrifugation erhaltenen Zellen für 1-10 Tage im Schritt iii) wie etwa 1-7 Tage oder 1-5 Tage zur Bildung von 3D-Strukturen inkubiert werden.

6. Verfahren nach einem der vorhergehenden Ansprüche, wobei die Entwicklung von Clustern im Schritt iv) 1-30 Tage wie etwa 1-15 Tage, 1-10 Tage oder 1-5 Tage beträgt, bis die kardiomyozytenähnlichen Cluster gebildet werden.

7. Verfahren nach einem der vorhergehenden Ansprüche, wobei die Dissoziation im Schritt i) mechanisch durchgeführt wird.

8. Verfahren nach Anspruch 1, wobei die Konzentration von jedem des einen oder mehrerer Wachstumsfaktoren ungefähr 5 bis ungefähr 40 ng/ml beträgt.

9. Verfahren nach Anspruch 1 oder 8, wobei die dem Kulturmedium zugegebene Konzentration von Aktivin A ungefähr 5 bis 30 ng/ml wie etwa 5 bis 20 ng/ml, 5 bis 15 ng/ml wie etwa 8 ng/ml, 9 ng/ml, 10 ng/ml, 11 ng/ml oder 12 ng/ml beträgt, und wobei die dem Kulturmedium zugegebene Konzentration von bFGF 5 bis 30 ng/ml wie etwa 5 bis 20 ng/ml, 5 bis 15 ng/ml wie etwa 10 ng/ml, 11 ng/ml, 12 ng/ml, 13 ng/ml oder 14 ng/ml beträgt.

10. Verfahren nach einem der vorhergehenden Ansprüche, wobei das im Schritt i) und/oder Schritt ii) und/oder iii) von Anspruch 1 verwendete Medium Aktivin A, bFGF und/oder FBS umfasst.

11. Verfahren nach einem der vorhergehenden Ansprüche, wobei das im Schritt iii) und/oder iv) von Anspruch 1 verwendete Medium einen p38 MAP-Kinasehemmer umfasst, und wobei das Mitglied der GSK-3-Hemmerfamilie SB 216763 ist, und das Mitglied der p38 MAP-Kinasehemmerfamilie SKF-860002 ist, wobei die dem Medium zugegebene Konzentration des GSK-3-Hemmers 1 bis 25 µM wie etwa 2 bis 10 µM oder 5 µM beträgt, und/oder die dem Kulturmedium zugegebene Konzentration des p38 MAP-Kinasehemmers 1 bis 25 µM wie etwa 2 bis 10 µM oder 5 µM beträgt.

12. Verfahren nach einem der vorhergehenden Ansprüche, wobei das im Schritt iii) und/oder iv) von Anspruch 1 verwendete Medium zusätzlich zwischen 100-2000 U/ml LIF wie etwa zwischen 500-1500 U/ml oder 1000 U/m beträgt, und wobei das LIF umfassende Medium nach 2-8 Tagen der Inkubation wie etwa nach 4-5 Tagen mit einem Medium ohne LIF ersetzt wird.

13. Verfahren nach einem der vorhergehenden Ansprüche, wobei die Platte bzw. Platten im Schritt iii) von Anspruch 1 eine gelatinebeschichtete Oberfläche aufweist bzw. aufweisen.

14. Cluster umfassend kardiomyozytenähnliche Zellen, erhältlich durch das Verfahren nach einem der Ansprüche 1-13, wobei der Cluster aufweist:
i) kontrahierende Zellen,
ii) elektrisch verbundene Zellen,
und exprimiert
iii) kardiale Marker einschließlich Nkx.2.5, Troponin und Myosin,
iv) Marker für funktionelle adrenerge Rezeptoren,
v) Marker für funktionelle Muscarinrezeptoren, vi) Marker für funktionelle lonenkanäle einschließlich hERG, Na+, Ca2+ und K+ Kanäle,
vii) einen oder mehrere endodermale Marker ausgewählt aus der Gruppe bestehend aus AFP, TF, APOA2, AHSG, SERPINA1, APOA1, APOC3, TTR, APOB und RBP4.
viii) Abwesenheit der Expression eines oder mehrerer der folgenden Marker für undifferenzierte Zellen: OCT-3/4, SSEA-4, TRA-1-60 und
den Cluster umfassend Gene, die hochgeregelt sind und aufweisen
i) Expressionswerte von 500 oder mehr,
ii) eine fache-Änderung in der Genexpression zwischen kardiomyozytenähnlichen Zellen und undifferenzierten hES-Zellen (FC_{CMLC}) von 10 oder mehr,
iii) ein Verhältnis zwischen FC_{CMLC} und FC_{MC} (d.h. die fache-Änderung zwischen gemischten differenzierten hES-Zellen und undifferenzierten hES-Zellen) von 10 oder mehr, und der Cluster alle der folgenden Gene exprimierenden Zellen umfasst:

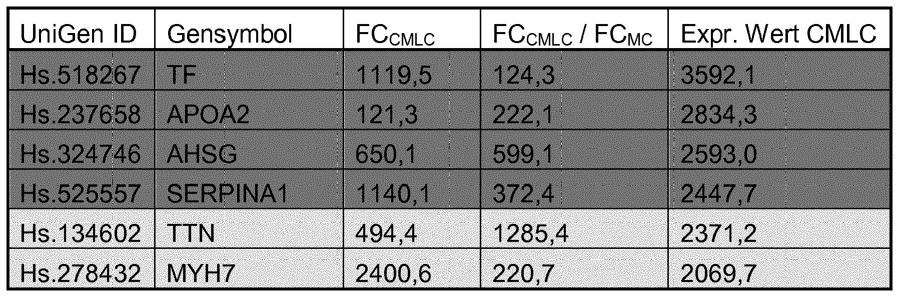

15. Cluster nach Anspruch 14, wobei der Cluster Zellen umfasst, die wenigstens 10, wenigstens 15, wenigstens 20, wenigstens 25 oder alle der folgenden Gene exprimieren:

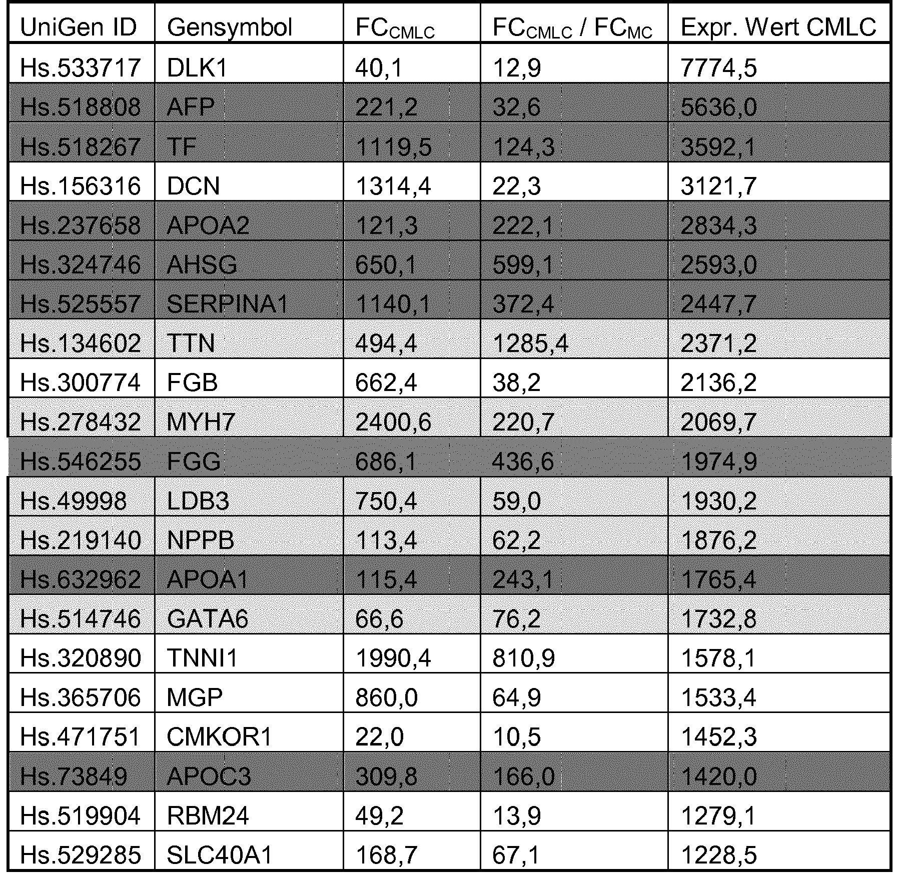

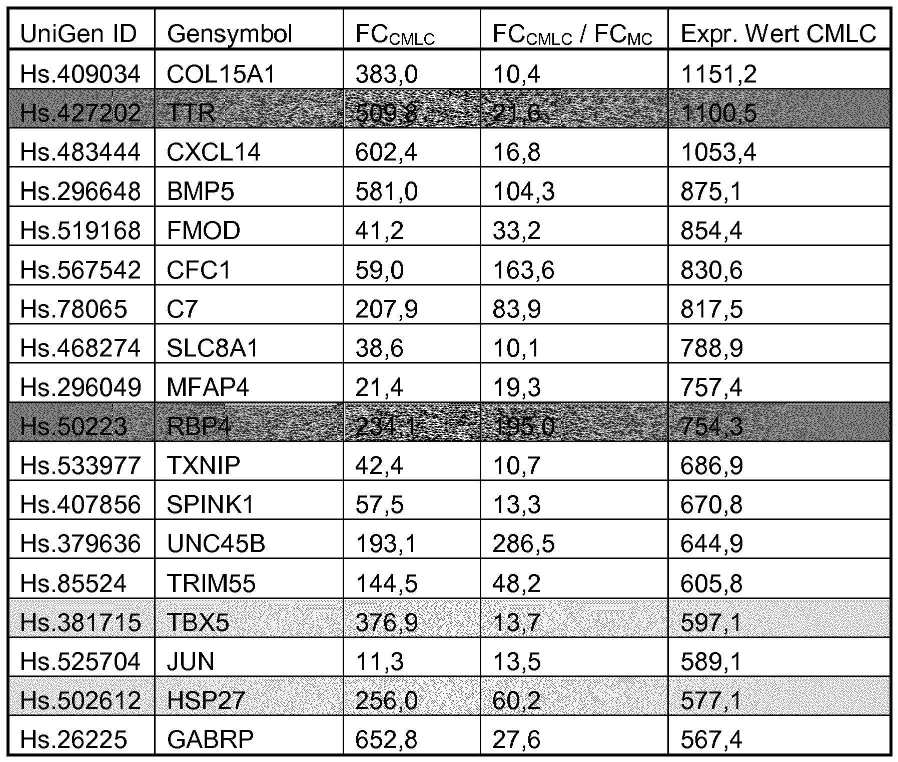

16. nach einem der Ansprüche 14-15 von hES-Zellen abgeleiteter Cluster mit einer oder mehreren der folgenden Eigenschaften:
i) die abgeleiteten hES-Zellen sind trisomische ein zusätzliches Chromosom 13 tragende hES-Zellen,
ii) die abgeleiteten hES-Zellen sind xeno-freie hES-Zellen, oder der von hES-Zellen abgeleiteter Cluster ist xeno-frei,
iii) Der Cluster enthält ungefähr 10 bis ungefähr 5000 Zellen.

17. Cluster nach einem der Ansprüche 14-16, mit einer oder mehreren der folgenden genetischen Eigenschaften:
i) 2 oder mehr wie beispielsweise 4 oder mehr, 6 oder mehr, 8 oder mehr, 10 oder mehr, 12 oder mehr oder 16 oder mehr der hochregulierten Gene sind mit kardialen Zellen assoziierte Gene (in der hierin beschriebenen Tabelle II);
ii) 2 oder mehr wie beispielsweise 4 oder mehr, 6 oder mehr, 8 oder mehr der hochregulierten Gene sind mit endodermalen Zellen assoziierte Gene (in der hierin beschriebenen Tabelle II);
iii) 2 oder mehr wie beispielsweise 4 oder mehr, 6 oder mehr, 8 oder mehr der hochregulierten Gene sind mit nicht kardialen oder nicht endodermalen Zellen assoziierte Gene, in der hierin beschriebenen Tabelle II;
iv) die hochregulierten Gene umfassen 10 oder mehr wie beispielsweise 20 oder mehr, 30 oder mehr, 40 oder mehr, 50 oder mehr, 55 oder mehr oder alle Gene, die hierin in der Tabelle II aufgeführt sind.

18. Zusammensetzung von kardiomyozytenähnlichen Clustern nach einem der Ansprüche 14-17, wobei die Cluster eine Mischung von nodalähnlichen Zellen, atrialähnlichen Zellen, ventrikelähnlichen Zellen enthalten.

19. Zusammensetzung von kardiomyozytenähnlichen Clustern nach Anspruch 18, wobei das Verhältnis zwischen der Anzahl von nodalähnlichen Zellen und der Anzahl von atrialähnliche Zellen enthaltenden Clustern innerhalb eines Bereichs von ungefähr 1:100 bis ungefähr 50:100 wie etwa von ungefähr 10:100 bis ungefähr 40:100, von ungefähr 20:100 bis ungefähr 40:100, von ungefähr 30:100 bis ungefähr 40:100 oder ungefähr 33:100-34:100 liegt, oder wobei das Verhältnis zwischen der Anzahl von nodalähnlichen Zellen und der Anzahl von ventrikelähnlichen Zellen innerhalb eines Bereichs von ungefähr 1:100 bis ungefähr 80:100 wie etwa von ungefähr 10:100 bis ungefähr 70:100, von ungefähr 30:100 bis ungefähr 70:100, von ungefähr 45:100 bis ungefähr 55:100 oder ungefähr 50:100 liegt, oder wobei das Verhältnis zwischen der Anzahl von ventrikelähnlichen Zellen und der Anzahl von atrialähnlichen Zellen innerhalb eines Bereichs von ungefähr 1:100 bis ungefähr 90:100 wie etwa von ungefähr 40:100 bis ungefähr 80:100, von ungefähr 50:100 bis ungefähr 75:100 oder ungefähr 66:100 liegt.

20. Zusammensetzung von kardiomyozytenähnlichen Clustern nach Anspruch 18, wobei das Verhältnis zwischen den nodalähnlichen Zellen, den atrialähnlichen Zellen und den ventrikelähnlichen Zellen 17:50:33 beträgt.

## Revendications

1. Procédé pour la préparation d'un agrégat comprenant des cellules de type cardiomyocyte, le procédé comprenant les étapes consistant à:
i) la suspension et la dissociation de cellules souches embryonnaires humaines (hES) non différenciées dans un milieu de culture, ledit milieu étant un milieu de base de culture de cellules, tel que Knock Out Dulbecco Modified Eagles Medium (DMEM) ou Modified Eagle Medium (MEM), éventuellement suppléé avec un ou plusieurs de sérum, tel que le sérum de foetus bovin, le sérum de veau foetal, le sérum humain ou le remplacement de sérum, pénicilline-streptomycine, GlutaMAX⁽™⁾-mercaptoéthanol et des acides aminés non-essentiels
ii) la soumission des agrégats ainsi dissociés à l'agrégation forcée,
iii) l'incubation des agrégats de cellules agrégées ainsi forcés dans un milieu de culture comprenant éventuellement un ou plusieurs facteurs de croissance pour obtenir une ou plusieurs structures 3D,
iv) le transfert d'une ou de plusieurs structures en 3D à une ou plusieurs plaques et l'incubation des structures 3D dans ledit milieu de culture comprenant éventuellement un ou plusieurs facteurs de croissance pour les développer en un ou plusieurs agrégats comprenant des cellules contractants, ledit milieu utilisé dans l'étape iii) et/ou iv) comprenant un inhibiteur GSK-3, l'activine A en tant que membre de la superfamille du facteur de croissance transformant bêta, la concentration de l'activine A suppléée au milieu de culture étant comprise entre 5 et 40 ng/ml, et bFGF en tant que membre de la famille du facteur de croissance des fibroblastes, la concentration de bFGF suppléée au milieu de culture étant comprise entre 5 et 40 ng/ml.

2. Procédé selon la revendication 1, comprenant en outre l'étape consistant à isoler un ou plusieurs agrégats en supprimant un ou plusieurs agrégats à partir de ladite plaque.

3. Procédé selon la revendication 1 ou 2, dans lequel l'agrégation forcée est réalisée par centrifugation, éventuellement performée à 100-800 x g pendant 2 à 20 minutes, par exemple de 200 à 600 x g pendant 5 à 10 minutes, ou 300-500 x g pendant 5 à 10 minutes, ou à 400 x g pendant 5 minutes.

4. Procédé selon la revendication 1, dans lequel l'agrégation forcée est réalisée par sédimentation pendant environ 1 à 36 heures, par exemple pendant environ 2 à 24 heures ou pendant environ 3 à 12 heures.

5. Procédé selon l'une quelconque des revendications 3 à 5, dans lequel les cellules obtenues après la sédimentation et/ou la centrifugation sont incubées pendant 1 à 10 jours dans l'étape iii), par exemple de 1 à 7 jours ou de 1 à 5 jours, pour la formation de structures 3D.

6. Procédé selon l'une quelconque des revendications précédentes, dans lequel le développement des agrégats à l'étape iv) est pendant 1 à 30 jours, par exemple de 1 à 15 jours, de 1 à 10 jours, ou de 1 à 5 jours, jusqu'à la formation des agrégats de cellules de type cardiomyocyte.

7. Procédé selon l'une quelconque des revendications précédentes, dans lequel la dissociation dans l'étape i) est effectuée mécaniquement.

8. Procédé selon la revendication 1, dans lequel la concentration de chacun de l'un ou de plusieurs facteurs de croissance est comprise entre environ 5 et environ 40 ng/ml.

9. Procédé selon la revendication 1 ou 8, dans lequel la concentration de l'activine A suppléée au milieu de culture est comprise entre 5 et 30 ng/ml, par exemple entre 5 et 20 ng/ml, entre 5 et 15 ng/ml, par exemple 8 ng/ml, 9 ng/ml, 10 ng/ml, 11 ng/ml ou 12 ng/ml, et la concentration de bFGF suppléée au milieu de culture étant comprise entre 5 et 30 ng/ml, par exemple entre 5 et 20 ng/ml, entre 5 et 15 ng/ml par exemple 10 ng/ml, 11 ng/ml, 12 ng/ml, 13 ng/ml ou 14 ng/ml.

10. Procédé selon l'une quelconque des revendications précédentes, dans lequel ledit milieu utilisé dans l'étape i) et/ou l'étape ii) et/ou iii) selon la revendication 1 comprend l'activine A, bFGF et/ou de FBS.

11. Procédé selon l'une quelconque des revendications précédentes, dans lequel ledit milieu utilisé dans l'étape iii) et/ou iv) selon la revendication 1 comprend un inhibiteur de la MAP-kinase p38, et dans lequel le membre de la famille des inhibiteurs de GSK-3 est SB 216763 et le membre de la famille des inhibiteurs de la MAP-kinase p38 est SKF-860002, la concentration d'inhibiteurs de GSK-3 suppléée audit milieu étant comprise entre 1 et 25 µM, par exemple entre 2 et 10 µM ou 5 µM et/ou la concentration des inhibiteurs de la MAP-kinase p38 suppléée au milieu de culture étant comprise entre 1 et 25 µM, par exemple entre 2 et 10 µM, ou 5 µM.

12. Procédé selon l'une quelconque des revendications précédentes, dans lequel ledit milieu utilisé dans l'étape iii) et/ou l'étape iv) selon la revendication 1 en outre comprend entre 100 et 2000 U/ml LIF, par exemple entre 500 et 1500 U/ml, ou 1000 U/m, et dans lequel le milieu comprenant LIF est remplacé après 2 à 8 jours d'incubation, par exemple après 4 à 5 jours, avec un milieu sans LIF.

13. Procédé selon l'une quelconque des revendications précédentes, dans lequel la ou les plaques de l'étape iii) selon la revendication 1 présente(nt) une surface revêtue de gélatine.

14. Agrégat comprenant des cellules de type cardiomyocyte pouvant être obtenu par un procédé selon l'une quelconque des revendications 1 à 13, dans lequel l'agrégat comprend:
i) des cellules contractiles,
ii) des cellules qui sont électriquement connectées,
et exprime
iii) des marqueurs cardiaques, y compris Nkx.2.5, la troponine et la myosine,
iv) des marqueurs pour les récepteurs adrénergiques fonctionnels,
v) des marqueurs pour les récepteurs muscariniques fonctionnels, vi) des marqueurs pour des canaux ioniques fonctionnels comprenant les canaux hERG, Na+, Ca2+ et K+,
vii) un ou plusieurs marqueurs endodermiques sélectionnés dans le groupe constitué par AFP, TF, APOA2, AHSG, SERPINA1, APOA1, APOC3, TTR, APOB et RBP4.
viii) l'absence d'expression de l'un ou de plusieurs marqueurs suivants pour des cellules indifférenciées: OCT-3/4, SSEA-4, TRA-1-60, et
l'agrégat comprenant des gènes qui sont régulés en amont et présentent,
i) des valeurs d'expression de 500 ou plus,
ii) un facteur de changement de l'expression des gènes entre les cellules de type cardiomyocyte et des cellules indifférenciées de hES (FC_{CMLC}) de 10 ou plus,
iii) un rapport entre FC_{CMLC} et FC_{MC} (à savoir le facteur de changement entre les cellules différenciées de hES et des cellules indifférenciées de hES) de 10 ou plus, et l'agrégat comprend des cellules exprimant les gènes suivants:

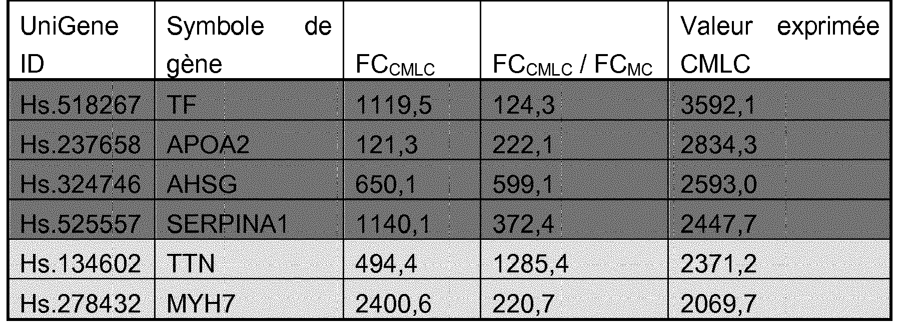

15. Agrégat selon la revendication 14, dans lequel l'agrégat comprend des cellules exprimant au moins 10, au moins 15, au moins 20, au moins 25 ou la totalité des gènes suivants:

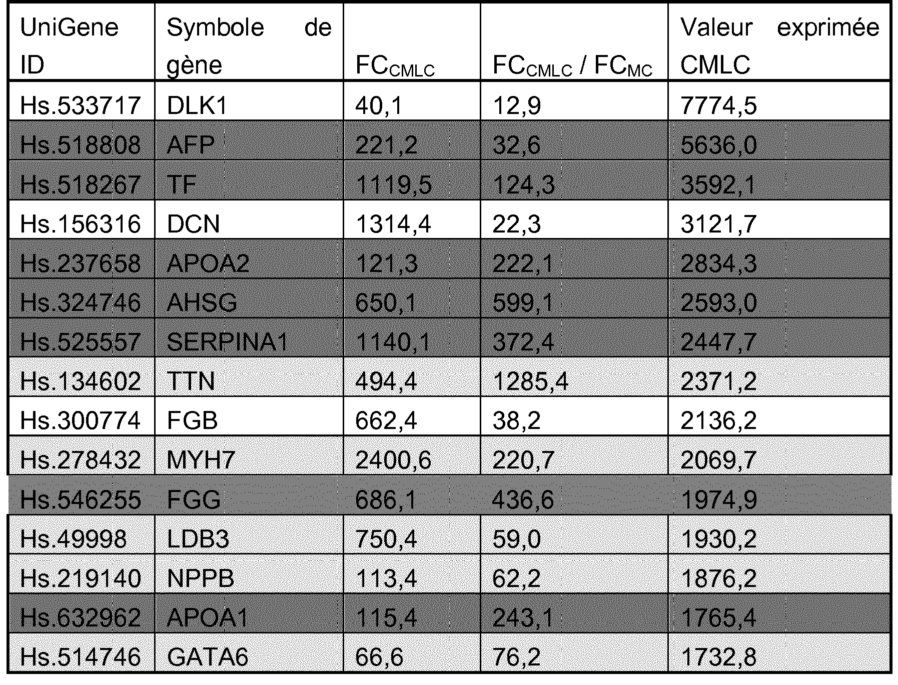

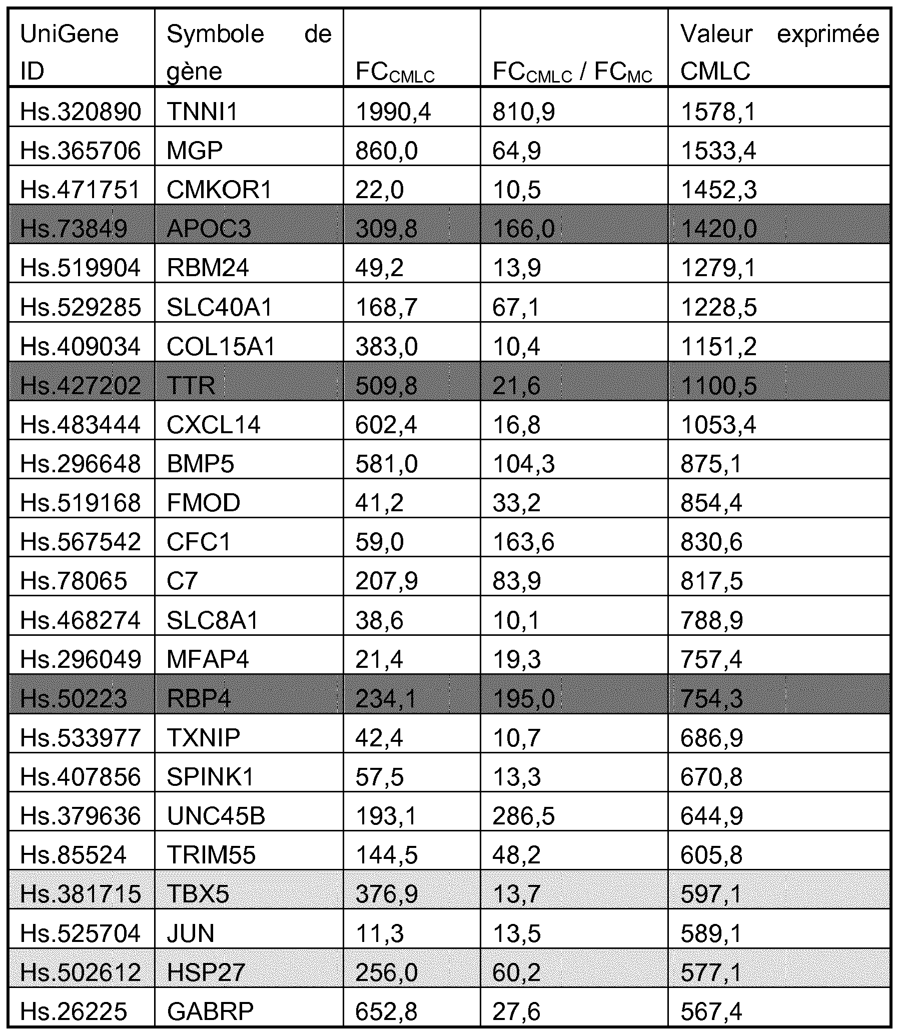

16. Agrégat selon l'une quelconque des revendications 14 à 15, dérivé de cellules hES, avec l'une ou plusieurs des propriétés suivantes:
i) les cellules dérivées de hES sont des cellules trisomiques de hES portant un chromosome supplémentaire 13,
ii) les cellules dérivées de hES sont des cellules xéno-libres de hES, ou l'agrégat dérivé de cellules de hES est xéno-libre,
iii) l'agrégat contient d'environ 10 à environ 5000 cellules.

17. Agrégat selon l'une quelconque des revendications 14 à 16, avec l'une ou plusieurs des propriétés génétiques suivantes:
i) 2 ou plus, par exemple 4 ou plus, 6 ou plus, 8 ou plus, 10 ou plus, 12 ou plus ou 16 ou plus des gènes régulés en amont sont des gènes associés à des cellules cardiaques (décrits dans le Tableau II ici).
ii) 2 ou plus, par exemple 4 ou plus, 6 ou plus, 8 ou plus des gènes régulés en amont sont des gènes associés à des cellules endodermiques (décrits dans le Tableau II ici).
iii) 2 ou plus, par exemple 4 ou plus, 6 ou plus, 8 ou plus des gènes régulés en amont sont des gènes associés à des cellules non cardiaques ou non endodermiques, décrits dans le Tableau II ici;
iv) les gènes régulés en amont comprennent 10 or plus, par exemple 20 ou plus, 30 ou plus, 40 ou plus, 50 ou plus, 55 ou plus ou tous les gènes décrits dans le Tableau II ici.

18. Composition d'agrégats de type cardiomyocyte selon l'une quelconque des revendications 14 à 17, dans laquelle les agrégats comprennent un mélange de cellules de type nodal, de cellules atriales et de cellules ventriculaires.

19. Composition d'agrégats de type cardiomyocyte selon la revendication 18, dans laquelle le rapport entre le nombre de cellules de type nodal et le nombre d'agrégats contenant des cellules atriales est compris entre environ 1:100 et environ 50:100 par exemple entre environ 10:100 et environ 40:100, entre environ 20:100 et environ 40:100, entre environ 30:100 et environ 40:100 ou environ 33:100-34:100 ou dans laquelle le rapport entre le nombre de cellules de type nodal et le nombre de cellules ventriculaires est compris entre environ 1:100 et environ 80:100 par exemple entre environ 10:100 et environ 70:100, entre environ 30:100 et environ 70:100, entre environ 45:100 et environ 55:100 ou environ 50:100, ou dans laquelle le rapport entre le nombre de cellules ventriculaires et le nombre de cellules atriales est compris entre environ 1:100 et environ 90:100 par exemple entre environ 40:100 et environ 80:100, entre environ 50:100 et environ 75:100 ou environ 66:100.

20. Composition d'agrégats de type cardiomyocyte selon la revendication 18, dans laquelle le rapport entre les cellules de type nodal, les cellules atriales et les cellules ventriculaires est 17:50:33.
